# EUROPEAN PATENT APPLICATION

(11) **EP 3 683 227 A1**
(43) Date of publication of application: **22.07.2020**
(21) Application number: 19152181.4
(22) Date of filing: 16.01.2019
(51) Int. Cl.: C07K 14/245, C12N 9/00, C12N 1/21

(54) **CELL FACTORIES FOR IMPROVED PRODUCTION OF COMPOUNDS AND PROTEINS DEPENDENT ON IRON SULFUR CLUSTERS**

(71) Applicant: Biosyntia ApS, 2100 Copenhagen Ø (DK)
(72) Inventor: BALI, Anne Pihl, 2100 København Ø (DK); ACEVEDO-ROCHA, Carlos G., 2100 København Ø (DK); GENEE, Hans Jasper, 2100 København Ø (DK); LAURIDSEN, Lasse Holm, 2100 København Ø (DK); GRONENBERG, Luisa, 2100 København Ø (DK); GOLABEK, Monika, 2100 København Ø (DK); MYLING-PETERSEN, Nils, 2100 København Ø (DK); LUBRANO, Paul, 2100 København Ø (DK)
(74) Representative: Birkeland, Morten

(57) **Abstract**

The invention relates to a genetically modified prokaryotic cell capable of improved iron-sulfur cluster delivery, characterized by a modified gene encoding a mutant Iron Sulfur Cluster Regulator (IscR) as well as one or more transgenes or upregulated endogenous genes encoding iron-sulfur (Fe-S) cluster polypeptides or proteins that catalyze complex radical-mediated molecular rearrangements, electron transfer, radical or non-redox reactions, sulfur donation or perform regulatory functions. Prokaryotic cells of the invention are characterized by enhanced activity of these iron-sulfur (Fe-S) cluster polypeptides, thereby enhancing their respective functional capacity, as well as facilitating enhanced yields of diverse compounds in free and protein-bound forms, including heme, hemoproteins, tetrapyrroles, B vitamins, amino acids, δ-aminolevulinic acid, biofuels, isoprenoids, pyrroloquinoline quinone, ammonia, indigo or their precursors, whose biosynthesis depends on their activity. The invention further relates to a method for producing each of said compounds, or their precursors using the genetically modified prokaryotic cell of the invention; as well as the use of the genetically modified prokaryotic cell.

## Description

### Field of the invention

The invention relates to a genetically modified prokaryotic cell capable of improved iron-sulfur cluster delivery, characterized by a modified gene encoding a mutant Iron Sulfur Cluster Regulator (IscR) as well as one or more transgenes or upregulated endogenous genes encoding iron-sulfur (Fe-S) cluster polypeptides that catalyze complex radical-mediated molecular rearrangements, electron transfer, radical or non-redox reactions, sulfur donation or perform regulatory functions. One example are radical SAM enzymes, which are involved in the biosynthesis of vitamins, cofactors, antibiotics and natural prodducts, metalloprotein cluster formation, enzyme activation as well as amino acid, nucleic acid and sugar post-transcriptional and post-translational modification like methylation. Protein production and metabolic pathways for the synthesis of a wide range of biological compounds are dependent on their Fe-S clusters. Prokaryotic cells of the invention are characterized by enhanced activity of these iron-sulfur (Fe-S) cluster polypeptides, thereby enhancing their respective functional capacity, as well as facilitating enhanced yields of diverse compounds, including heme, δ-aminolevulinic acid, amino acids (glutamate and branched-chain amino acids), vitamins (B₃, B₅ and B₁₂ derivatives), biofuels, isoprenoids, pyrroloquinoline quinone, ammonia, indigo or their precursors, as well as proteins containing said compounds in protein-bound form (tetrapyrrole proteins, hemoproteins, quinoproteins and quinohemoproteins); and whose biosynthesis depends on their activity. The invention further relates to a method for producing each of said compounds, or their precursors using the genetically modified prokaryotic cell of the invention; as well as the use of the genetically modified prokaryotic cell.

### Background of the Invention

The production of a wide range of compounds (such as vitamins, pharmaceuticals, food supplements, flavors, fragrances, biofuels, fertilizers, and dyes) currently relies on chemical synthesis, which is costly. Biosynthetic methods for their manufacture would provide an alternative, more cost-effective means for meeting current and future needs. Biosynthetic pathways suitable for synthesis of many such compounds are dependent on one or more iron-sulfur (Fe-S) cluster proteins. Development of cell factories tailor-made for the production of those compounds whose biosynthesis depends on such pathways include:
Biotin (also known as vitamin B₇ or vitamin H), vitamin B₃ (NAD, NR, and NMN), cobalamin (also known as vitamin B₁₂) and pantothenate (vitamin B₅), and vitamin K are essential dietary vitamins for humans, because in common with other metazoans, they cannot produce biotin, nicotinamide-derived vitamins or cobalamin.

Heme is a member of the tetrapyrrole family, encompassing several important molecules of diverse metabolic functions (e.g. vitamin B₁₂, chlorophyll, heme, siroheme, chlorophyll, and cofactor F430). Heme plays an essential role in both prokaryotes and eukaryotes for heme-containing proteins. Hemoproteins are involved in enzymatic reactions (e.g. cytochrome P450, cytochrome c oxidase, peroxidase, ligninase, catalase, tryptophan 2,3-dioxygenase, nitric oxide synthase), oxygen transport (e.g. myoglobin, hemoglobin, neuroglobin, cytoglobin and leghemoglobin) and electron transport (e.g. cytochromes in respiratory chain). Heme and hemoproteins find various applications such as in iron supplies for treating anemia, iron supplements for artificial meat, and use in biocatalysts to produce a wide range of compounds in the food, feed, pharmaceutical, chemical and cosmetic industries.

Isoprenoids and terpenoids are large families of natural compounds with many applications in the food, feed, pharmaceutical, flavor, fragrance, chemical and cosmetics industries.

Pyrroloquinoline quinone (PQQ), also called methoxatin, is a redox cofactor found in several enzymes. It has been shown to have potent antioxidant properties, as well as being a growth promoting agent, with applications as a food or pharmaceutical ingredient. Quinoproteins (e.g. methanol dehydrogenase, glucose dehydrogenase) can be also used in biosensing and bioconversion of useful compounds for medical applications as well as in bioremediation.

Additional dietary compounds that might advantageously be produced in a cell factory include the amino acids L-valine, L-leucine, L-isoleucine and L-glutamate. A biosynthetic route for their production, unlike chemical synthesis, ensures that the product is exclusively L-isomers. Since L-glutamate is the precursor of monosodium glutamate, this finds use as a food flavor. Biofuel production (such as butanol and isobutanol) may also be achieved using such a cell factory, since a biosynthetic route for production of such biofuels shares key catalytic steps in common with the branched-chain amino acids. Glutamate is a precursor of δ-aminolevulinic acid, which is the building block of pharmaceuticals, plastics and many different chemicals. δ-aminolevulinic acid is also the precursor of tetrapyrroles (porphyrins), which are compounds that can be produced in free- or protein-bound form with many applications in the medical, pharmaceutical, electronics and chemical industries.

Ammonium, obtained by means of biological Nitrogen Fixation (BNF) in a cell factory, would provide a source of natural fertilizers for plants, and replace current polluting nitrogen fertilizers used by agricultural industries. BNF also takes place in plants that form symbiotic associations with diazotrophs, where the efficiency of nitrogen deficiency determines the need for nitrogen fertilizers.

Indigo is a blue dye in high demand in the global textile industry whose industrial production currently relies on a chemical and polluting process.

The use of prokaryote-based cell factories is a potential route for the biosynthetic production of the above nutrient supplements. The advantages of recombinant *E. coli* as a cell factory for production of bio-products are widely recognized due to the fact that: (i) it has unparalleled fast growth kinetics; with a doubling time of about 20 minutes when cultivated in glucose-salts media and under optimal environmental conditions, (ii) it easily achieves a high cell density; where the theoretical density limit of an *E. coli* liquid culture is estimated to be about 200 g dry cell weight/L or roughly 1 × 10¹³ viable bacteria/mL. Additionally, there are many molecular tools and protocols at hand for genetic modification of *E. coli;* as well as being an organism that is amenable to the expression of heterologous proteins; both of which may be essential for obtaining high-level production of desired bio-products.

In general, there exists a need to identify the bottlenecks in those complex biosynthetic pathways that are dependent on Fe-S clusters and often required to facilitate the production of diverse compounds in prokaryote-based cell factories (e.g. *E. coli*), and to provide tailor-made cell factories that overcome the diversity of factors that may limit their use and productivity.

### Summary of the invention

According to a first embodiment the invention provides a genetically modified prokaryotic cell comprising:
a. a transgene or a genetically modified iscR gene encoding a mutant IscR polypeptide, and
b. transgenes or endogenous genes encoding at least one Fe-S cluster polypeptide,
wherein the at least one FE-S cluster polypeptide is not any one of i. biotin synthase (EC: 2.8.1.6), ii. lipoic acid synthase (EC: 2.8.1.8), iii. HMP-P synthase (EC: 4.1.99.17), and vi. tyrosine lyase (EC: 4.1.99.19); and
wherein the endogenous genes are operably linked to genetically modified regulatory sequences capable of enhancing expression of said endogenous genes, and
wherein the mutant IscR polypeptide as compared to a corresponding non-mutant IscR polypeptide has an increased apoprotein:holoprotein ratio in the cell; and wherein the production of at least one compound resulting from the catalytic activity of the at least one Fe-S cluster polypeptide is enhanced when compared to a prokaryotic cell comprising the genetically unmodified iscR gene and the transgenes or the endogenous genes encoding at least one Fe-S polypeptide.
In a further embodiment, the amino acid sequence of said mutant IscR polypeptide comprises at least one amino acid modification and exits only as an apoprotein.
In a further embodiment, the genetically unmodified iscR gene is endogenous or heterologous to the cell.

In a further embodiment, the at least one Fe-S cluster polypeptide is heterologous or endogenous to the cell.

In a further embodiment, said at least one Fe-S cluster polypeptide is selected from the group consisting of:a HemN polypeptide having oxygen-independent coproporphyrinogen III oxidase synthase (EC: 1.3.98.3) activity; a HemZ polypeptide having oxygen-independent, coproporphyrinogen III oxidase synthase (EC: 1.3.98.3) activity; a P450 polypeptide having monooxygenase activity (EC: 1.14.14.1); a NadA polypeptide having quinolate synthase (EC: 2.5.1.72) activity; a CobG polypeptide having precorrin-3B synthase (EC: 1.3.98.3); an IlvD polypeptide having dihydroxy-acid dehydratase activity (EC: 4.2.1.9); an IspG polypeptide having 4-hydroxy-3-methylbut-2-en-1-yl diphosphate synthase (EC: 1.17.7.3); an IspH polypeptide having 4-hydroxy-3-methylbut-2-enyl diphosphate reductase activity (EC: 1.17.7.4); a large chain GltB polypeptide and a small chain GltD polypeptide having glutamate synthase [NADPH] activity (EC: 1.4.1.13); a PqqE polypeptide having PqqA peptide cyclase activity (EC: 1.21.98.4); a NifB polypeptide having nitrogenase FeMo cofactor biosynthesis activity; a NdoB and NdoC polypeptide having naphthalene 1,2-dioxygenase activity (EC: 1.14.12.12) together with a NdoA and NdoR polypeptide having ferredoxin-NAD(P)+ reductase activity (EC: 1.18.1.7); and a hydA polypeptide having hydrogenase activity (EC: 1.18.99.1)
In a further embodiment, the invention provides a cell culture, comprising the genetically modified prokaryotic cell, and a growth medium.
According to a second embodiment, the invention provides a method for producing a compound resulting from catalytic activity of said Fe-S cluster polypeptide, comprising the steps of:
a. introducing a genetically modified prokaryote of the invention, into a growth medium to produce a culture;
b. cultivating the culture; and
c. recovering said compound produced by said culture, and optionally purifying the recovered compound.
According to a third embodiment, the invention for a use of a genetically modified gene encoding a mutant iscR polypeptide to increase production of at least one compound resulting from the catalytic activity of at least one Fe-S cluster polypeptide in a genetically modified prokaryotic cell as compared to a prokaryotic cell comprising the genetically unmodified iscR gene, wherein said Fe-S cluster polypeptideis not any one of biotin synthase (EC: 2.8.1.6), lipoic acid synthase (EC: 2.8.1.8), HMP-P synthase (EC: 4.1.99.17), and tyrosine lyase (EC: 4.1.99.19), wherein the mutant IscR polypeptide as compared to a non-mutant IsR polypeptide has an increased apoprotein:holoprotein ratio in the cell.

### Brief description of the drawings

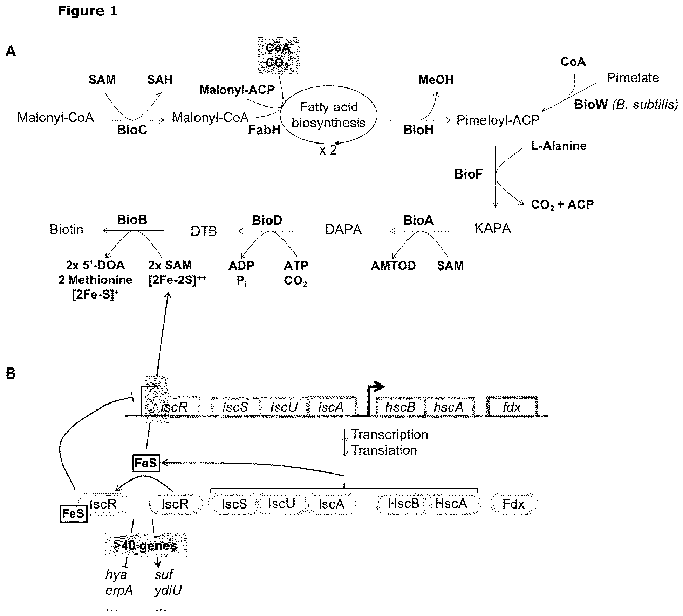

**Figure 1** Cartoon showing A) intermediates of the biotin pathway in bacteria and the respective enzymatic steps leading to synthesis of biotin. SAM: S-adenosyl-L -methionine, SAH: S-Adenosyl-L-homocysteine, CoA: coenzyme A, ACP: Acyl Carrier Protein, KAPA: 7-keto-8-aminopelargonic acid, AMTOD: S-adenosyl-2-oxo-4-thiomethylbutyrate, DAPA: 7,8-Diaminopelargonic Acid, DTB: desthiobiotin, 5'DOA: 5'-deoxyadenosine. B) *isc*-operon structure and role in Fe-S-cluster formation as well as the regulatory mechanism of IscR. The *isc* operon comprises an *iscR* gene encoding the IscR that regulates expression of the genes: *iscS* (cysteine desulfurase), *iscU* (scaffold), *iscA* (A-type protein), *hscB* (Dnaj-like co-chaperone), *hscA* (DnaK-like chaperone), and *fdx* (ferredoxin). IscR also regulates >40 genes including the genes *hyaA, ydiU, erpA,* and *sufA.*

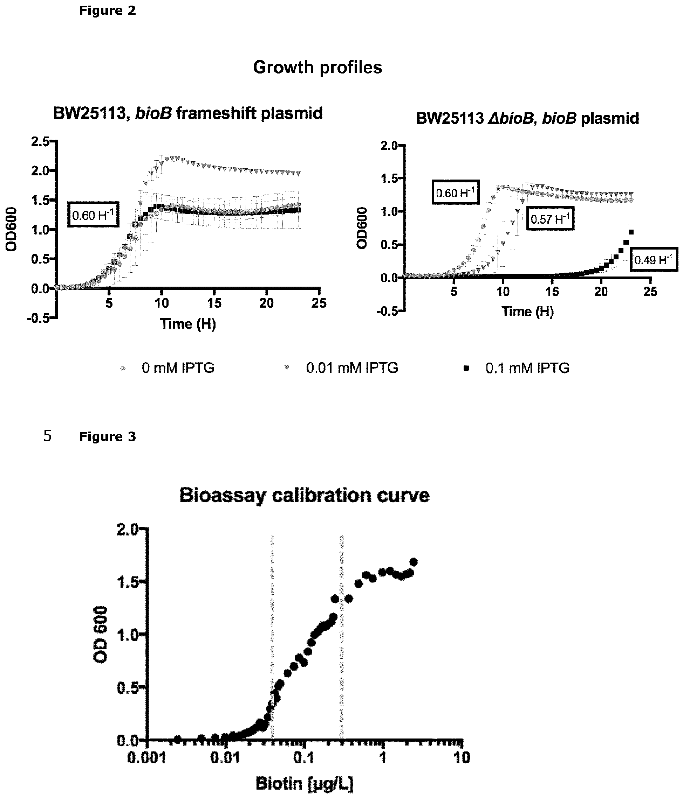

**Figure 2** Graphical presentation of the cell density (measured at OD₆₀₀), measured over time, of a (*E. coli* BW25113) Δ*bioB* strain comprising an IPTG inducible *bioB* expression plasmid (right panel); and the reference (*E. coli* BW25113) strain comprising an IPTG inducible frameshifted *bioB* (premature stop codon) expression plasmid (left panel). OD₆₂₀ was measured using a plate reader and converted to OD₆₀₀, for 4 biological strain replicates grown in 200 µL mMOPS with 0.1 g/L DTB, 50 µg/mL kanamycin and either 0 (dots) 0.01 (triangles) or 0.1 (squares) mM IPTG. Respective exponential growth rate values are shown in the adjacent boxes.
**Figure 3** Graphical presentation of a scatter plot showing the final cell density (measured at OD₆₀₀) of cultures of *E*. *coli* BS1011 comprising plasmid pBS451 grown on 150 µL mMOPS medium with 40 µg/mL zeocin supplemented with zero or increasing concentrations of biotin, ranging up to 0.244 µg biotin/mL after incubation for 20 hours at 37°C with 275 rpm shake (providing a bioassay calibration curve for quantifying biotin). The stippled vertical grey lines identify an optimal concentration range of between 0.04 to 0.24 µg biotin/L for the biotin bioassay.

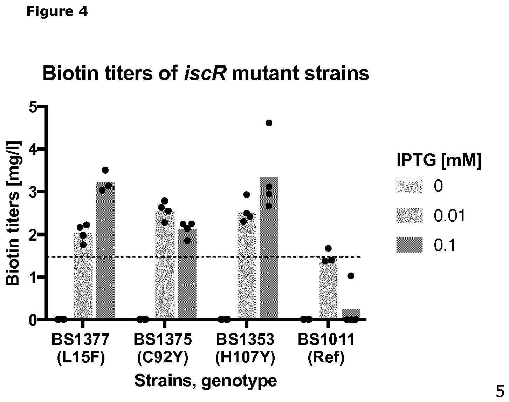

**Figure 4** Bar diagram showing biotin production of 3 different *iscR* mutant strains expressing mutant IscR having amino acid mutations (BS1377, L15F), (BS1375, C92Y) and (BS1353, H107Y) and an *E. coli* BW25113 Δ*bioB* strain (BS1011, Ref) (see Table 1 for strains) in 4 biological replicates each comprising an IPTG-inducible *bioB* expression plasmid (pBS412). Strains were grown in 400 µL mMOPS with 0.1 g/L DTB and 50 µg/mL kanamycin for 24 hours at 37°C with 275 rpm shake. Bars illustrate the mean biotin production value (height) and IPTG induction level (gray shade), black dots show biotin production from individual replicate cultures and the horizontal stippled line indicates the maximum biotin production from a reference wild type strain. Note that none of the strains produced detectable levels of biotin when cultured in the absence of IPTG.

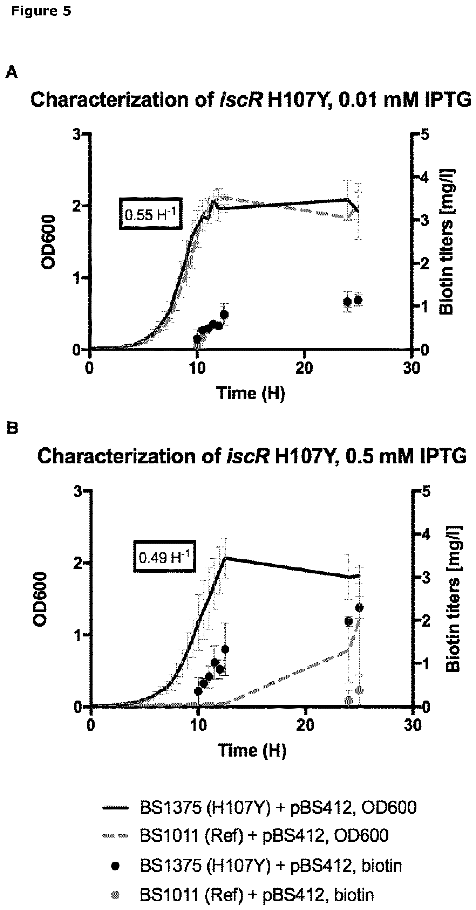

**Figure 5** Graphical presentation of the cell density and biotin production of the iscR mutant strain expressing the mutant IscR (BS1353, H107Y), and an *E. coli* BW25113 *ΔbioB* strain (BS1011, Ref), wherein each stain comprises an IPTG-inducible *bioB* gene expression plasmid (pBS412). The data represents the average measured OD₆₀₀ of three biological replicates each of the *iscR* H107Y mutant strain (solid dark line) and the reference strain, *E. coli* BW25113 *ΔbioB* strain (stippled light grey) and biotin production by the respective *iscR* H107Y mutant strain (solid dark dots) and the reference strain, E. coli BW25113 *ΔbioB* strain (light gray dots) monitored over a period of 25 hours. The strains were grown in 50 mL mMOPS with 0.1 g/L DTB, 0.01 (A) or 0.5 mM IPTG (B) and 50 µg/mL kanamycin in a 250 mL baffled shake flask at 37°C with 275 rpm shaking. Growth rates are shown in the black box.

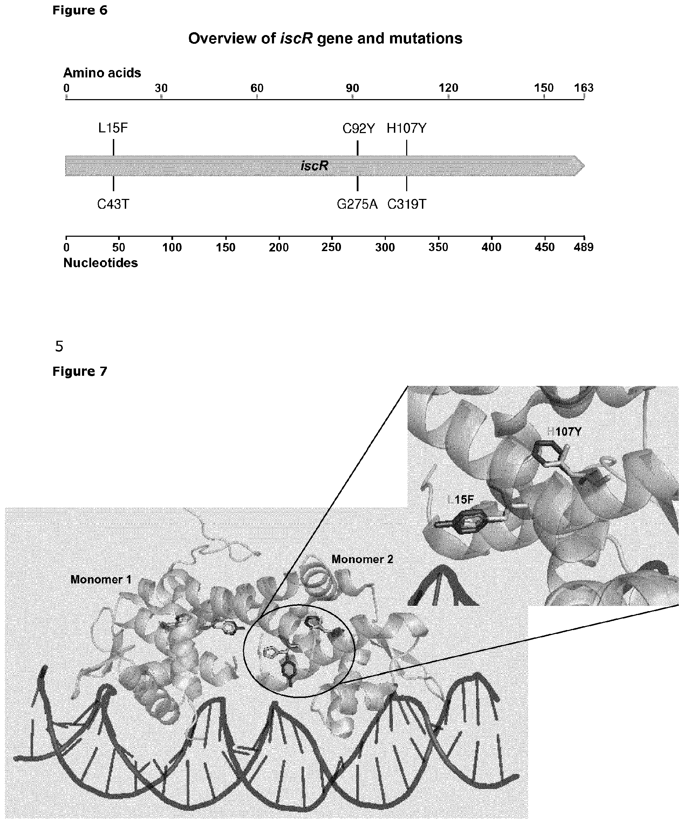

**Figure 6** Cartoon showing the IscR coding sequence annotated to show the location of the nucleotide and amino acid sequence mutations in the *iscR* genes of the identified mutant strains.
**Figure 7** Cartoon showing the crystal structure (PDB entry 4HF1) of IscR dimer (grey) bound to *hya* DNA binding site (black) with L15F and H107Y *iscR* mutants indicated as sticks (WT amino acid in grey and mutant amino acid in black); and an expanded image highlighting the mutated residues.

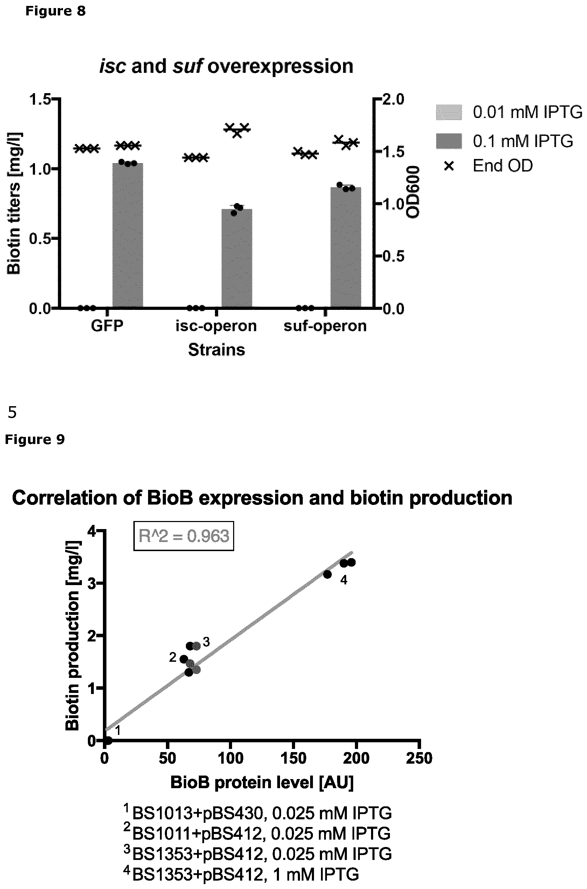

**Figure 8** Bar diagram showing biotin production of an *E. coli* strain comprising an IPTG-inducible *bioB* expression plasmid and either a plasmid comprising an *isc*-operon (*iscSUA-hscBA-fdx,* corresponding to a native *E. coli isc* operon structure lacking *iscR* gene) or the *E. coli suf*operon (*sufABCDSE*) operably linked to a strong ribosomal binding site (RBS) and a T5 LacO repressed promoter from a medium copy number plasmid (p15A ori) or a control plasmid. The control plasmid comprised an IPTG-inducible gene encoding GFP instead of the *suf-* or *isc*-operon. Biological triplicates of each strain were cultured in mMOPS with 100 µg/mL ampicillin and 50 µg/mL spectinomycin under low (0.01 mM IPTG) and high (0.1 mM IPTG) induction and providing 0.1 g/L (DTB) as substrate. The strains were grown in deep well plates for 24 hours at 37 °C with 275 rpm, after which biotin production was evaluated using a growth-based bioassay. Bars illustrate the mean biotin production value (height) and IPTG induction level (gray shade), black dots show biotin production from individual replicates and the crosses show end-point (end) cell density of each strain, measured as OD₆₀₀. Note that none of the strains produced detectable levels of biotin when induced with 0.01 mM IPTG.
**Figure 9** Graphical presentation of the correlation between BioB protein expression levels and biotin production in 4 different samples performed in triplicate. The strains are BS1013 (E. *coli* BW25113, background strain) with pBS430 (*bioB* frameshift IPTG inducible plasmid), BS1011 (BS1013 with Δ*bioB*) with pBS412 (*bioB* IPTG inducible plasmid), BS1353 (BS1011 with *iscR H107Y* mutation) with pBS412. Strains were grown in mMOPS with 0.1 g/L DTB and IPTG as indicated in the graph.

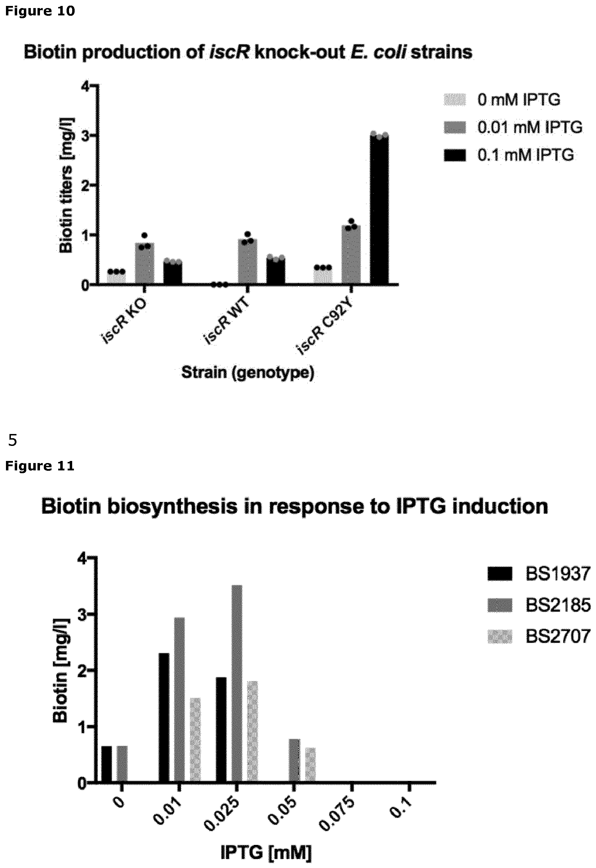

**Figure 10** Bar diagram showing biotin *de novo* production of *E. coli* Δ*bioB* strains comprising an IPTG-inducible *bioB* expression plasmid and either of the following genomic variants of *iscR:* wild type (*iscR* WT), knock-out mutant encoding E22* glutamic acid mutated to a stop codon on position 22 (*iscR* KO), mutant (*iscR* C92Y) encoding a cysteine to tyrosine substitution at position 92. Bars illustrate the mean biotin production value (height) at given levels of IPTG induction (shade of gray), dots show biotin production from individual replicates. Biological triplicates of each strain were cultured in mMOPS with 100 µg/mL ampicillin under no (0 mM IPTG), low (0.01 mM IPTG) and high (0.1 mM IPTG) induction and providing 0.1 g/L DTB as substrate. Each strain was grown in a deep well plate for 24 hours at 37 °C with 275 rpm, after which biotin production was evaluated using a growth-based bioassay.
**Figure 11** Bar diagram showing biotin production by *E. coli ΔbioABFCD iscR* H107Y (encoding a histidine to tyrosine substitution at position 107) strains comprising IPTG-inducible BioB overexpression plasmid pBS679 alone (BS1937) or in addition pBS1112 (BS2185) with constitutive overexpression of FldA-Fpr or pBS1054 (BS2707) with constitutive overexpression of GFP. Each strain was cultured in mMOPS with100 µg/ml ampicillin, 0.1 g/L desthiobiotin (DTB) as substrate and either 0, 0.01, 0.025, 0.05, 0.075 or 0.1mM IPTG. The medium for BS2185 and BS2707 was identical except for the inclusion of 50µg/mL kanamycin. The strains were grown in deep well plates for 24 hours at 37 °C with 275 rpm, after which biotin production was evaluated using a growth based bioassay. Bars illustrate biotin production value (height) by the respective strains: BS1937 (black bars); BS2185 (grey bars); and BS2707 (checkered grey bars).

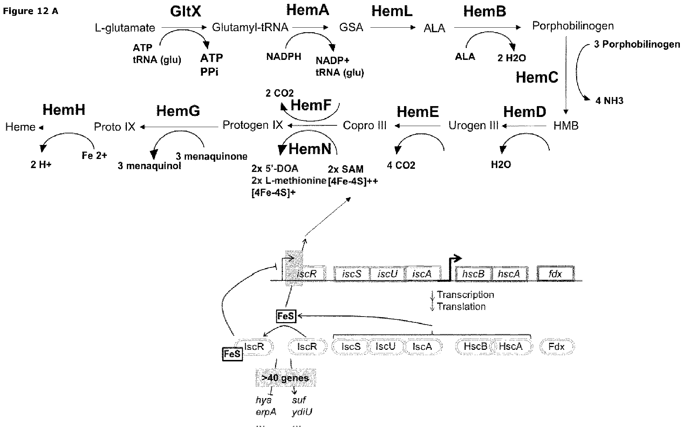

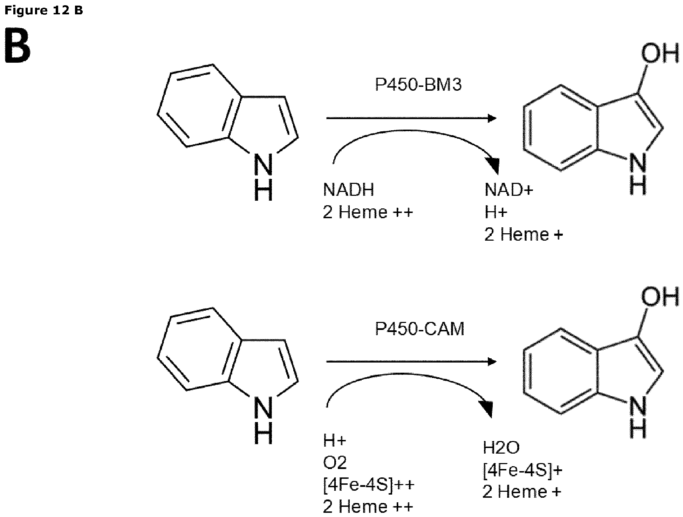

**Figure 12** **A** Cartoon showing (upper part) the heme biosynthetic pathways in a prokaryote (e.g. *Escherichia coli*), catalyzed by ten enzymatic steps. The key enzymes in the pathway include Glutamate-tRNA ligase (GltX); Glutamyl-tRNA reductase (HemA); Glutamate-1-semialdehyde 2,1-aminomutase (HemL); Delta-aminolevulinic acid dehydratase (HemB); Porphobilinogen deaminase (HemC); Uroporphyrinogen III methyltransferase (HemD); Uroporphyrinogen decarboxylase (HemE). The synthesis of Protoporphyrinogen IX from Coproporphyrin III is catalyzed by either HemN or HemF. HemN is an [4Fe-4S] cluster-dependent enzyme which uses two molecules of S-adenosyl-methionine (SAM) as cofactors, releasing 5'-deoxyadenosine (5'-DOA) and methionine per turnover. HemF is an oxygen-dependent coproporphyrinogen III oxidase synthase (EC: 1.3.3.3), which consumes two molecules of the co-factor S-adenosyl-methionine (SAM). Protoporphyrinogen IX is further catalyzed into Protoporphyrin IX and finally heme is formed via menaquinone (HemG) and ferrochelatase (HemH). Abbreviations: L-glutamate 1-semialdehyde (GSA); δ-aminolevulinic acid (ALA); hydroxymethylbilane (HMB); Uroporphyrinogen III (Urogen III); Copropophyrin III (Copro III); Protoporphyrinogen IX (Protogen IX); Protoporphyrin IX (Proto IX); Adenosyl Tri Phosphate (ATP); Nictotinamide Adenine Dinucleotide (NADH). (Lower part) shows the isc-operon structure and role in Fe-S-cluster formation as well as the regulatory mechanism of IscR (see Figure 1B). **B)** Cartoon showing a model reaction for enhanced production of hemoproteins. The model reaction is illustrated by the enzymes P450-BM3 (Fe-S cluster- independent), and P450-CAM ([2Fe-2S] cluster dependent) using indole as substrate, which is oxidased to indoxyl, and where the end product indigo is detected.

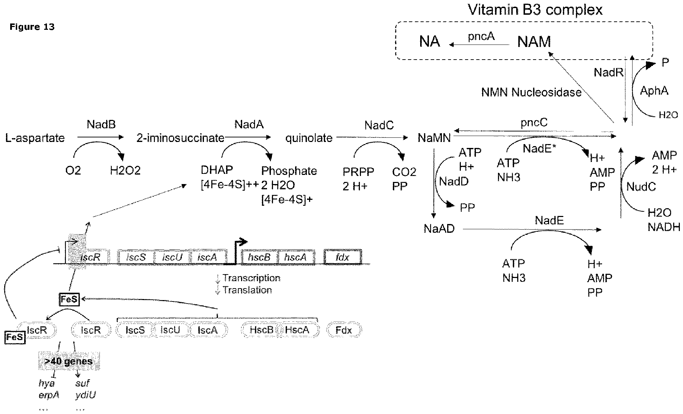

**Figure 13** Cartoon showing (upper part) Vitamin B3 complex (NR, NAM and NA) biosynthetic pathway in a prokaryotes. The intermediate Quinolate is made by the condensation and cyclisation of 2-iminosuccinate by the [4Fe-4S] cluster enzyme NadA, requiring dihydroxy acetone phosphate (DHAP) as a co-factor. Nicotinic acid mononucleotide (NaMN) synthesis from quinolate is catalyzed by NadC and Nicotinic acid adenine dinucleotide (NaAD) formation from NaMN is catalyzed by NadD. Nicotinamide adenine dinulceotide (NAD+) formation is catalysed by NadE, via ATP-dependent amidation of NaAD. NudC and aphA subsequently convert NAD+ to nicotinamide mononucleotide (NMN) and Nicotinamide riboside (NR), respectively. NadE* is a NadE homologoue which prefers NaMN as a substrate and can convert NaMN to NMN directly. Nicotinamide (NAM) is produced by phosphatase activity of the NMN nucleosidase. NAM is converted to nicotinic acid by pncA. ATP = Adenosyl triphosphate; AMP = Adenosyl mono-phosphate; PP = Diphosphate; PRPP = 5-phospho-alpha-D-ribose-1-diphosphate. (Lower part) shows the isc-operon structure and role in Fe-S-cluster formation as well as the regulatory mechanism of IscR (see Figure 1B).

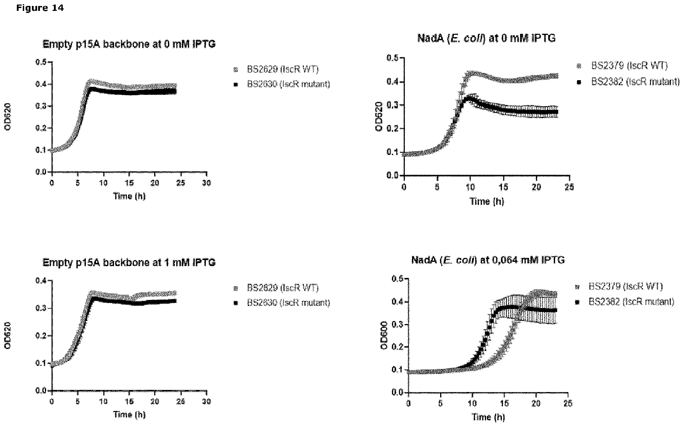

**Figure 14** Diagrams showing the OD₆₂₀ₙₘ of cultures over time, as a measure of cell growth, for *E. coli* strains expressing IscR WT protein (BS2629/BS2379) and IscR mutant protein (BS2630/BS2382) and comprising either a control empty plasmid (left panels) or a plasmid encoding *E. coli* NadA (right panels) without induction (top panels) or with IPTG induction (bottom panels). The strains with empty plasmid (BS2629/BS2630) and plasmid encoding *E. coli* nadA (BS2379/BS2382) were induced with 1 and 0.064 mM IPTG, respectively (bottom left and right). The error bars denote the standard deviation of four biological replicates.

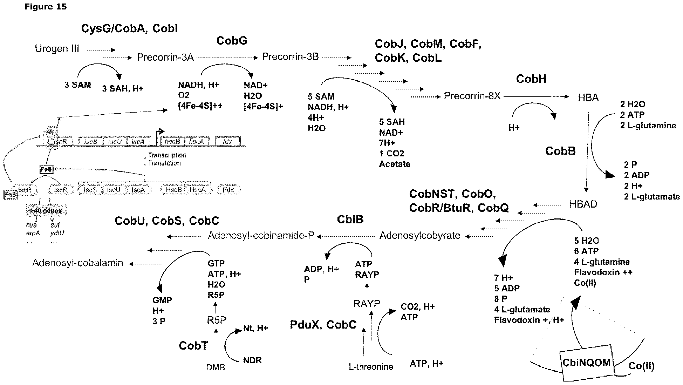

**Figure 15** Cartoon showing the aerobic cobalamin biosynthetic pathway in a prokaryote (e.g. *Pseudomonas denitrificans*)*.* The synthesis of Precorrin-3-B from Precorrin-3-A is catalyzed by the [4Fe-4S] cluster enzyme CobG. The isc-operon structure and its role in supplying Fe-S-clusters to the CobG enzyme is shown. Cobalt in imported in the cell factory by using the cobal transporter made of the proteins CbiNQOM. SAM = S-Adenosyl Methionine; SAH = S-Adenosyl-Homocysteine; ATP = Adenosyl Tri Phosphate; ADP = Adenosyl Bi Phosphate; HBA = Hydrogenobyrinic Acid; HBAD = Hydrogenobyrinic Acid a,c diamide; RAYP = (*R*)-1-amino-2-propanol *O*-2-phosphate; DMB = 5,6-dimethylbenzimidazole; NDR = β-nicotinate D-ribonucleotide; R5P = α-ribazole 5'-phosphate; Nt = Nicotinate; GTP = Guanosyl Tri Phosphate; GMP = Guanosyl Mono Phosphate. At the left side, the isc-operon is shown and the role in Fe-S-cluster formation as well as the regulatory mechanism of IscR (see Figure 1B).

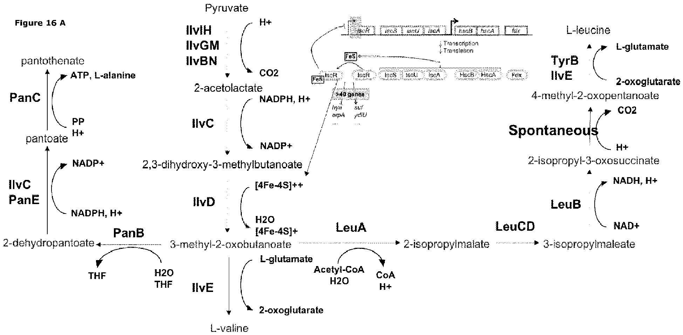

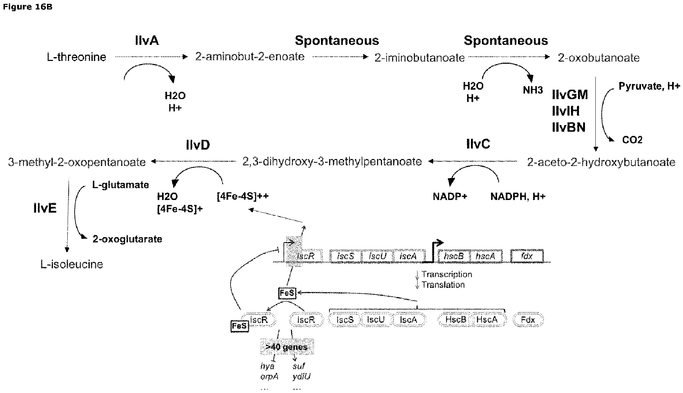

**Figure 16** Cartoon showing the common pantothenate and branched amino acid biosynthetic pathways. A) is the L-leucine, L-valine and pantothenate biosynthetic pathway; which share a common precursor, 3-methyl-2-oxobutanoate, whose synthesis from 2,3-dihydroxy-3-methylbutanoate is catalyzed by IlvD. (B) is the L-isoleucine biosynthetic pathway, where IlvD also catalyzes the synthesis of 3-methyl-2-oxopentanoate from 2,3-dihydroxy-3-methylpentanoate. NADPH = Nicotinamide adenine dinucleotide phosphate. The isc-operon structure and its role in supplying Fe-S-clusters to the IlvD enzyme is shown.

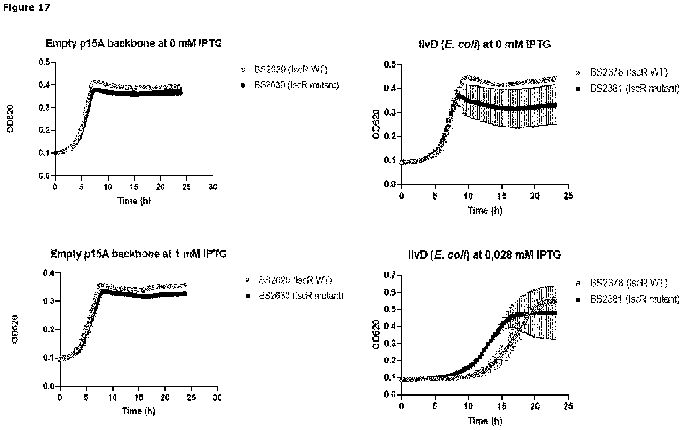

**Figure 17** Diagrams showing the OD₆₂₀ₙₘ of cultures over time, as a measure of cell growth, for *E. coli* strains expressing IscR WT protein (BS2629/BS2378) and IscR mutant protein (BS2630/BS2381) and comprising either a control empty plasmid (left panels) or a plasmid encoding *E. coli* ilvD (right panels) without induction (top panels) or with IPTG induction (bottom panels). The strains with empty plasmid (BS2629/BS2630) and plasmid encoding *E. coli* ilvD (BS2379/BS2381) were induced with 1 and 0.028 mM IPTG, respectively (bottom left and right). The error bars denote the standard deviation of four biological replicates.

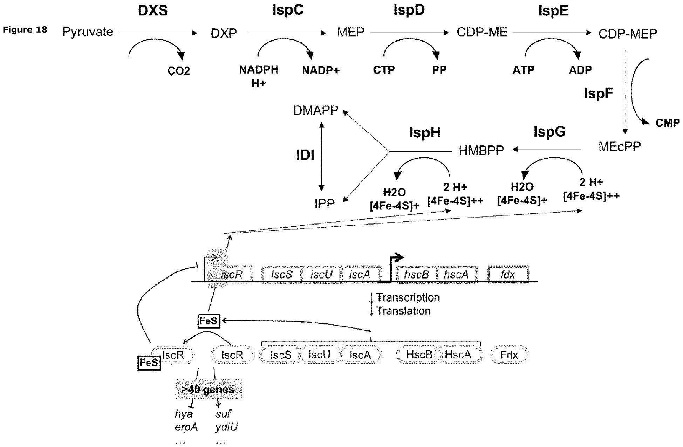

**Figure 18** Cartoon showing the Methyl Erythritol Pathway (MEP) isoprenoid pathway in a prokaryote (e.g. *Escherichia coli*)*.* The isc-operon structure and its role in supplying Fe-S-clusters to the IspG and IspH enzymes is shown. ADP = adenosinediphosphate; ATP = adenosine triphosphate; CMP = cytidine monophosphate; CTP = cytidine triphosphate; DHAP = dihydroxyacetone phosphate; DMAPP = dimethylallyl diphosphate; DXP = 1-deoxyxylulose 5-phosphate; HMBPP = 1-hydroxy-2-methyl-2-(E)-butenyl 4-diphosphate; IPP = isopentenyl diphosphate; MEcPP = 2-C-methyl-D-erythritol-2,4 cyclodiphosphate; MEP = 2-C-methyl-D-erythritol-4-phosphate; NAD+ = nicotinamide adenine dinucleotide (oxidized); NADH = nicotinamide adenine dinucleotide (reduced); NADP+ = nicotinamide adenine dinucleotide phosphate (oxidized); NADPH = nicotinamide adenine dinucleotide phosphate (reduced); PP = pyrophosphate.

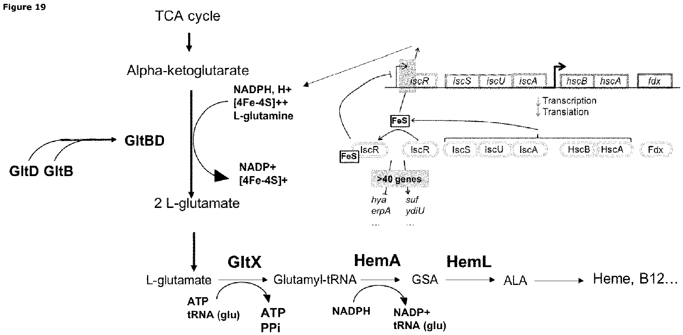

**Figure 19** Cartoon showing the glutamic acid and δ-aminolevulinic acid biosynthesis pathway in *E. coli* from alpha-ketoglutarate, a product of the TCA cycle. The [4Fe-4S] cluster enzyme GltDB, is a complex of two subunits GltB and GltD and catalyses the formation of two L-glutamate molecules from one alpha-ketoglutarate and one L-glutamine molecule. L-glutamate can thereafter be converted to ALA catalyzed by the proteins GltX, HemA and HemL, and then for heme biosynthesis. The isc-operon structure and its role in supplying Fe-S-clusters to the GltBD enzyme is shown. TCA = Tricarboxylic acid; ALA = δ-aminolevulinic acid; NADPH = Nicotinamide adenine dinucleotide phosphate.

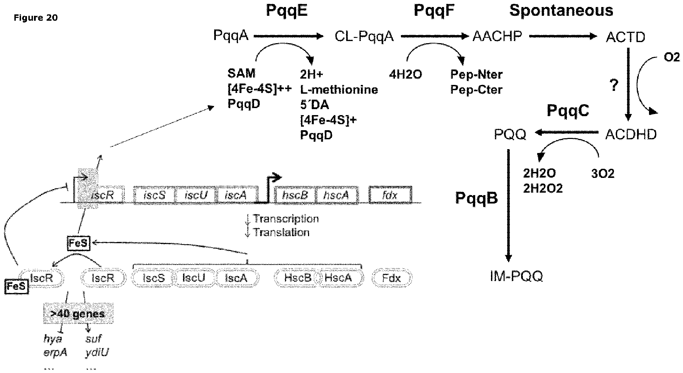

**Figure 20** Cartoon showing the proposed PQQ biosynthetic pathway. The synthesis starts from the short peptide PqqA and its cyclisation by the radical-SAM [4Fe-4S] enzyme PqqE that collaborates with the protein PqqD. The peptide is further cleaved by the enzyme PqqF. After further catalytic steps, of one carried by a yet unknown enzyme, PQQ is finally synthetised and transfered to the bacterial membrane by the enzyme PqqB. CL-PqqA = Crosslinked PqqQ peptide; AACHP = 2-amino-4-[5-(2-amino-2-carboxylatoethyl)-2-hydroxyphenyl]pentanedioate; ACTD = 6-(2-amino-2-carboxylatoethyl)-1,2,3,4-tetrahydroquinoline-2,4-dicarboxylate; ACDHD=6-(2-amino-2-carboxyethyl)-7,8-dioxo-1,2,3,4,7,8-hexahydroquinoline 2,4-dicarboxylate; PQQ = pyrroloquinoline quinone; IM-PQQ = Inter-membrane pyrroloquinoline quinone; SAM = S-adenosyl-methionine; 5'DA = 5'Deoxyadenosine; Pep-Nter = cleaved N-ter part of CL-PqqA; Pep-Cter = cleaved C-ter part of CL-PqqA.

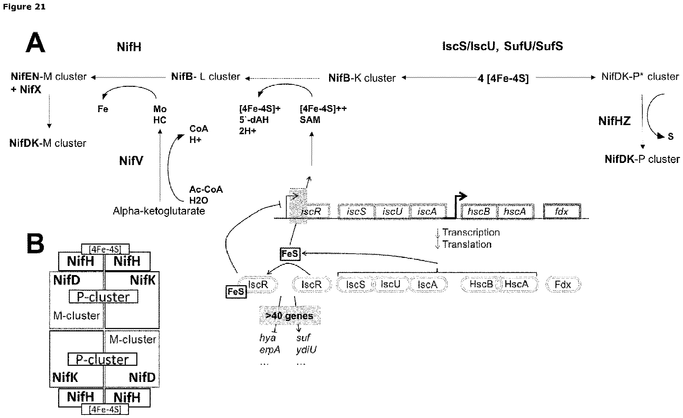

**Figure 21** A) Cartoon showing a biosynthetic pathway for nitrogenase M-cluster and P-cluster assembly, comprising a [Fe8-S9-C] cluster bound to a homocitrate (HC) by a molybdenum atom (Mo). NifB catalyses the incorporation of [Fe8-S9-C] carbon by an S-adenosyl-methionine (SAM) radical mechanism dependent on its own [4Fe-4S] cluster. (B) Cartoon showing the nitrogenase complex. 5'-dAH = 5'-deoxy-adenosine homocysteine. HC = Homocysteine. (B) Cartoon showing the nitrogenase complex. 5'-dAH = 5'-deoxy-adenosine homocysteine.

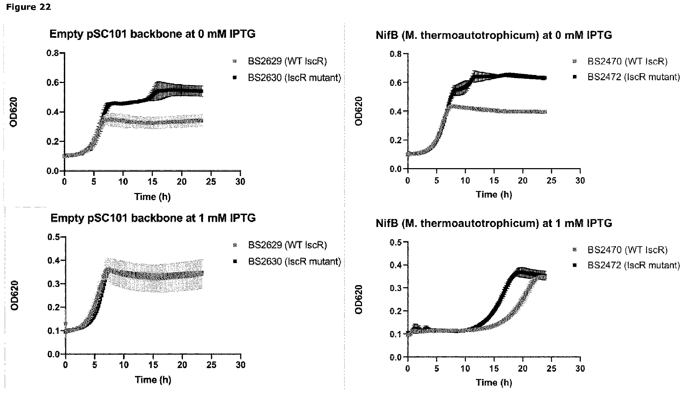

**Figure 22** Diagrams showing the OD₆₂₀ₙₘ of cultures over time, as a measure of cell growth, for *E. coli* strains expressing IscR WT protein (BS2629/BS2470) and IscR mutant protein (BS2630/BS2472) and comprising either a control empty plasmid (left panels) or a plasmid encoding *E. coli* ilvD (right panels) without induction (top panels) or with IPTG induction (bottom panels). The strains with empty plasmid (BS2629/BS2630) and plasmid encoding *E. coli* ilvD (BS2470/BS2472) were induced with 1.0 mM IPTG, respectively (bottom left and right). The error bars denote the standard deviation of four biological replicates.

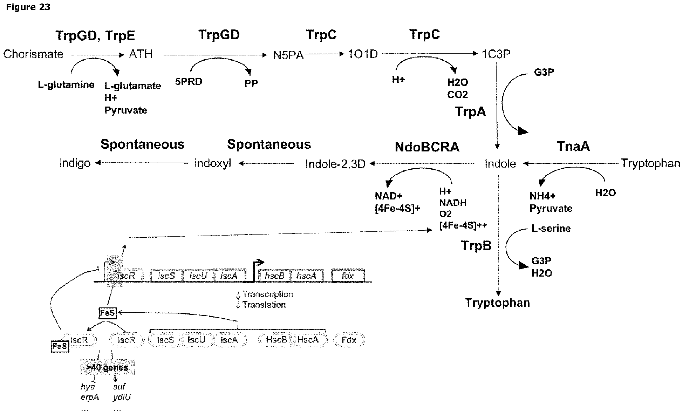

**Figure 23** Cartoon showing *de novo* and tryptophan-dependent biosynthetic pathways for production of indigo in *E. coli.* Both pathways depend on the conversion of indole to indoxyl catalyzed by a monoxygenase such as the Rieske oxygenase complex (e.g. NodBCRA from *Pseudomonas putida*), requiring an [2Fe-2S] cluster. The tryptophan-dependent pathway is mediated by the enzyme TnaA converting this precursor into indole. ATH = Anthranilate; N5PA = N-(5-phosphoribosyl)-anthranilate; 1O1D = 1-(o-carboxyphenylamino)-1'-deoxyribulose 5'-phosphate; 1C3P = (1S,2R)-1-C-(indol-3-yl)glycerol 3-phosphate; Indole-2,3D = cis-indole-2,3-dihydrodiol.

### Definitions:

**Amino acid sequence identity:** The term "sequence identity" as used herein, indicates a quantitative measure of the degree of homology between two amino acid sequences of substantially equal length. The two sequences to be compared must be aligned to give a best possible fit, by means of the insertion of gaps or alternatively, truncation at the ends of the protein sequences. The sequence identity can be calculated as ((Nref-Ndif)100)/(Nref), wherein Ndif is the total number of non-identical residues in the two sequences when aligned and wherein Nref is the number of residues in one of the sequences. Sequence identity calculations are preferably automated using the BLAST program e.g. the BLASTP program (Pearson W.R and D.J. Lipman (1988)) (www.ncbi.nlm.nih.gov/cgi-bin/BLAST). Multiple sequence alignment is performed with the sequence alignment method ClustalW with default parameters as described by Thompson J., et al 1994, available at http://www2.ebi.ac.uk/clustalw/.

Preferably, the numbers of substitutions, insertions, additions or deletions of one or more amino acid residues in the polypeptide as compared to its comparator polypeptide is limited, i.e. no more than 1, 2, 3, 4, 5, 6, 7, 8, 9, or 10 substitutions, no more than 1, 2, 3, 4, 5, 6, 7, 8, 9, or 10 insertions, no more than 1, 2, 3, 4, 5, 6, 7, 8, 9, or 10 additions, and no more than 1, 2, 3, 4, 5, 6, 7, 8, 9, or 10 deletions. Preferably the substitutions are conservative amino acid substitutions: limited to exchanges within members of group 1: Glycine (G), Alanine (A), Valine (V), Leucine (L), Isoleucine (I); group 2: Serine (S), Cysteine (C), Selenocysteine (U), Threonine (T), Methionine (M); group 3: proline (P); group 4: Phenylalanine (F), Tyrosine (Y), Tryptophan (W); Group 5: Aspartate (D), Glutamate (E), Asparagine (N), Glutamine (Q) and Group 6: Arginine (R), histidine (H) and lysine (K).

**Endogenous gene:** is a gene in a prokaryotic cell genome that is homologous in origin to a host prokaryotic cell (i.e. a native gene of the host prokaryotic cell). The endogenous gene may be genetically modified using tools known in the art whereby the genetically modified endogenous gene encodes a mutant polypeptide whose amino acid sequence differs at one or more position from the polypeptide encoded by the parent endogenous gene from which it was derived.

**Genome:** is the genetic material present in a prokaryotic cell; said genome comprising all of the information needed to build and maintain that cell or organism; and includes the genetic material in both chromosome(s) and any episomal genetic element(s) (including plasmid(s)) present within the cell or organism.

**Genetically modified regulatory sequence:** is a regulatory sequence that is operably linked to a gene comprising a protein coding or non-coding sequence; wherein said regulatory sequence is capable of enhancing transcription of said operably linked gene as compared to the native regulatory sequence of said gene; wherein said regulatory sequence is selected from 1) a promoter region sequence and any enhancer element sequence therein; and 2) is-regulatory elements (for example ribosomal binding site) that provide binding sites of transcription factors that are capable of enhancing transcription of said gene.

**GFP:** Green Fluorescent Protein.

**gi number:** (genInfo identifier) is a unique integer which identifies a particular sequence, independent of the database source, which is assigned by NCBI to all sequences processed into Entrez, including nucleotide sequences from DDBJ/EMBL/GenBank, protein sequences from SWISS-PROT, PIR and many others.

**Heterologous gene:** encodes a polypeptide derived from an organism that is different from the organism into which the heterologous gene is expressed.

**Isc pathway:** iron sulfur cluster pathway; encoded by the *isc* operon including the *iscR* gene.

**Multiskan:** filter-based microplate photometer; for measuring absorbance from 96 or 384-well plate formats in the wavelength range of 340 to 850 nm, including 600 - 620nm. Plates are incubated in the photometer at the selected temperature, of up to 50°C. The photometer is supplied by Thermo Scientific.

**Native gene:** endogenous coding or non-coding gene in a bacterial cell genome, homologous to host bacterium.

**Non-native promoter:** in the context of a genetically modified prokaryotic cell of the present invention, is a promoter that is operably-linked to a gene or transgene in said cell, where said promoter would not be found operably-linked to said gene or transgene in a prokaryotic cell found in nature.

**OD:** Optical Density

**Promoter activity:** is the measured strength in arbitory units i.e. measured relative activity of a promoter to drive expression of a reporter gene encoding Fluorescent Protein in *E. coli,* as described by Mutalik et al.2013. A promoter that is capable of enhancing expression of an operably linked endogenous gene as compared to the native regulatory sequence of said gene is defined herein as a promoter having a measured strength of 370 AU, which is the coincidentally the value for promoter apFAB306.

**Transgene:** is an exogenous gene that has been introduced into the genome of a prokaryotic cell by means of genetic engineering. In the context of the present invention, said exogenous gene may have a nucleotide sequence identical to a native or non-native gene of said prokaryotic cell and may encode a protein that is native or non-native to said prokaryotic cell; wherein said genome includes both chromosomal and episomal genetic elements.

**Upregulated endogenous gene:** is an endogenous gene operably linked to a genetically modified regulatory sequence capable of enhancing expression of said endogenous gene as compared to the native regulatory sequence of said gene.

### Detailed description of the invention

A common feature of many biological compounds when produced by fermentation in prokaryotes is that their biosynthesis employs a step catalyzed by, or dependent on, the activity of one or more iron-sulfur (Fe-S) cluster protein. Optimal product titers by microbial fermentation however are hampered by the fact that overexpression of such pathways leads to growth inhibition. For example, to produce biotin the overexpression of the biotin operon, or even a mutant biotin operon insusceptible to feedback regulation by the BirA repressor, led to a strong inhibition of growth (Ifuku, O. *et al.,* 1995). In the absence of any evidence-based explanation for the observed growth inhibition; alternative approaches were needed to identify the cellular factors that may account for the toxicity of elevated synthesis of Fe-S cluster proteins.

The solution to this problem, provided by the present invention, is shown to be equally applicable for enhancing the synthesis of any Fe-S cluster protein in a prokaryotic cell factory (for example *E. coli*). The approach pursued to solve this problem was to generate libraries of *E. coli* cells having evolved genomic diversity due to the accumulation of background mutations generated by imperfect error-correcting polymerases. Cells of such libraries were transformed with a plasmid comprising an IPTG-inducible *bioB* gene expression cassette, encoding a polypeptide of the Fe-S cluster protein, biotin synthase. Candidate mutants were those cells in a library that were capable of growth in the presence of IPTG at a concentration sufficient to induce BioB expression toxicity in the parent *E. coli* strain from which the mutant cells were derived.

The genetic basis for the growth of the selected BioB-expressing mutant strains was established by whole genome sequencing. Surprisingly three of the strains were found to have mutations in the native Iron Sulfur Cluster Regulator gene (*iscR*); which encodes a pleiotropic transcription factor (IscR) [SEQ ID No.: 2]. Fe-S clusters are co-factors of many proteins and essential enzymes, endowing them with diverse biochemical abilities that are not solely required for the synthesis of S-containing compounds, but also as regulatory proteins in the form of sensors for redox- or iron-related stress conditions, as well as for mediating electron transfer, radical or non-redox reactions, and sulfur donation.

IscR exists in two states, either as an Fe-S cluster holo-protein, or as the apo-protein without the Fe-S cluster. Assembly of the Fe-S cluster of IscR is catalyzed by the Isc pathway encoded by the *isc* operon. The *isc* operon encodes firstly the regulator (IscR), followed by a cysteine desulfurase (IscS), a scaffold (IscU), an A-type protein (IscA), a DnaJ-like co-chaperone (HscB), a DnaK-like chaperone (HscA) and a ferredoxin (Fdx). In addition to being essential for the assembly of the IscR holoenzyme, the Isc pathway is the primary pathway for Fe-S cluster biogenesis in *E. coli* (Figure 1B).

The ratio between the two forms of IscR is determined by the cellular level of [2Fe-2S] clusters, which in turn is influenced by several factors including iron- and oxygen levels (Py, B. & Barras, 2010). Under iron-rich conditions, IscR exists mainly as the holoenzyme, which then acts as a transcriptional repressor of the *isc* operon. However, under iron-low conditions (low level of [2Fe-2S] clusters), IscR returns to its apo-protein state, allowing transcription of the *isc* operon. In its apo-protein state, IscR serves as an activator of the *sufABCDSE* operon, which catalyzes Fe-S cluster biogenesis under oxidative stress.

In addition to regulating expression of the two Fe-S-cluster assembly systems in *E. coli,* IscR regulates >40 genes involved in diverse mechanisms of action such as improved assembly of Fe-S clusters (e.g., *suf* operon), oxidative stress mechanisms (e.g. *sodA*)*,* specific and global regulators (e.g. *yqjI* and *soxS*), amino acid biosynthesis (e.g *argE*) and a range of genes with unknown functions. The role of IscR is further complicated by the fact that the IscR regulatory landscape changes between aerobic and anaerobic conditions (Giel et al., 2006).

In view of the homeostatic role of IscR and its role in global gene regulation; the consequences of any modification of its regulatory properties are unpredictable and probably profound for cellular metabolism. Furthermore, cellular conditions where Fe-S cluster biogenesis is increased, due to elevated expression of both the sulfur formation (suf) and isc pathways creates the risk that the accumulated Fe-S clusters generate peroxide radicals by Fenton reactions.

In this light, it was highly unexpected that IscR should be found so important for the activity and toxicity of a cellular Fe-S cluster protein, such as BioB, as demonstrated by the three isolated individual mutants. Furthermore, the impact of the mutant IscR protein on end-product (e.g. biotin) biosynthesis was unexpected, since over-expression of the *isc* operon or *suf* operons giving an increased capacity to synthesize and assemble Fe-S clusters was not found to enhance biotin production in cells over-expressing *bioB* (see example 1, Figure 8). Furthermore, removal of cellular iscR regulation by knock-out of the *iscR* gene also failed to enhance biotin production in the cell (see example 1, Figure 10).

The three different mutations in the IscR protein that eliminated the toxicity of BioB expression in the mutant cells, were single amino acid substitutions of the amino acids L15 [SEQ ID No.: 16], C92 [SEQ ID No.: 18] and H107 [SEQ ID No.: 20] (Figure 4). Two of the three mutations correspond precisely to those residues in IscR, each of which is known to be essential for the formation of the IscR holo-protein. IscR, as seen in *E. coli,* has an unusual Fe-S cluster ligation mechanism, whereby the residues essential for Fe-S cluster ligation are C92, C98, and C104, as well as H107. This atypical ligation may confer a lower stability of the holoenzyme state of IscR relative to other Fe-S proteins that in turn accounts for the switch to the apo-protein state during low Fe-S conditions (Fleischhacker et al., 2012).

While not wishing to be bound by theory, this suggests that homeostatic control of Fe-S cluster biogenesis and global gene regulation required for cell growth are uniquely preserved in cells expressing a mutant *iscR* gene of the invention, while facilitating the assembly of Fe-S cluster containing enzymes, even during their over-expression.

In summary, the inventors have identified a mutant *iscR* gene encoding a mutant IscR polypeptide, characterized by the lack of one or more of amino acid residues required for ligation of Fe-S clusters, such that the expressed mutant IscR protein exists solely in the apo-protein form. Synthesis of iron-sulfur cluster containing polypeptides is shown to constitute a significant bottleneck in efforts to enhance production of the biochemical products of pathways requiring such Fe-S proteins in prokaryotes. The solution to this problem, as provided by the present invention, is facilitated by over-expression of these Fe-S polypeptides in a cell factory comprising a gene encoding a mutant IscR polypeptide that exists only in the apo-protein state.

The various embodiments of the invention are described in more detail below.

### I A genetically modified prokaryotic cell for over-expression of Fe-S polypeptide(s)

According to a first embodiment, the invention provides a genetically modified prokaryotic cell comprises a genetically modified iscR gene encoding a mutant IscR, as well as either at least one transgene, or at least one endogenous gene operably linked to a genetically modified regulatory sequence capable of enhancing expression of said endogenous gene (herein called an upregulated endogenous gene); wherein the transgene or endogenous gene encodes any Fe-S cluster polypeptide excluding biotin synthase (EC: 2.8.1.6), lipoic acid synthase (EC: 2.8.1.8), HMP-P synthase (EC: 4.1.99.17), and tyrosine lyase (EC: 4.1.99.19).

The mutant IscR polypeptide is derived from a wild-type member of a family of IscR polypeptides, whereby the mutant IscR is characterized by an amino acid sequence comprising at least one amino acid substitution when compared to its wild-type parent IscR polypeptide; and as a consequence of said at least one substitution the mutant IscR polypeptide, when expressed in a genetically modified prokaryotic cell of the invention, exits only as an apoprotein (i.e. it is unable to exist in a Fe-S cluster holoprotein state). The prokaryotic cell may comprise a genetically modified endogenous iscR gene; or alternatively a transgene encoding a mutant IscR polypeptide but where the native IscR gene is inactive. When a Fe-S cluster polypeptide is over-expressed in a genetically modified prokaryotic cell of the invention, the cells exhibit enhanced growth, and the activity of the respective Fe-S cluster polypeptide is enhanced, as compared to over-expression in a prokaryotic cell having gene encoding a wild-type IscR polypeptide.

The Fe-S cluster polypeptide, encoded by a gene expressed in a prokaryotic cell of the invention, has one of a wide diversity of biochemical abilities, and may be one selected from the group set out in Table A:

| **TABLE A** | | | | |
|---|---|---|---|---|
| **Uniprot Entry** | **Protein names** | **EC number** | **Molecule class** | **Cross-reference (RefSeq)** |
| P32131 | Oxygen-independent coproporphyrinogen III oxidase | 1.3.98.3 | Porphyrins and derivatives | WP_000116090.1 |
| P11458 | Quinolate synthase | 2.5.1.72 | Quinolate and derivatives | WP_000115290.1 |
| M4XAX7 | Precorrin-3B synthase | 1.14.13.83 | Porphyrins and derivatives | WP_015475360.1 |
| P05791 | Dihydroxy-acid dehydratase | 4.2.1.9 | 2-keto-isovalerate and derivatives and 2-keto-3-methyl-valerate and derivatives | WP_001127399.1 |
| O67496 | 4-hydroxy-3-methylbut-2-en-1-yl diphosphate synthase | 1.17.7.3 | Isoprenoids and derivatives | WP_000551807.1 |
| P62623 | 4-hydroxy-3-methylbut-2-enyl diphosphate reductase | 1.17.7.4 | Isoprenoids and derivatives | WP_001166395.1 |
| P09831 | glutamate synthase [NADPH] large chain | 1.4.1.13 | Glutamate and derivatives | WP_001300352.1 |
| P09832 | Glutamate synthase [NADPH] small chain | 1.4.1.13 | Glutamate and derivatives | WP_000081674.1 |
| P27507 | PqqA peptide cyclase | 1.21.98.4 | Pyrroloquinoline quinone and derivatives | WP_004184158.1 |
| C1DMB1 | Nitrogenase FeMo cofactor biosynthesis protein | - | Nitrogen and derivatives | WP_012703541 |
| P0A110 | Naphthalene 1,2-dioxygenase system, large oxygenase component | 1.14.12.12 | Indigo and derivatives | WP_011117400.1 |
| P0A112 | Naphthalene 1,2-dioxygenase system, small oxygenase component | 1.14.12.12 | Indigo and derivatives | WP_011117401.1 |
| P0A185 | Naphthalene 1,2-dioxygenase system, ferredoxin component | 1.18.1.7 | Indigo and derivatives | WP_011117470.1 |
| Q52126 | Naphthalene 1,2-dioxygenase system reductase component | 1.18.1.7 | Indigo and derivatives | WP_011117469.1 |
| A0A1B7K 3G1 | NADH-ubiquinone oxidoreductase subunit E | 1.6.5.3 | Xanthine and derivatives | WP_064543883.1 |
| A0A085A 9T8 | NADH-ubiquinone oxidoreductase chain E | 1.6.5.3; 1.6.99.5 | Xanthine and derivatives | WP_038156621.1 |
| A0A1J5N EE5 | Limonene hydroxylase | 1.1.1.144; 1.1.1.243; 1.14.13.49 | Limonene and derivatives | WP_011393389.1 |
| Q47914 | Tetrachlorobenzoquinone | 1.1.1.404 | Benzoquinone and derivatives | WP_013849281.1 |
| Q8E8Z1 | Formate dehydrogenase molybdopterin-binding subunit FdhA | 1.1.5.6 | Hydrogenase, hydrogen or derivatives | WP_011074128.1 |
| A0A0K0H QP3 | Formate dehydrogenase major subunit | 1.2.1.2 | Hydrogenase, hydrogen or derivatives | WP_025799130.1 |
| A0A0P1G 7P8 | Formate dehydrogenase H | 1.1.99.33 | Hydrogenase, hydrogen or derivatives | WP_058289583.1 |
| I9ZCE7 | Quinone-reactive Ni/Fe hydrogenase small subunit (HydA) | 1.12.1.2 | Hydrogenase, hydrogen or derivatives | WP_000499095.1 |
| Q8U2E5 | Sulfur reductase subunit (HydB) | 1.12.98.4 | Hydrogenase, hydrogen or derivatives | WP_011012026.1 |
| D5E3H2 | Mevastatin hydroxylase | 1.14.-.- | Mevastatin and derivatives | WP_013060111.1 |
| O85673 | Anthranilate 1,2-dioxygenase large subunit | 1.14.12.1 | Anthranilate and derivatives | WP_004928945.1 |
| P07769 | Benzoate 1,2-dioxygenase subunit alpha | 1.14.12.10 | Benzoates or derivatives | WP_004925496.1 |
| P37333 | Biphenyl dioxygenase subunit alpha | 1.14.12.18 | Biphenyls and derivatives | WP_011494299.1 |
| P0ABR6 | 3-phenylpropionate/cinnamic acid dioxygenase | 1.14.12.19 | Cinnamic acid and derivatives | WP_000211172.1 |
| Q8G8B6 | Carbazole 1,9-dioxygenase | 1.14.12.22 | Carbazole and derivatives | WP_011077878.1 |
| Q51974 | p-cumate 2,3-dioxygenase system | 1.14.12.25 | p-cumeate and derivatives | WP_012052617.1 |
| A5W4F2 | Benzene and toluene dioxygenase | 1.14.12.3; 1.14.12.11 | Benzene and derivatives Toluene and derivatives | WP_012052601.1 |
| H9N290 | Methylxanthine N3-demethylase | 1.14.13.179 | Methylxanthine and derivatives | WP_046819639.1 |
| F0KFI5 | Carnitine monooxygenase oxygenase subunit | 1.14.13.239 | Carnitine and derivatives | WP_002120432.1 |
| P22868 | Methane monooxygenase component C | 1.14.13.25 | Methanol and derivatives | WP_010960487.1 |
| A0A162M ZU2 | Limonene hydroxylase | 1.14.13.48 | Limonene and derivatives | WP_068747318.1 |
| Q7WTJ2 | Phenol hydroxylase | 1.14.13.7 | Catechol and derivatives | WP_014206216.1 |
| P9WPP2 | Methyl-branched lipid omega-hydroxylase | 1.14.15.14 | Methyl-branched lipids and derivatives | WP_003917608.1 |
| A0A059 WLZ7 | Chloroacetanilide N-alkylformylase | 1.14.15.23 | Chloroacetanilide and derivatives | WP_051743970.1 |
| P9WPL5 | Steroid monooxygenase | 1.14.15.28 | Steroids and derivatives | WP_003900082.1 |
| P63710 | Steroid monooxygenase | 1.14.15.29 | Steroids and derivatives | WP_003419304.1 |
| P71875 | 3-ketosteroid-9-alpha-monooxygenase | 1.14.15.30 | Steroids and derivatives | WP_003419211.1 |
| Q82IY3 | Pentalenene oxygenase | 1.14.15.32 | pentalenene and derivatives | WP_010984430.1 |
| Q00441 | 6-deoxyerythronolide B hydroxylase | 1.14.15.35 | Erythromycin and derivatives | WP_009950397.1 |
| P17055 | Spheroidene monooxygenase | 1.14.15.9 | Spheroidene and derivatives | WP_013066422.1 |
| Q9FCA6 | Biflaviolin synthase CYP158A2 | 1.14.19.69 | Bioflaviolin and derivatives | WP_003977624.1 |
| P9WPP7 | Mycocyclosin synthase | 1.14.19.70 | Mycolysin and derivatives | WP_003411685.1 |
| B1WQF1 | 4-hydroxy-3-methylbut-2-en-1-yl diphosphate synthase | 1.17.7.1 | Isoprenoids and derivatives | WP_009544989.1 |
| Q8GI14 | Carbazole 1,9a-dioxygenase | 1.18.1.3 | Carbazole and derivatives | WP_011077884.1 |
| G9F1Y9 | Cinnamate reductase | 1.3.1.- | Cinnamic acid and derivatives | WP_003493847.1 |
| Q7M826 | 8-methylmenaquinol:fumarate reductase iron-sulfur subunit | 1.3.5.- | Menaquinol and derivatives | WP_011139713.1 |
| P45866 | Probable iron-sulfur-binding oxidoreductase FadF | 1.3.8.7 | Long-chain (2E)-enoyl-CoA | WP_003244084.1 |
| Q8F1F5 | CRISPR-associated exonuclease Cas4/endonuclease Cas1 fusion | 3.1.12.1 | CRISPR enzymes and derivatives | WP_002068643.1 |
| Q18CP4 | 4-hydroxyphenylacetate decarboxylase small subunit | 4.1.1.83 | Methylphenol and derivatives | WP_003425410.1 |
| A0A0U5K 4Y8 | Cyclic pyranopterin monophosphate synthase | 4.1.99.18 | Pyranopterin and derivatives | WP_057572494.1 |
| Q2RHR5 | 5-hydroxybenzimidazole synthase | 4.1.99.23 | Hydroxybenzoimidazole and derivatives | WP_011393224.1 |
| Q8EJW3 | 2-methylcitrate dehydratase | 4.2.1.117 | Isocitrate and derivatives | WP_011070708.1 |
| P14407 | Fumarate hydratase class I, anaerobic | 4.2.1.2; 4.2.1.81 | Fumarate and derivatives | WP_000066707.1 |
| P0AC34 | Fumarate hydratase class I, aerobic | 4.2.1.2; 5.3.2.2 | Fumarate and derivatives | WP_000066639.1 |
| Q2A1K3 | Aconitate hydratase A | 4.2.1.3; | Iisocitrate and derivatives | WP_003017291.1 |
| Q8XBK7 | L(+)-tartrate dehydratase subunit alpha | 4.2.1.32 | Oxaloactetate and derivatives | WP_000986788.1 |
| Q8U0C0 | 3-isopropylmalate dehydratase large subunit | 4.2.1.33 | Isopropyl malate and derivatives | WP_011012825.1 |
| P81291 | Isopropylmalate/citramalate isomerase large subunit | 4.2.1.35; 4.2.1.31 | Isopropyl malate and derivatives | WP_010870000.1 |
| P71349 | L-serine dehydratase | 4.3.1.17 | Serine and derivatives | WP_005693026.1 |
| A9A9J5 | L-cysteine desulfidase | 4.4.1.28 | L-cysteine and derivatives | WP_012193958.1 |
| Q53U14 | Neomycin C epimerase | 5.1.3.- | Neomycin and derivatives | WP_031132490.1 |
| Q89B32 | L-lysine 2,3-aminomutase | 5.4.3.- | Beta-lysines and derivatives | WP_011091166.1 |
| Q8TUB9 | 3-methylornithine synthase (Pyrrolysine) | 5.4.99.58 | Methyl-ornithine and derivatives | WP_011020212.1 |
| A0A084G FF7 | Xanthine dehydrogenase | 1.2.99.7; 1.3.7.9 | Xanthines and derivatives | XP_016645868.1 |
| Q0JCU7 | Zeaxanthin epoxidase | 1.14.15.21 | Xanthine and derivatives | XP_015636352.1 |
| P47990 | Xanthine dehydrogenase/oxidase | 1.17.1.4; 1.17.3.2 | Xanthine and derivatives | NP_990458.1 |
| Q07973 | Vitamin D3 hydroxylase | 1.14.15.16 | Vitamin D and derivatives | XP_016883182.1 |
| O35084 | Vitamin D3 hydroxylase | 1.14.15.18 | Vitamin D and derivatives | NP_034139.2 |
| P18326 | Vitamin D3 dihydroxylase | 1.14.15.22 | Vitamin D and derivatives | - |
| P12609 | Vanillate O-demethylase oxygenase | 1.14.13.82 | Vanilin and derivatives | - |
| Q3C1D2 | Terephthalate 1,2-dioxygenase | 1.14.12.15 | Terephtalate and derivatives | - |
| O52379 | Salicylate 5-hydroxylas | 1.14.13.172 | Salicylate and derivatives | - |
| P51135 | Cytochrome b-c1 complex subunit Rieske-5 | 1.10.2.2 | Quinones and derivatives | NP_001312589.1 |
| O87605 | Cytochrome P450 monooxygenase PikC | 1.14.15.33 | Pikromycin and derivatives | - |
| Q05183 | Phthalate 4,5-dioxygenase oxygenase subunit | 1.14.12.7 | Phthalate and derivatives | - |
| Q52185 | Phenoxybenzoate dioxygenase | 1.14.12.- | Phenoxybenzoate and derivatives | - |
| Q0QLF3 | Nicotinate dehydrogenase small FeS subunit | 1.17.1.5 | Nicotinate and derivatives | - |
| Q9X404 | Methanesulfonate monooxygenase hydroxylase | 1.14.13.111 | Methanesulfonate and derivatives | - |
| Q0QLF7 | 6-hydroxynicotinate reductase | 1.3.7.1 | Hydroxynicotinate and derivatives | - |
| A0A093D J52 | Dimethyl sulfoxide reductase | 1.8.5.3 | Dimethyl sulfoxide and derivatives | - |
| Q02318 | Vitamin D3 25-hydroxylase | 1.14.15.15 | Steriods and derivatives | NP_000775.1 |
| Q51601 | 2-halobenzoate 1,2-dioxygenase large subunit | 1.14.12.13 | Catechol or derivatives | - |
| P00183 | Camphor 5-monooxygenase | 1.14.15.1 | Camphor and derivatives | - |
| P16640 | Putidaredoxin reductase CamA (Pdr) | 1.18.1.5 | Camphor and derivatives | - |
| H9N289 | Methylxanthine N1-demethylase NdmA | 1.14.13.178 | Caffeine and derivatives | - |
| Q8G907 | Butirosin biosynthesis protein N | 1.1.99.38 | Butirosin or derivatives | - |
| P54973 | Beta-carotene hydroxylase | 1.14.15.24 | beta-carotenes and derivatives | - |
| Q8GMH2 | 2-amino-4-deoxychorismate dehydrogenase | 1.3.99.24 | Anthranilate and derivatives | - |
| Q5SK48 | Aminodeoxyfutalosine synthase | 2.5.1.120 | Menaquinone and derivatives | |
| Q60AV6 | hopanoid C-3 methylase | 2.1.1.- | Hopanoid and derivatives | WP_010960068.1, |
| Q5SK48 | aminofutalosine synthase (cofactor biosynthesis - menaquinone via futalosine) | 2.5.1.120 | Futalosin and derivatives | WP_011172898.1, |
| Q58826 | FO synthase, CofH subunit (cofactor biosynthesis - F420) | 2.5.1.147 | Cofactor F420 and derivatives | WP_010870949.1, |
| P69848 | GTP 3',8-cyclase (molybdenum cofactor) | 4.1.99.22 | GTP and derivatives | WP_000230173.1, |
| Q57888 | FO synthase, CofG subunit (cofactor biosynthesis - F420) | 4.3.1.32 | Cofactor F420 and derivatives | WP_064496498.1, |
| O31677 | 7-carboxy-7-deazaguanine (CDG) synthase | 4.3.99.3 | Guanine and derivatives | WP_003232460.1, |

A genetically modified regulatory sequence capable of enhancing expression of said endogenous gene encoding an Fe-S cluster polypeptide as compared to the native regulatory sequence of said endogenous gene includes both a promoter sequence (for example having a measured promoter strength of at least 370 AU (see definitions); or a RBS conferring increased transcription (see example 7).

### II A genetically modified prokaryotic cell for production of heme

According to a further embodiment, the present invention provides a genetically modified prokaryotic cell capable of producing enhanced levels of heme and hemoproteins. The prokaryotic cell is genetically modified to express a mutant IscR in substitution for a wild type IscR, as well as comprising a transgene or up-regulated endogenous *hemN* gene (i.e an endogenous *hemN* gene operably linked to a genetically modified regulatory sequence capable of enhancing expression of said endogenous gene, as described in section I) encoding a HemN polypeptide having oxygen-independent coproporphyrinogen III oxidase (EC: 1.3.98.3) activity. Optionally, the genetically modified prokaryotic cell may further comprise one or more additional transgenes or upregulated endogenous genes encoding polypeptides that catalyze additional steps in the heme pathway (Figure 12). An increase in the levels of those polypeptides that catalyze steps in the heme pathway enhances the synthesis of both intermediates in the heme pathway, and the end product of the pathway (heme) in the prokaryotic cell. Optionally, the genetically modified prokaryotic cell may further comprise one or more additional transgenes or upregulated endogenous genes (as defined herein) encoding polypeptides that use heme as a cofactor for the production of heme-containing proteins as well as other molecules whose production is dependent on such hemoproteins, e.g. cytochrome P450s, catalases, peroxidases and myoglobins (Figure 12B).

The mutant IscR polypeptide is derived from a wild-type member of a family of IscR polypeptides, whereby the mutant IscR as compared to a corresponding non-mutant IscR polypeptide has an increased apoprotein:holoprotein ratio in the cell.

In one embodiment, mutant IscR polypeptide has an amino acid sequence comprising at least one amino acid modification (by substitution, addition or deletion), when compared to its wild-type parent IscR polypeptide and can only exit as an apoprotein. In one embodiment the amino acid sequence of a wild-type member of a family of IscR polypeptides has at least 70, 75, 80, 85, 90, 95, 96, 98, 100% amino acid sequence identity to a sequence selected from any one of: SEQ ID No.: 2, 4, 6, 8, 10, 12 and 14, 15-26. The amino acid sequence of the mutant IscR polypeptide according to the invention differs from the amino acid sequence of the corresponding wild-type IscR polypeptide from which it was derived by at least one amino acid substitution; wherein said substitution is selected from L15X, C92X, C98X, C104X, and H107X; wherein X, the substituting amino acid, is any amino acid other than the amino acid found at the corresponding position in the wild type IscR from which the mutant was derived.

In alternative embodiments, the amino acid substitution in the mutant IscR is selected from either L15X, wherein X is any amino acid other than L, more preferably X is selected from phenylalanine (F), tyrosine (Y), methionine (M) and tryptophan (W); C92X, wherein X is any amino acid other than C, more preferably X is selected from tyrosine (Y), alanine (A), methionine (M), phenylalanine (F) and tryptophan (W); C98X, wherein X is any amino acid other than C, more preferably X is selected from alanine (A), valine (V), isoleucine (I), leucine (L), phenylalanine (F) and tryptophan (W); Cys104X, wherein X is any amino acid other than C, more preferably X is selected from alanine (A), valine (V), isoleucine (I), leucine (L), phenylalanine (F), and tryptophan (W); and His107X, wherein X is any amino acid other than H, more preferably X is selected from alanine (A), tyrosine (Y), valine (V), isoleucine (I), and leucine (L). For example, the amino acid substitution in the mutant IscR may be selected from among L15F, C92Y, C92A, C98A, Cys104A, H107Y, and H107A.

The mutant IscR expressed by the genetically modified prokaryotic cell of the invention (instead of a wild type IscR), is encoded by a genetically modified gene, located in the genome of the cell, either on the chromosome or on a self-replicating plasmid. The genetically modified *iscR* gene in the chromosome can be located in the genome at the same position of the wild-type *iscR* gene in the native genome. The genome of the genetically modified prokaryotic cell of the invention lacks a native wild type *iscR* gene, since the native wild type *iscR* gene is either deleted or directly substituted by the genetically modified *iscR* gene. The promoter driving expression of the genetically modified *iscR* gene may be the native promoter of the wild type *iscR* gene from which the genetically modified *iscR* gene was derived or replaced by. Alternatively, the promoter may be a heterologous constitutive or inducible promoter. When the promoter is a heterologous constitutive promoter, then a suitable promoter includes: *apFab* family [SEQ ID Nos.:230-232] while a suitable inducible promoter includes: *pBad* (arabinose-inducible) [SEQ ID No.:38] and *LacI* [SEQ ID No.:40]. Suitable terminators include members of the apFAB terminator family including [SEQ ID No.: 41].

A polypeptide having oxygen-independent coproporphyrinogen III oxidase synthase (EC: 1.3.98.3) activity, according to the invention, catalyzes the conversion of coproporphyrin III into protoporphyrinogen IX, consuming two molecules of the co-factor S-adenosyl-methionine (SAM) (see Figure 12). The members of this family of HemN polypeptides are encoded by hemN genes found in bacteria belonging to a wide range of genera. The amino acid sequence of the polypeptide having oxygen-independent coproporphyrinogen III oxidase synthase (EC: 1.3.98.3) activity has at least 70, 75, 80, 85, 90, 95, 96, 98, 100% amino acid sequence identity to a sequence selected from any one of: SEQ ID No.:96 (origin: *Escherichia coli);* SEQ ID No.:97 (origin: Synechocystis sp.); SEQ ID No.: 98 (origin: *Thermus Aquaticus*); SEQ ID No.:99 (origin: *Candidatus Heimdallarchaeota*); SEQ ID No.:100 (origin: *Cryomorphaceae bacterium*); SEQ ID No.:101 (origin: *Thermotoga neapolitana*); SEQ ID No.:102 (origin: *Rhodothermus marinus*); SEQ ID No.:103 (origin: *Aquifex aeolicus);* SEQ ID No.:104 (origin: *Cupriavidus necator*); SEQ ID No.:105 (origin: *Lactococcus lactis phingomonas paucimobilis*); and SEQ ID No.:106 (origin: *Bacillus subtilis*).

The polypeptides that are encoded by additional transgenes or additional upregulated endogenous genes (as defined herein) in the genetically modified prokaryotic cell, and whose activity serves to enhance the synthesis of both intermediates and products of the heme pathway, are as follows:
a) a polypeptide having glutamyl-tRNA reductase activity (**HemA;** EC:1.2.1.70) activity; such as a polypeptide with an amino acid sequence having 80, 85, 90, 95 or 100% sequence identity to SEQ ID No.:107 (origin: *Escherichia coli);* or a heme feed-back insensitive mutant HemA polypeptide having a C170A substitution in SEQ ID No: 107;
b) a polypeptide having glutamate-1-semialdehyde 2,1-aminomutase activity (**HemL;** EC 5.4.3.8), such as a polypeptide with an amino acid sequence having 80, 85, 90, 95 or 100% sequence identity to SEQ ID No.: 108(origin: *Escherichia coli);*
c) a polypeptide having delta-aminolevulinic acid dehydratase activity (**HemB;** EC:4.2.1.24) such as a polypeptide with an amino acid sequence having 80, 85, 90, 95 or 100% sequence identity to SEQ ID No.: 109(origin: *Escherichia coli);*
d) a polypeptide having porphobilinogen deaminase activity (**HemC;** EC:2.5.1.61), such as a polypeptide with an amino acid sequence having 80, 85, 90, 95 or 100% sequence identity to SEQ ID No.:110(origin: *Escherichia coli);*
f) a polypeptide having uroporphyrinogen III methyltransferase activity (**HemD;** EC:4.2.1.75) such as a polypeptide with an amino acid sequence having 80, 85, 90, 95 or 100% sequence identity to SEQ ID No.:111(origin: *Escherichia coli);*
g) a polypeptide having uroporphyrinogen decarboxylase activity (**HemE;** EC 4.1.1.37); such as a polypeptide with an amino acid sequence having 80, 85, 90, 95 or 100% sequence identity to SEQ ID No.:112 (origin: *Escherichia coli);*
h) a polypeptide having protoporphyrinogen IX dehydrogenase (menaquinone) activity (**HemG;** EC: 1.3.5.3); such as a polypeptide with an amino acid sequence having 80, 85, 90, 95 or 100% sequence identity to SEQ ID No.:113 (origin: *Escherichia coli);*
i) a polypeptide having protoporphyrin ferrochelatase activity (**HemH;** EC: 4.99.1.1); such as a polypeptide with an amino acid sequence having 80, 85, 90, 95 or 100% sequence identity to SEQ ID No.:114 (origin: *Escherichia coli*)

The genetically modified prokaryotic cell of the invention can also make use of heme as a cofactor for the production of heme-containing proteins or molecules whose production is dependent on hemoproteins. Such hemoproteins include the enzymes: cytochrome P450 monooxygenase (EC: 1.14.-.-); polypeptides having Fe-S clusters (EC: 1.14.15.-), peroxidase (EC: 1.11.1.-), perooxygenase (EC: 1.11.2.-), catechol oxidase (EC: 1.10.3.-), hydroperoxide dehydratase (EC: 4.2.1.-), tryptophan 2,3-dioxygenase (EC: 1.13.11.-), and cytochrome c oxidase (EC: 1.9.3.-). Additionally the hemoproteins myoglobin, hemoglobin, neuroglobin, cytoglobin, leghemoglobin can be produced using the genetically modified prokaryotic cell of the invention.

Accordingly, the prokaryotic cell may comprise transgenes or upregulated endogenous genes (as defined herein) encoding a HemN polypeptide, and preferably also a HemB polypeptide, as well as comprising one or more additional transgenes or upregulated endogenous genes (as defined herein) encoding such hemopolypeptides. For example, cytochrome P450s catalyze the stereoselective insertion of two hydroxy groups into indole in two consecutive enzymatic steps into cis-indole-2,3-dihydrodiol, which spontaneously oxidizes to indigo. Accordingly, in one embodiment the cytochrome P450 comprises a monooxygenase, e.g. BM3 having monooxygenase activity (EC: 1.14.14.1), which is a self-sufficient enzyme composed of a single polypeptide with a heme domain and a reductase domain having NAD(P)H reductase activity (EC: 1.6.2.4).

A P450-BM3 polypeptide having monooxygenase activity (EC: 1.14.14.1) and NAD(P)H reductase activity (EC: 1.6.2.4), is characterized by an amino acid sequence having at least 70, 75, 80, 85, 90, 95, 96, 98, 100% amino acid sequence identity to: SEQ ID No.: 115 (origin: *Bacillus megaterium*)*.* The amino acid sequence of a mutant derivative of said P450-BM3 (called P450-BM3*) has the following amino acid mutations: A74G, F87V, L188Q and V445A; which enable P450 BM3* to oxidise indole into indigo.

When the gene(s) encoding HemN together with any additional polypeptides that catalyze the above listed additional steps in the heme pathway, as well as any cytochrome P450 polypeptide, are transgenes, they are located in the genome of the genetically modified prokaryotic cell, either integrated into the cell chromosome or on a self-replicating plasmid. The transgenes encoding said heme pathway enzymes (HemALBCDEGH) and/or cytochrome P450 polypeptide may be present in the genome within one or more operon.

The promoter driving expression of the transgenes is preferably a non-native promoter, which may be a heterologous constitutive-promoter or an inducible-promoter. When expression driven by the promoter is constitutive, then a suitable promoter includes apFab family [SEQ ID Nos.:93] while a suitable inducible promoter includes: pBad (arabinose-inducible) [SEQ ID No.:38] and lac promoter lac p, which is regulated by repressor lacI [SEQ ID No.:40]. Suitable terminators include members of the apFAB terminator family including [SEQ ID No.: 41]. The selected promoter and terminator may be operably linked to the coding sequence for HemN; and to the coding sequences of the one or more coding sequences for the HemA, L, C, B, C, D, E, G, and H polypeptides and cytochrome P450 polypeptide or may be operably linked to the one or more operon encoding the selected Hem polypeptides.

### III A method for producing and detecting heme using a genetically modified bacterium according to the invention

Heme can be produced and exported using genetically modified prokaryotic cells of the invention (e.g. genetically modified *E. coli* cells) by introducing the cells into a culture medium suitable for supporting growth as well as comprising a carbon source suitable for the biosynthesis of heme; and finally recovering the heme produced by the culture, as illustrated in Example 3, Figure 12A.

The genetically modified prokaryotic cells of the invention comprising a transgene encoding a HemN polypeptide, and preferably a transgene encoding HemB, will produce enhanced levels of heme when the supplied carbon source includes aminolaevulinic acid (ALA). When the genetically modified prokaryotic cells of the invention additionally comprise transgenes encoding each of HemA, L, C, B, C, D, E, G, and H polypeptides, they will produce heme when the supplied carbon source is selected from among glucose, maltose, galactose, fructose, sucrose, arabinose, xylose, raffinose, mannose, and lactose (example 3, Figure 12A).

A method for producing and quantifying heme and porphyrin produced by a genetically modified prokaryotic cell of the invention is described in example 3.01. Production of heme under anaerobic conditions, employing the HemN catalyzed pathway has the additional advantage of (i) reducing equipment cost (air compressor, gas processing); (ii) reducing electricity cost (air management, reduced stirring needs, reducing cooling); and (iii) reducing contamination risks due to unfavorable conditions for opportunistic organisms.

### IV A genetically modified prokaryotic cell for production of vitamin B₃ complex: nicotinamide riboside (NR), nicotinic acid (NA), nicotinamide (NA), nicotinamide mononucleotide (NMN), nicotinamide adenine dinucleotide (NAD), or the intermediate quinolate

According to a further embodiment, the present invention provides a genetically modified prokaryotic cell capable of producing enhanced levels of B₃ vitamins and/or the intermediate quinolate. The prokaryotic cell is genetically modified to express a mutant IscR, according to the invention (see Section I and II), in substitution for a wild type IscR, as well as comprising a transgene or up-regulated nadA endogenous gene (i.e an endogenous nadA gene operably linked to a genetically modified regulatory sequence capable of enhancing expression of said endogenous gene, as described in section I) encoding a NadA polypeptide having quinolate synthase activity (EC: 2.5.1.72). NadA is an [4Fe-4S] cluster-dependent enzyme that catalyzes the condensation and cyclisation of 2-iminosuccinate with dihydroxyacetone to synthesize quinolate (Figure 13). The growth of cells over-expressing this enzyme is shown to be dependent on an increased supply of [4Fe-4S] clusters provided in cells expressing the mutant IscR (Example 4, Figure 14). Genetically modified prokaryotic cells of the invention that further comprise an additional transgenes encoding a NadE* polypeptide having nicotinic acid mononucleotide amidating activity are able to synthesize both quinolate and NR. Additionally including one or more of the transgenes with NMN nucleosidase activity (EC: 3.2.2.14) and pncA with nicotinamide deamidase activity (EC: 3.5.1.19) will allow the cells to synthesize both NA and NAM (Figure 13). Optionally, the cells may comprise one of more transgenes or upregulated endogenous genes (as defined herein) encoding polypeptides that catalyze other steps in the NR synthesis pathway (Figure 13), such as a NadB polypeptide having aspartate oxidase activity (EC: 1.4.3.16). Preferably, the NAD salvage pathway is down-regulated, for example by deletion or inactivation of the nadR and/or pncC genes in the genetically modified prokaryotic cell, thereby reducing NR consumption.

An increase in the levels of those polypeptides that catalyze steps in the NR pathway enhances the synthesis of both intermediates and end products of the NR pathway in the prokaryotic cell.

Polypeptides having quinolate synthase activity (EC: 2.5.1.72) are encoded by genes found in a wide range of bacteria and plants belonging to a wide range of genera. The amino acid sequence of the NadA polypeptide having quinolate synthase activity has at least 70, 75, 80, 85, 90, 95, 96, 98, 100% amino acid sequence identity to a sequence selected from any one of: SEQ ID No.: 117 (origin: *Escherichia coli);* SEQ ID No.: 118 (origin: *Thermotoga maritima*); SEQ ID No.: 119 (origin: *Acidobacterium capsulatum*); SEQ ID No.: 120 (origin: *Aquifex aeolicus);* SEQ ID No.: 121 (origin: *Bacillus subtilis*); SEQ ID No.: 122 (origin: *Corynebacterium glutamicum*) SEQ ID No.: 123 (origin: *Pseudomonas putida);* SEQ ID No.: 124 (origin: *Sulfolobus solfataricus*); SEQ ID No.: 125 (origin: *Synechococcus elongatus*); SEQ ID No.: 126 (origin: *Thermus thermophilus*); and SEQ ID No.: 127 (origin: *Arabidopsis thaliana).*

The polypeptides that are encoded by the additional transgenes or upregulated endogenous genes in the genetically modified prokaryotic cell, and whose activity serves to enhance the synthesis of both intermediates and products of the NR pathway, are as follows:
a) a NadB polypeptide having aspartate oxidase activity (synthesizes iminoaspartate from L-aspartate (EC: 1.4.3.16); such as a polypeptide with an amino acid sequence having 80, 85, 90, 95 or 100% sequence identity to SEQ ID No.:128 (origin: *Escherichia coli);*
b) a NadC polypeptide having nicotinate-nucleotide pyrophosphorylase (EC: 2.4.2.19); such as a polypeptide with an amino acid sequence having 80, 85, 90, 95 or 100% sequence identity to SEQ ID No.:133 (origin: *Escherichia coli);*
c) a NadE* polypeptide having NH(3)-dependent NAD(+) synthetase activity (EC: 6.3.1.5), such as a polypeptide with an amino acid sequence having 80, 85, 90, 95 or 100% sequence identity to SEQ ID No.:129 (origin: *Mannheimia succiniciproducens;*
d) an AphA polypeptide having Class B acid phosphatase; such as a polypeptide with an amino acid sequence having 80, 85, 90, 95 or 100% sequence identity to SEQ ID No.:130 (Origin *E. coli)*
e) a PncA polypeptide having nicotinamide deamidase activity (EC: 3.5.1.19); such as a polypeptide with an amino acid sequence having 80, 85, 90, 95 or 100% sequence identity to SEQ ID No.:131 (origin: *Escherichia coli);* and
f) a Chi polypeptide having nucleosidase NMN nucleosidase activity (EC: 3.2.2.14); such as a polypeptide with an amino acid sequence having 80, 85, 90, 95 or 100% sequence identity to SEQ ID No.:132;
   When the gene(s) encoding quinolate synthase together with one or more additional polypeptides that catalyze additional steps in the quinolate and B₃ vitamins pathways are transgenes, they are located in the genome of the genetically modified prokaryotic cell, either integrated into the prokaryotic cell chromosome or on a self-replicating plasmid. The transgene encoding NadA and one or more of the transgenes (*nadB,* and *nadE*) encoding enzymes in the NR pathway enzymes may be present in the genome within one or more operon.

The promoter driving expression of the transgene encoding NadB and one or more additional transgenes is preferably a non-native promoter, which may be a heterologous constitutive-promoter or an inducible-promoter. When expression driven by the promoter is constitutive, then a suitable promoter includes apFab family [SEQ ID Nos.:97] while a suitable inducible promoter includes: pBad (arabinose-inducible) [SEQ ID No.:38] and lac promoter lac p, which is regulated by repressor lacI [SEQ ID No.:40]. Suitable terminators include members of the *apFAB* terminator family including [SEQ ID No.: 41]. The selected promoter and terminator may be operably linked to the respective gene, either to provide individual gene regulation or for regulation of an operon.

### V A method for producing and detecting B3 vitamins and the intermediate quinolate using a genetically modified bacterium to the invention

B₃ vitamins and quinolate can be produced using genetically modified prokaryotic cells of the invention (e.g. genetically modified *E. coli* cells) by introducing the cells into a suitable culture medium; and finally recovering the said products of the cells, as illustrated in the example 4.

The genetically modified prokaryotic cells of the invention comprising a transgene encoding a quinolate synthase (NadB) will produce quinolate when supplied with a suitable carbon source for example a source selected from among glucose, maltose, galactose, fructose, sucrose, arabinose, xylose, raffinose, mannose, and lactose.

A method for quantifying cellular quinolate and NR produced by a genetically modified prokaryotic cell of the invention is described in example 4.

### VI A genetically modified prokaryotic cell for production of cobalamin

According to a further embodiment, the present invention provides a genetically modified prokaryotic cell capable of producing enhanced levels of cobalamin. The prokaryotic cell is genetically modified to express a mutant IscR, according to the invention (see section I and II), in substitution for a wild type IscR, as well as comprising a transgene or up-regulated endogenous cobG gene (i.e an endogenous cobG gene operably linked to a genetically modified regulatory sequence capable of enhancing expression of said endogenous gene, as described in section I) encoding a CobG polypeptide having precorrin-3B synthase (EC: 1.3.98.3).

CobG is an [4Fe-4S] cluster-dependent enzyme that catalyzes the conversion of precorrin-3-A to Precorrin-3-B, using dioxygen (O2) and nicotinamide adenine dinucleotide (NADH) as co-factors (Figure 15). The growth of cells over-expressing this enzyme is dependent on an increased supply of [4Fe-4S] clusters provided in cells expressing the mutant IscR (Example 5). Genetically modified prokaryotic cells of the invention that further comprise an additional transgene or additional upregulated endogenous genes (as defined herein) encoding polypeptides CobIMFKHLJ are able to enhance flux through the pathway, and production of the intermediate hydrogenobyrinic acid (HBA). Cells of the invention that further comprise transgenes encoding polypeptides that catalyze the subsequent steps in the cob synthesis pathway (Figure 15), in particular genes encoding CobNST, CobC, CobD, CobT, PduX, CobU, CobS, CbiB, CbiN, CbiQ, CbiO, and CbiM are able to produce cobalamin.

CobG polypeptides having precorrin-3B synthase (EC: 1.14.13.83) are encoded by genes found in a wide range of microorganisms belonging to a wide range of genera. The amino acid sequence of the polypeptide having precorrin-3B synthase activity has at least 70, 75, 80, 85, 90, 95, 96, 98, 100% amino acid sequence identity to a sequence selected from any one of: SEQ ID No.: 135 (origin: *Pseudomonas denitrificans);* SEQ ID No.: 136 (origin: *Cornybacterium glutamicum*); SEQ ID No.: 137 (origin: *Frankia canadensis*); SEQ ID No.: 138 (origin: *Nostoc sp. CENA543*); SEQ ID No.: 139(origin: *Rhizobium leguminosarum*); SEQ ID No.: 140 (origin: *Mycoplana dimorpha*) SEQ ID No.: 141 (origin: *Rhodobacter sphaeroides*); SEQ ID No.: 142 (origin: *Granulicella tundricola*); SEQ ID No.: 143 (origin: *Sinorhizobium meliloti*); SEQ ID No.: 144 (origin: *Streptomyces cattleya*); and SEQ ID No.: 145 (origin: *Pannonibacter phragmitetus*).

The polypeptides that are encoded by the additional transgenes or upregulated endogenous genes (as defined herein) in the genetically modified prokaryotic cell, and whose activity serves to enhance the synthesis of both intermediates of the Cob pathway, are as follows:
a) a CobI polypeptide having precorrin-2 C20-methyltransferase activity (EC: 2.1.1.130), catalyses the synthesis of precorrin-3A from precorrin-2, such as a polypeptide with an amino acid sequence having 80, 85, 90, 95 or 100% sequence identity to SEQ ID No.: 146 (origin: *Pseudomonas denitrificans);*
c) a CobM polypeptide having precorrin-3 methylase activity (EC: 2.1.1.133), catalyses the synthesis of precorrin-5 from precorrin-4, such as a polypeptide with an amino acid sequence having 80, 85, 90, 95 or 100% sequence identity to SEQ ID No.:147 (origin: *Pseudomonas denitrificans);*
d) a CobF polypeptide having cobalt-precorrin-6A synthase activity (EC: 2.1.1.195), catalyses the synthesis of precorrin-6A from precorrin-5B, such as a polypeptide with an amino acid sequence having 80, 85, 90, 95 or 100% sequence identity to SEQ ID No.:148 (origin: *Pseudomonas denitrificans);*
e) a CobK polypeptide having precorrin-6A reductase activity (EC: 1.3.1.54), catalyses the synthesis of precorrin-6B from precorrin-6A, such as a polypeptide with an amino acid sequence having 80, 85, 90, 95 or 100% sequence identity to SEQ ID No.:149 (origin: *Pseudomonas denitrificans);*
f) a CobH polypeptide having precorrin isomerase activity (EC: 5.4.99.61), catalyses the conversion of precorrin-8X to hydrogenobyrinate, such as a polypeptide with an amino acid sequence having 80, 85, 90, 95 or 100% sequence identity to SEQ ID No.:150 (origin: Pseudomonas denitrificans);
g) a CobL polypeptide having Precorrin-6Y C(5,15)-methyltransferase activity (EC: 2.1.1.132), catalyses the conversion of C-5 and C-15 in precorrin-6Y to form precorrin-8X, such as a polypeptide with an amino acid sequence having 80, 85, 90, 95 or 100% sequence identity to SEQ ID No.:151 (origin: Pseudomonas denitrificans);
h) a CobJ polypeptide having Precorrin-3B C(17)-methyltransferase (EC: 2.1.1.131), catalyses the methylation of precorrin-3B to form precorrin-4, such as a polypeptide with an amino acid sequence having 80, 85, 90, 95 or 100% sequence identity to SEQ ID No.:152 (origin: Pseudomonas denitrificans);
   The polypeptides that are encoded by additional transgenes or upregulated endogenous genes (as defined herein) in the genetically modified prokaryotic cell, include those whose activity serves to complete the pathway and permit production of cobalamin, are as follows:
j) a CobN, CobS and CobT are polypeptide subunits of an enzyme having aerobic cobaltochelatase activity (EC: 6.6.1.2), which catalyzes cobalt insertion in the corrin ring, such as a polypeptide with an amino acid sequence having 80, 85, 90, 95 or 100% sequence identity to SEQ ID No.:153, 154 and 155 respectively (origin: *Brucella melitensis*)*.*
K) CobR polypeptide having 4-hydroxyphenylacetate 3-monooxygenase activity (EC: 1.14.14.9) wherein the amino acid sequence of the polypeptide has 80, 85, 90, 95 or 100% sequence identity to SEQ ID No.:156, (origin:*Brucella melitensis*);
l) CobO polypeptide having corrinoid adenosyltransferase activity (EC: 2.5.1.17) synthesizes adenosylcobalamin from cob(II)yrinate a,c-diamide, wherein the amino acid sequence of the polypeptide has 80, 85, 90, 95 or 100% sequence identity to SEQ ID No.:157, (origin: *Pseudomonas denitrificans*);
m) CobQ polypeptide having cobyric acid synthase activity (EC: 6.3.5.10) catalyses aminidations of adenosylcobyrinic A,C-diamide, wherein the amino acid sequence of the polypeptide has 80, 85, 90, 95 or 100% sequence identity to SEQ ID No.:158, (origin: *Pseudomonas denitrificans);*
n) BtuR polypeptide having corrinoid adenosyltransferase (EC: 2.5.1.17), wherein the amino acid sequence of the polypeptide has 80, 85, 90, 95 or 100% sequence identity to SEQ ID No.:159, (origin *E. coli);*
o) CobU polypeptide having bifunctional adenosylcobalamin biosynthesis 4-hydroxyphenylacetate 3-monooxygenase activity (EC: 2.7.1.156), wherein the amino acid sequence of the polypeptide has 80, 85, 90, 95 or 100% sequence identity to SEQ ID No.:160, (origin: *E. coli);*
p) CobD, polypeptide having threonine-phosphate decarboxylase activity (EC: 4.1.1.81) that decarboxylates L-threonine-O-3-phosphate to yield (R)-1-amino-2-propanol O-2-phosphate, the precursor for the linkage between the nucleotide loop and the corrin ring in cobalamin, wherein the amino acid sequence of the polypeptide has 80, 85, 90, 95 or 100% sequence identity to SEQ ID No.:161, (origin: *Salmonella typhimurium*);
q) CobC polypeptide having Adenosylcobalamin/alpha-ribazole phosphataseactivity (EC: 3.1.3.73) converting adenosylcobalamin 5'-phosphate to adenosylcobalamin, wherein the amino acid sequence of the polypeptide has 80, 85, 90, 95 or 100% sequence identity to SEQ ID No.:162, (origin: *E. coli);*
r) CobT polypeptide having Nicotinate-nucleotide-dimethylbenzimidazole phosphoribosyltransferase activity (EC: 2.4.2.21), wherein the amino acid sequence of the polypeptide has 80, 85, 90, 95 or 100% sequence identity to SEQ ID No.:163, (origin: *E. coli);*
r) CobS polypeptide having Adenosylcobinamide-GDP ribazoletransferase activity (EC: 2.7.8.26), wherein the amino acid sequence of the polypeptide has 80, 85, 90, 95 or 100% sequence identity to SEQ ID No.:164, (origin: *E*. *coli*);
r) CbiB polypeptide having cobalamin biosynthesis activity (EC: 6.3.1.10) converting cobyric acid into cobinamide, wherein the amino acid sequence of the polypeptide has 80, 85, 90, 95 or 100% sequence identity to SEQ ID No.:165, (origin: *Salmonella typhimurium*);
s) PduX polypeptide having L-threonine kinase activity (EC: 2.7.1.177) converting L-threonine to L-threonine-O-3-phosphate, wherein the amino acid sequence of the polypeptide has 80, 85, 90, 95 or 100% sequence identity to SEQ ID No.:166, (origin: *Salmonella typhimurium*);
t) CbiN is a polypeptide having the function of a cobalt transport protein; wherein the amino acid sequence of the polypeptide has 80, 85, 90, 95 or 100% sequence identity to SEQ ID No.:167, (origin: *Salmonella typhimurium*); and
u) ChiQ is a polypeptide having the function of a cobalt transport protein; wherein the amino acid sequence of the polypeptide has 80, 85, 90, 95 or 100% sequence identity to SEQ ID No.:168, (origin: *Salmonella typhimurium);*
v) CbiM is a polypeptide having the function of a cobalt transport protein; wherein the amino acid sequence of the polypeptide has 80, 85, 90, 95 or 100% sequence identity to SEQ ID No.:169, (origin: *Salmonella typhimurium);* and w) CbiO polypeptide having cobalt import ATP-binding protein activity (EC: 3.6.3..-); wherein the amino acid sequence of the polypeptide has 80, 85, 90, 95 or 100% sequence identity to SEQ ID No.:170, (origin: *Salmonella typhimurium);.*
   When the gene(s) encoding the CobG polypeptide, precorrin-3B synthase, together with one or more additional polypeptides that catalyze additional steps in the Cob pathway are transgenes, they are located in the genome of the genetically modified prokaryotic cell, either integrated into the prokaryotic cell chromosome or on a self-replicating plasmid. The transgene encoding CobG and one or more of the transgenes Cob pathway enzymes may be present in the genome within one or more operon.

The promoter driving expression of the transgene encoding CobG and one or more additional transgenes is preferably a non-native promoter, which may be a heterologous constitutive-promoter or an inducible-promoter. When expression driven by the promoter is constitutive, then a suitable promoter includes apFab family [SEQ ID Nos.:97] while a suitable inducible promoter includes: pBad (arabinose-inducible) [SEQ ID No.:38] and lac promoter lac p, which is regulated by repressor lacI [SEQ ID No.:40]. Suitable terminators include members of the *apFAB* terminator family including [SEQ ID No.: 98]. The selected promoter and terminator may be operably linked to the respective gene, either to provide individual gene regulation or for regulation of an operon.

### VII A method for producing and detecting cobalamin using a genetically modified bacterium according to the invention

Cobalamin can be produced using genetically modified prokaryotic cells of the invention (e.g. genetically modified *E. coli* cells) by introducing the cells into a suitable culture medium; and finally recovering the cobalamin, as illustrated in the Example 5. A suitable culture medium includes a carbon source selected from among glucose, maltose, galactose, fructose, sucrose, arabinose, xylose, raffinose, mannose, and lactose.

A method for quantifying cobalamin produced by a genetically modified prokaryotic cell of the invention is described in example 5; and may include the use of High Pressure Liquid Chromatography, relative to a cobalamin standard.

### VIII A genetically modified prokaryotic cell for production of pantothenic acid and branched chain amino acids

According to a further embodiment, the present invention provides a genetically modified prokaryotic cell capable of producing enhanced levels of the branched chain amino acids L-valine, L-leucine and L-isoleucine and vitamin B₅ (pantothenate). The prokaryotic cell is genetically modified to express a mutant IscR, according to the invention (see section I and II), in substitution for a wild type IscR, as well as comprising a transgene or up-regulated endogenous ilvD gene (i.e an endogenous ilvD gene operably linked to a genetically modified regulatory sequence capable of enhancing expression of said endogenous gene, as described in section I) encoding a IlvD polypeptide having dihydroxy-acid dehydratase activity (EC: 4.2.1.9).

IlvD is an [4Fe-4S] cluster-dependent enzyme that catalyzes the dehydration of 2,3-dihydroxy-3-methylbutanoate into 3-methyl-2-oxobutanoate (Figure 16A) as well as the dehydration of 2,3-dihydroxy-3-methylpentanoate into 3-methyl-2-oxopentanoate (Figure 16B). The growth of cells over-expressing this enzyme is dependent on an increased supply of [4Fe-4S] clusters provided in cells expressing the mutant IscR (Example 6). Genetically modified prokaryotic cells of the invention that further comprise one or more additional transgenes encoding polypeptides IlvB, IlvBN and IlvC are capable of enhanced synthesis of both L-valine, L-leucine and L-isoleucine and pantothenate, due to enhanced flux through the ilv pathway (based on detectable levels of the intermediates 3-methyl-2-oxobutanoate and 3-methyl-2-oxopentanoate. Optionally, the cells may comprise one of more transgenes or upregulated endogenous genes (as defined herein) encoding polypeptides that catalyze other steps in the ilv pathway (Figure 16), such as IlvE to further enhance L-valine production and IlvNbis to enhance flux into the Ilv pathway.

Additionally, the genetically modified cells of the invention may further comprise transgenes or upregulated endogenous genes (as defined herein) encoding the L-valine exporter *ygaZH* and global regulator leucine responsive protein (Lrp), to enhance net L-valine export.

Polypeptides having dihydroxy-acid dehydratase activity (EC: 4.2.1.9) are encoded by genes found in a wide range of microorganisms belonging to a wide range of genera. The amino acid sequence of the IlvD polypeptide having dihydroxy-acid dehydratase activity has at least 70, 75, 80, 85, 90, 95, 96, 98, 100% amino acid sequence identity to a sequence selected from any one of: SEQ ID No.: 172 (origin: *E. coli);* SEQ ID No.: 173 (origin: *Acidobacterium capsulatum*); SEQ ID No.: 174 (origin: *Saccharomyces cerevisiae*); SEQ ID No.: 175 (origin: *Aquifex aeolicus);* SEQ ID No.: 176 (origin: *Bacillus subtilis);* SEQ ID No.: 177(origin: *Corynebacterium glutamicum*) SEQ ID No.: 178 (origin: *Deinococcus radiodurans*); SEQ ID No.: 179 (origin: *Methanococcus maripaludis*); SEQ ID No.: 180 (origin: *Pseudomonas putida);* SEQ ID No.: 181 (origin: *Synechococcus elongatus*); and SEQ ID No.: 182 (origin: *Thermotoga maritima*)*.*

The polypeptides that are encoded by the additional transgenes in the genetically modified prokaryotic cell, and whose activity serves to enhance the synthesis of both intermediates and products of the ilv pathway, are as follows:
a) an IlvB polypeptide having acetolactate synthase isozyme 1 large subunit activity (EC: 2.2.1.6) and together with IlvN catalyses the conversion of pyruvate into (2S)-2-acetolactate; such as a polypeptide with an amino acid sequence having 80, 85, 90, 95 or 100% sequence identity to SEQ ID No.:183 (origin: *E. coli*);
b) an IlvN polypeptide having acetolactate synthase isozyme 1 small subunit activity (EC: 2.2.1.6) and together with IlvB catalyses the conversion of pyruvate into (2S)-2-acetolactate; such as a polypeptide with an amino acid sequence having 80, 85, 90, 95 or 100% sequence identity to SEQ ID No.:184 (origin: *E. coli*);
c) an IlvC polypeptide having ketol-acid reductoisomerase (NADP+) activity (EC: 1.1.1.86) catalyses the conversion of (2S)-2-acetolactate into (2S)-2-hydroxy-2-methyl-3-oxobutanoate; such as a polypeptide with an amino acid sequence having 80, 85, 90, 95 or 100% sequence identity to SEQ ID No.:185 (origin: *E. coli*);
b) an IlvE polypeptide having branched-chain-amino-acid aminotransferase activity (EC: 2.6.1.42), catalyses the synthesis of L-valine from 3-methyl-2-oxobutanoate, such as a polypeptide with an amino acid sequence having 80, 85, 90, 95 or 100% sequence identity to SEQ ID No.:186(origin: *E. coli).*
e) an IlvNbis polypeptide (mutant IlvN polypeptide), said mutation enhancing acetolactate synthase isozyme 1 small subunit activity (EC: 2.2.1.6) and together with IlvB catalyses the conversion of pyruvate into (2S)-2-acetolactate; such as a polypeptide with an amino acid sequence having 80, 85, 90, 95 or 100% sequence identity to SEQ ID No.:187 (origin: *E. coli*);
f) a YgaZ and YgaH polypeptides, that together confer L-valine transmembrane transporter activity, such as polypeptides characterized by an amino acid sequence having 80, 85, 90, 95 or 100% sequence identity to SEQ ID No.:188 and 189 respectively (origin: *E. coli*);
g) a LrP polypeptide having leucine responsive regulator protein activity, such as a polypeptide with an amino acid sequence having 80, 85, 90, 95 or 100% sequence identity to SEQ ID No.:190 (origin: *E. coli*);
   When the gene(s) encoding the IlvD polypeptide, dihydroxy-acid dehydratase activity, together with one or more additional polypeptides that catalyze additional steps in the ilv pathway (encoding IlvB, IlvN or IlvNbis, IlvC, and optionally IlvE) as well as polypeptides conferring net valine export (YgaZ, YgaH and LrP) are transgenes, they are located in the genome of the genetically modified prokaryotic cell, either integrated into the prokaryotic cell chromosome or on a self-replicating plasmid. The transgene encoding IlvD and one or more of the transgenes encoding Ilv pathway enzymes may be present in the genome within one or more operon.

The promoter driving expression of the transgene encoding IlvD and one or more additional transgenes is preferably a non-native promoter, which may be a heterologous constitutive-promoter or an inducible-promoter. When the promoter is a heterologous constitutive promoter, then a suitable promoter includes the *apFab* family [SEQ ID Nos.:97], while a suitable inducible promoter includes: pBad (arabinose inducible [SEQ ID No.:38] and LacI [SEQ ID No.:40]. Suitable terminators include members of the *apFAB* terminator family including [SEQ ID No.: 98]. The selected promoter and terminator may be operably linked to the respective gene, either to provide individual gene regulation or for regulation of an operon.

### IX A method for producing and detecting branched-chain amino acids and pantothenic acid using a genetically modified bacterium according to the invention

Branched chain amino acids (valine, leucine and isoleucine) as well as pantothenoate can be produced using genetically modified prokaryotic cells of the invention (e.g. genetically modified *E. coli* cells) by introducing the cells into a suitable culture medium; and finally recovering the synthesized products, as illustrated in the Example 6. A suitable culture medium includes a carbon source selected from among glucose, maltose, galactose, fructose, sucrose, arabinose, xylose, raffinose, mannose, and lactose.

A method for quantifying branched chain amino acids and pantothenate produced by a genetically modified prokaryotic cell of the invention is described in example 6.

The present invention provides a genetically modified prokaryotic cell capable of producing enhanced levels of the branched chain amino acids L-valine, L-leucine and L-isoleucine and vitamin B₅ (pantothenate).

### X A genetically modified prokaryotic cell for production of isoprenoids

According to a further embodiment, the present invention provides a genetically modified prokaryotic cell capable of producing enhanced levels of isoprenoids and the isoprenoid precursors isopentenyl diphosphate (IPP) and dimethylallyl diphosphate (DMAPP) via the MEP pathway (Figure 18). The prokaryotic cell is genetically modified to express a mutant IscR, according to the invention (see section I and II), in substitution for a wild type IscR, as well as comprising transgenes or up-regulated endogenous genes (i.e an endogenous genes operably linked to a genetically modified regulatory sequence capable of enhancing expression of said endogenous gene, as described in section I) encoding an IspG polypeptide having 4-hydroxy-3-methylbut-2-en-1-yl diphosphate synthase (EC: 1.17.7.3) and an IspH polypeptide having 4-hydroxy-3-methylbut-2-enyl diphosphate reductase activity (EC: 1.17.7.4).

Both IspG and IspH are [4Fe-4S] cluster-dependent enzymes that catalyze the final two steps in the MEP pathway. IspG catalyzes the synthesis of HMBPP from MEcPP; while IspH catalyzes the synthesis of IPP and DMAPP in a 3:1 molar ratio from the substrate HMBPP.

Genetically modified prokaryotic cells of the invention that further comprise one or more additional transgenes or upregulated endogenous genes (as defined herein) encoding polypeptides are able to synthesize enhanced levels of both IPP and DMAPP.

IspG polypeptides having 4-hydroxy-3-methylbut-2-en-1-yl diphosphate synthase (EC: 1.17.7.3) are encoded by genes found in a wide range of microorganisms and plants belonging to a wide range of genera. The amino acid sequence of the polypeptide having 4-hydroxy-3-methylbut-2-en-1-yl diphosphate synthase activity has at least 70, 75, 80, 85, 90, 95, 96, 98, 100% amino acid sequence identity to a sequence selected from any one of: SEQ ID No.: 192 (origin: *E. coli);* SEQ ID No.: 193 (origin: *Acidobacterium capsulatum*); SEQ ID No.: 194 (origin: *Aquifex aeolicus);* SEQ ID No.: 195 (origin: *Arabidopsis thaliana*); SEQ ID No.: 196 (origin: *Bacillus subtilis);* SEQ ID No.: 197 (origin: *Clostridium acetobutylicum*) SEQ ID No.: 198 (origin: *Corynebacterium glutamicum*); SEQ ID No.: 199 (origin: *Deinococcus radiodurans*); SEQ ID No.: 200 (origin: *Pseudomonas putida);* and SEQ ID No.: 201 (origin: *Synechococcus elongatus*).

Correspondingly, IspH polypeptides having 4-hydroxy-3-methylbut-2-enyl diphosphate reductase activity (EC: 1.17.7.4) are encoded by genes found in a wide range of microorganisms and plants. The amino acid sequence of the polypeptide having 4-hydroxy-3-methylbut-2-enyl diphosphate reductase activity activity has at least 70, 75, 80, 85, 90, 95, 96, 98, 100% amino acid sequence identity to a sequence selected from any one of: SEQ ID No.: 203 (origin: *E. coli);* SEQ ID No.: 204 (origin: *Acidobacterium capsulatum*); SEQ ID No.: 205 (origin: *Aquifex aeolicus*); SEQ ID No.: 206 (origin: *Arabidopsis thaliana*); SEQ ID No.: 207 (origin: *Bacillus subtilis*); SEQ ID No.: 208 (origin: *Clostridium acetobutylicum*) SEQ ID No.: 209 (origin: *Corynebacterium glutamicum*); SEQ ID No.: 210 (origin: *Deinococcus radiodurans*); SEQ ID No.: 211 (origin: *Pseudomonas putida);* SEQ ID No.: 212 (origin: *Synechococcus elongatus*) and SEQ ID No.: 213 (origin: *Thermotoga maritima*)*.*

The genetically modified prokaryotic cell may be further modified in order to overexpress one or more polypeptides whose activity serves to enhance the flux through the isoprenoid biosynthesis pathway and thereby enhance synthesis of both intermediates and products of the pathway, as follows:
a) DXS having 1-deoxy-D-xylulose-5-phosphate synthase (EC: 2.2.1.7), whose amino acid sequence has at least 70, 75, 80, 85, 90, 95, 96, 98, 100% amino acid sequence identity to a sequence selected from any one of: SEQ ID No.: 214 (origin: *E. coli*);
b) Ipi, having Isopentenyl-diphosphate Delta-isomerase EC:5.3.3.2, whose amino acid sequence has at least 70, 75, 80, 85, 90, 95, 96, 98, 100% amino acid sequence identity to a sequence selected from any one of: SEQ ID No.: 215 (origin: *E. coli*) and
b) IspC (dxr) 1-deoxy-D-xylulose 5-phosphate reductoisomerase (EC: 1.1.1.267), whose amino acid sequence has at least 70, 75, 80, 85, 90, 95, 96, 98, 100% amino acid sequence identity to a sequence selected from any one of: SEQ ID No.: 216(origin: *E. coli*)
d) RpoS having the function of an RNA polymerase subunit sigma factor σ, whose amino acid sequence has at least 70, 75, 80, 85, 90, 95, 96, 98, 100% amino acid sequence identity to a sequence selected from any one of: SEQ ID No.: 217 (origin: *E. coli*)

Optionally the genetically modified prokaryotic cell may be further modified in order to delete the ytjC gene encoding a phosphoglycerate mutase enzyme (EC: 5.4.2.-) in order to increase metabolic flux through the isoprenoid biosynthesis pathway.

When the genes encoding the IspG and IspH polypeptides, together with one or more additional polypeptides that catalyze additional steps that enhance synthesis of isoprenoids and their precursors IPP and DMAPP are transgenes, they are located in the genome of the genetically modified prokaryotic cell, either integrated into the prokaryotic cell chromosome or on a self-replicating plasmid. The transgene encoding IspG and IspH and one or more of the transgenes encoding other MEP pathway enzymes may be present in the genome within one or more operon.

The promoter driving expression of the transgene encoding IspG and IspH and one or more additional transgenes is preferably a non-native promoter, which may be a heterologous constitutive-promoter or an inducible-promoter. When expression driven by the promoter is constitutive, then a suitable promoter includes apFab family [SEQ ID Nos.:97] while a suitable inducible promoter includes: pBad (arabinose-inducible) [SEQ ID No.:38] and lac promoter lac p, which is regulated by repressor lacI [SEQ ID No.:40]. Suitable terminators include members of the *apFAB* terminator family including [SEQ ID No.: 98]. The selected promoter and terminator may be operably linked to the respective gene, either to provide individual gene regulation or for regulation of an operon.

### XI A method for producing isoprenoid precursors and their derivatives using a genetically modified bacterium of the invention

The isoprenoid precursors IPP and DMAPP and their derivatives can be produced using genetically modified prokaryotic cell s of the invention (e.g. genetically modified *E. coli* cells) by introducing the cells into a suitable culture medium; and finally recovering the isoprenoid precursors or their derivatives, as illustrated in the Example 7. A suitable culture medium includes a carbon source selected from among glucose, maltose, galactose, fructose, sucrose, arabinose, xylose, raffinose, mannose, and lactose.

A method for quantifying IPP and DMAPP produced by a genetically modified prokaryotic cell of the invention is described in example 7.

The present invention provides a genetically modified prokaryotic cell capable of producing enhanced levels of the IPP and DMAPP as well as their isoprenoid derivatives.

### XII A genetically modified prokaryotic cell for production of L-glutamic acid and δ-aminolevulinic acid

Micro-organisms can produce L-glutamic acid by means of the glutamine synthase - glutamate synthase (glutamine:2-oxoglutarate aminotransferase (GS-GOGAT) pathway, particularly at low ammonia concentrations. The present invention provides a genetically modified prokaryotic cell capable of producing enhanced levels of the L-glutamic acid via the GS-GOGAT pathway as well as δ-aminolevulinic acid (ALA) (Figure 19). The prokaryotic cell is genetically modified to express a mutant IscR, according to the invention (see section I and II), in substitution for a wild type IscR, as well as comprising transgenes or up-regulated endogenous genes (i.e endogenous genes operably linked to a genetically modified regulatory sequence capable of enhancing expression of said endogenous gene, as described in section I) encoding a GltB polypeptide having glutamate synthase [NADPH] large chain, (EC: 1.4.1.13) and glutamate synthase [NADPH] small chain, GltD (EC: 1.4.1.13).

The small and large chain polypeptides, GltB and GltD, together form the [4Fe-4S] cluster-dependent enzyme, GOGAT that catalyze GS-GOGAT reaction.

A GltB polypeptide comprising the glutamate synthase [NADPH] large chain of GOGAT, (EC: 1.4.1.13) has an amino acid sequence having at least 70, 75, 80, 85, 90, 95, 96, 98, 100% amino acid sequence identity to a sequence selected from any one of: SEQ ID No.: 219 (origin: *E. coli)* or SEQ ID No.: 220 (origin: *Pseudomonas putida);* SEQ ID No.: 221 (origin: *Deinococcus swuensis*); SEQ ID No.: 222 (origin: *Methanoculleus chikugoensis*); SEQ ID No.: 223 (origin: *Acidobacterium sp.*); SEQ ID No.: 224 (origin: *Corynebacterium glutamicum*); SEQ ID No.: 225 (origin: *Bacillus subtilis);* SEQ ID No.: 226 (origin: *Aquifex aeolicus);* SEQ ID No.: 227 (origin: *Synechocystis* sp);and SEQ ID No.: 228 (origin: *Petrotoga miotherma*).

A GltD polypeptide comprising the glutamate synthase [NADPH] small chain of GOGAT, (EC: 1.4.1.13) has an amino acid sequence having at least 70, 75, 80, 85, 90, 95, 96, 98, 100% amino acid sequence identity to a sequence selected from any one of: SEQ ID No.: 230 (origin: *E. coli);* SEQ ID No.: 231 (origin: *Pseudomonas putida*)*.* SEQ ID No.: 232 (origin: *Deinococcus swuensis*); SEQ ID No.: 233 (origin: *Methanoculleus chikugoensis*); SEQ ID No.: 234 (origin: *Acidobacterium sp.*); SEQ ID No.: 235 (origin: *Corynebacterium glutamicum*); SEQ ID No.: 236 (origin: *Bacillus subtilis);* SEQ ID No.: 237 (origin: *Aquifex aeolicus);* SEQ ID No.: 238 (origin: *Synechocystis* sp);and SEQ ID No.: 239 (origin: *Petrotoga miotherma*)*.*

A cell factory relying on increased GltDB catalysis can also be used to produce considerable amount of ALA, which can be used as a molecule itself or as precursors for other molecules of interest. Accordingly, a genetically modified prokaryotic cell of the invention, comprising transgenes or upregulated endogenous genes encoding GltB and GltD, may additionally comprise transgenes or upregulated endogenous genes encoding GltX, HemA and HemL.

A GltX polypeptide having glutamyl-tRNA synthetase activity, (EC: 6.1.1.17) has an amino acid sequence having at least 70, 75, 80, 85, 90, 95, 96, 98, 100% amino acid sequence identity to a sequence selected from any one of: SEQ ID No.: 240 (origin: *E. coli*)*.*

A HemA polypeptide having glutamyl-tRNA reductase activity, (EC: 1.2.1.70) has an amino acid sequence having at least 70, 75, 80, 85, 90, 95, 96, 98, 100% amino acid sequence identity to a sequence selected from any one of: SEQ ID No.: 107 (origin: *E. coli*).

A HemL polypeptide having Glutamate-1-semialdehyde 2,1-aminomutase activity, (EC: 5.4.3.8) has an amino acid sequence having at least 70, 75, 80, 85, 90, 95, 96, 98, 100% amino acid sequence identity to a sequence selected from any one of: SEQ ID No.: 108 (origin: *E. coli*).

When the genes encoding the GltB and GltD polypeptides and any additional polypeptides, optionally within an operon, are transgenes, they are located in the genome of the genetically modified prokaryotic cell, either integrated into the prokaryotic cell chromosome or on a self-replicating plasmid.

The promoter driving expression of each of the transgenes encoding GltB, GltD and any additional polypeptides is preferably a non-native promoter, which may be a heterologous constitutive-promoter or an inducible-promoter. When the promoter is a heterologous constitutive promoter, then a suitable promoter includes the *apFab* family [SEQ ID Nos.:97], while a suitable inducible promoter includes: pBad (arabinose inducible [SEQ ID No.:38] and LacI [SEQ ID No.:40]. Suitable terminators include members of the *apFAB* terminator family including [SEQ ID No.: 98]. The selected promoter and terminator may be operably linked to the respective gene, either to provide individual gene regulation or for regulation of an operon.

### XIII A method for producing L-glutamic acid and δ-aminolevulinic acid using a genetically modified bacterium of the invention

L-glutamic acid and δ-aminolevulinic acid can be produced using genetically modified prokaryotic cells of the invention (e.g. genetically modified *E. coli* cells) by introducing the cells into a suitable culture medium; and finally recovering the L-glutamic acid, as illustrated in the Example 8. A suitable culture medium includes a carbon source selected from among glucose, maltose, galactose, fructose, sucrose, arabinose, xylose, raffinose, mannose, and lactose.

A method for quantifying L-glutamic acid and δ-aminolevulinic acid produced by a genetically modified prokaryotic cell of the invention is described in example 8. The present invention provides a genetically modified prokaryotic cell capable of producing enhanced levels of L-glutamic acid.

### XIV A genetically modified prokaryotic cell for production of pyrroloquinoline quinone (PQQ) and its precursors and quinoproteins

PQQ is synthesized in nature from a precursor which is small peptide PqqA, containing the sequence motif -E-X-X-X-Y-, where an L-glutamate and an L-tyrosine are separated by three amino acid residues. The enzyme PqqA peptide cyclase (PqqE) catalyzes PQQ synthesis by means of a radical driven C-C bond formation linking the glutamate and tyrosine residues at atoms C9 and C9a of PQQ. All carbon and nitrogen atoms of PQQ are derived from the tyrosine and glutamate residues of the PqqA peptide. The PqqE enzyme features a tunnel through the whole protein and a cave at one end, which harbors the active site with an iron-sulfur-cluster and bound SAM. PqqA is suggested to move through the tunnel to the iron-sulfur cluster where the Glutamate and Tyrosine side chains are then connected. PqqE is the first catalytic step in PQQ synthesis from its precursor PqqA, the subsequent steps requiring genes expressing PqqB, PqqC, PqqD, PqqF as well as a gene encoding the precursor.

According to a further embodiment, the present invention provides a genetically modified prokaryotic cell capable of producing enhanced levels of PQQ via the PQQ biosynthetic pathway (Figure 20).

The prokaryotic cell is genetically modified to express a mutant IscR, according to the invention (see section I and II), in substitution for a wild type IscR, as well as comprising a transgene or up-regulated endogenous gene (i.e an endogenous gene operably linked to a genetically modified regulatory sequence capable of enhancing expression of said endogenous gene, as described in section I) encoding an PqqE polypeptide having PqqA peptide cyclase activity (EC: 1.21.98.4).

Genetically modified prokaryotic cells of the invention may further comprise one or more additional transgenes ot upregulated endogenous genes (as defined herein) encoding polypeptides catalyzing additional steps of the PQQ pathway, in particular encoding one or more of PqqA, PqqB, PqqC, PqqD, or PqqF such as to further enhance levels of both PQQ produced.

A PqqE polypeptide having PqqA peptide cyclase activity (EC: 1.21.98.4) is characterized by an amino acid sequence having at least 70, 75, 80, 85, 90, 95, 96, 98, 100% amino acid sequence identity to: SEQ ID No.: 242 (origin: *Klebsiella pneumoniae*); SEQ ID No.: 243 (origin: *Planctomycetaceae bacterium*); SEQ ID No.: 244 (origin: *Chroococcidiopsis cubana*); SEQ ID No.: 245 (origin: *Azotobacter vinelandii*); and SEQ ID No.: 246 (origin: *Klebsiella pneumoniae*)*.*

A PqqA polypeptide contains the sequence motif -E-X-X-X-Y-, where an L-glutamate and an L-tyrosine are separated by three amino acid residues, and is characterized by an amino acid sequence having at least 70, 75, 80, 85, 90, 95, 96, 98, 100% amino acid sequence identity to: SEQ ID No.: 247 (origin: *Klebsiella pneumoniae).*

A PqqB polypeptide having a putative PQQ carrier function is characterized by an amino acid sequence having at least 70, 75, 80, 85, 90, 95, 96, 98, 100% amino acid sequence identity to: SEQ ID No.: 248 (origin: *Klebsiella pneumoniae*)*.*

A PqqC polypeptide having Pyrroloquinoline-quinone synthase activity (EC: 1.3.3.11) is characterized by an amino acid sequence having at least 70, 75, 80, 85, 90, 95, 96, 98, 100% amino acid sequence identity to: SEQ ID No.: 249 (origin: *Klebsiella pneumoniae*)*.*

A PqqD polypeptide having PqqA binding protein is characterized by an amino acid sequence having at least 70, 75, 80, 85, 90, 95, 96, 98, 100% amino acid sequence identity to: SEQ ID No.: 250 (origin: *Klebsiella pneumoniae*)*.*

A PqqF polypeptide having metalloendopeptidase (EC: 3.4.24.-), involved in processing of the tyrosine and glutamate of PqqA at R1-R3, is characterized by an amino acid sequence having at least 70, 75, 80, 85, 90, 95, 96, 98, 100% amino acid sequence identity to: SEQ ID No.: 251 (origin: *Klebsiella pneumoniae*)*.*

When the genes encoding PqqE, together with one or more additional PqqABCD and F polypeptides that play a role in the PQQ pathway and enhance its synthesis are transgenes, they are located in the genome of the genetically modified prokaryotic cell, either integrated into the prokaryotic cell chromosome or on a self-replicating plasmid. The transgene encoding PqqE and one or more of the transgenes encoding PqqABCD and F polypeptides may be present in the genome within one or more operon.

The promoter driving expression of the transgene encoding PqqE and one or more additional transgenes is preferably a non-native promoter, which may be a heterologous constitutive-promoter or an inducible-promoter. When expression driven by the promoter is constitutive, then a suitable promoter includes apFab family [SEQ ID Nos.:97] while a suitable inducible promoter includes: pBad (arabinose-inducible) [SEQ ID No.:38] and lac promoter lac p, which is regulated by repressor lacI [SEQ ID No.:40]. Suitable terminators include members of the *apFAB* terminator family including [SEQ ID No.: 98]. The selected promoter and terminator may be operably linked to the respective gene, either to provide individual gene regulation or for regulation of an operon.

### XV A method for producing pyrroloquinoline quinone (PQQ), its precursors, and quinoproteins using a genetically modified bacterium of the invention

The pyrroloquinoline quinone (PQQ), its precursors, and quinoproteins can be produced using genetically modified prokaryotic cells of the invention (e.g. genetically modified *E. coli* cells) by introducing the cells into a suitable culture medium; and finally recovering the isoprenoid precursors or their derivatives, as illustrated in the Example 9. A suitable culture medium includes a carbon source selected from among glucose, maltose, galactose, fructose, sucrose, arabinose, xylose, raffinose, mannose, and lactose.

A method for quantifying IPP and DMAPP produced by a genetically modified prokaryotic cell of the invention is described in example 9.

The present invention provides a genetically modified prokaryotic cell capable of producing enhanced levels of PQQ as well as its precursors.

### XVI A genetically modified prokaryotic cell for enhanced nitrogenase activity and nitrogen fixation

According to a further embodiment, the present invention provides a genetically modified prokaryotic cell capable of enhanced assembly of nitrogenase that converts atmospheric di-nitrogen (N₂) into ammonium that is then used in diverse metabolic pathway such as protein synthesis, as well in biological nitrogen fixation in diazotrophs. The assembly of the nitrogenase is dependent on the FeMo cofactor biosynthesis protein enzyme, NifB, which functions as a radical S-Adenosyl-Methionine (SAM) [4Fe-4S] enzyme (Figure 21A). NifB plays a central role by catalyzing the assembly of the Fe-X Cofactor (X = V, Mo, Fe), and its transfer to nitrogenase, where it required for nitrogenase catalysis. The molybdenum-iron nitrogenase complex, on assembly comprises 4 proteins: NifH nitrogenase iron protein (EC: 1.18.6.1); NifD, nitrogenase protein alpha chain (EC: 1.18.6.1); NifK, nitrogenase molybdenum-iron protein beta chain (EC: 1.18.6.1) (Figure 21B).

The prokaryotic cell is genetically modified to express a mutant IscR, according to the invention (see section I and II), in substitution for a wild type IscR, as well as comprising a transgene or up-regulated endogenous gene (i.e an endogenous gene operably linked to a genetically modified regulatory sequence capable of enhancing expression of said endogenous gene, as described in section I and II) encoding a NifB polypeptide having nitrogenase iron-molybdenum cofactor biosynthesis protein activity.

The growth of cells over-expressing NifB enzyme is dependent on an increased supply of [4Fe-4S] clusters provided in cells expressing the mutant IscR (Example 10, Figure 22). Genetically modified prokaryotic cells of the invention that further comprise a *nifB* transgene or upregulated endogenous gene (as defined herein) alone, or in combination with one or more additional transgenes or upregulated endogenous gene (as defined herein) encoding polypeptides NifU (Nitrogen fixation protein) and NifS (cysteine desulfurase EC:2.8.1.7), as well as flavodoxin (fldA) are able to synthesize enhanced levels the nitrogenase complex. Optionally, the cells may comprise one of more transgenes or upregulated endogenous gene (as defined herein) encoding the polypeptides that form the nitrogenase complex, namely NifH, NifD, NifK, (Figure 21B) as well and transgene encoding NifE, NifN, NifX, NifV HesA.

Polypeptides functioning as nifB, FeMo cofactor biosynthesis proteins are encoded by genes found in a wide range of microorganisms belonging to a wide range of genera. The amino acid sequence of the polypeptide having dihydroxy-acid dehydratase activity has at least 70, 75, 80, 85, 90, 95, 96, 98, 100% amino acid sequence identity to a sequence selected from any one of: SEQ ID No.: 253 (origin: *Methanobacterium thermoautotrophicum*); SEQ ID No.: 254 (origin: *Paenibacillus polymyxa*); SEQ ID No.: 255 (origin: *Methanococcus infernus*); SEQ ID No.: 256 (origin: *Methanococcus acetivorans*); SEQ ID No.: 257 (origin: *Azotobacter vinelandii*); SEQ ID No.: 258 (origin: *Rhizobium leguminosarum*) SEQ ID No.: 259 (origin: *Thermocrinis albus*); SEQ ID No.: 260 (origin: *Frankia Alni*); SEQ ID No.: 261 (origin: *Leptospirilum ferrodiazotrophum*); and SEQ ID No.: 262 (origin: *Cupriavidus taiwanensis*); and.

The polypeptides that are encoded by the additional transgenes or upregulated endogenous gene (as defined herein) in the genetically modified prokaryotic cell, and whose activity serves to enhance the synthesis of both intermediates and products of the ilv pathway, are as follows:
c) a fldA polypeptide such as a flavodoxin characterized by an amino acid sequence having 80, 85, 90, 95 or 100% sequence identity to SEQ ID No.:65 (origin: *E. coli);*
d) a NifH polypeptide is a component of a complex having nitrogenase iron protein activity (EC: 1.18.6.1); such as a polypeptide characterized by an amino acid sequence having 80, 85, 90, 95 or 100% sequence identity to SEQ ID No.:263 (origin: *Paenibacillus polymyxa*);
e) a NifD polypeptide is a component of a complex having nitrogenase iron protein activity (EC: 1.18.6.1); such as a polypeptide characterized by an amino acid sequence having 80, 85, 90, 95 or 100% sequence identity to SEQ ID No.:264 (origin: *Paenibacillus polymyxa*);
e) a NifK polypeptide is a component of a complex having nitrogenase iron protein activity (EC: 1.18.6.1); such as a polypeptide characterized by an amino acid sequence having 80, 85, 90, 95 or 100% sequence identity to SEQ ID No.:265 (origin: *Paenibacillus polymyxa*);
f) a NifE polypeptide having Fe-Mo co-factor biosynthesis activity; such as a polypeptide characterized by an amino acid sequence having 80, 85, 90, 95 or 100% sequence identity to SEQ ID No.:266 (origin: *Paenibacillus polymyxa*);
h) a NifN polypeptide having nitrogenase iron-molybdenum cofactor biosynthesis protein activity; such as a polypeptide characterized by an amino acid sequence having 80, 85, 90, 95 or 100% sequence identity to SEQ ID No.:267 (origin: *Paenibacillus polymyxa*);
i) a NifX polypeptide having nitrogen fixation protein activity; such as a polypeptide characterized by an amino acid sequence having 80, 85, 90, 95 or 100% sequence identity to SEQ ID No.:268 (origin: *Paenibacillus polymyxa*);
j) a NifV polypeptide having homocysteine methyltransferase activity; such as a polypeptide characterized by an amino acid sequence having 80, 85, 90, 95 or 100% sequence identity to SEQ ID No.:269 (origin: *Paenibacillus polymyxa*)*;*
k) a HesA polypeptide; such as a polypeptide characterized by an amino acid sequence having 80, 85, 90, 95 or 100% sequence identity to SEQ ID No.:270 (origin: *Paenibacillus polymyxa*);
   When the genes encoding the NifB polypeptide, a radical S-Adenosyl-Methionine (SAM) [4Fe-4S] enzyme, together with one or more additional polypeptides that catalyze additional steps in the nif pathway (encoding NifU, NifS and optionally NifH, NifD, NifK, NifE, NifN, NifX, NifV HesA) as well as flavodoxin are transgenes, they are located in the genome of the genetically modified prokaryotic cell, either integrated into the prokaryotic cell chromosome or on a self-replicating plasmid. The transgene encoding NifB and one or more of the transgenes encoding Nif pathway enzymes may be present in the genome within one or more operon.

The promoter driving expression of the transgene encoding NifB and one or more additional transgenes is preferably a non-native promoter, which may be a heterologous constitutive-promoter or an inducible-promoter. When the promoter is a heterologous constitutive promoter, then a suitable promoter includes the *apFab* family [SEQ ID Nos.:97], while a suitable inducible promoter includes: pBad (arabinose inducible [SEQ ID No.:38] and LacI [SEQ ID No.:40]. Suitable terminators include members of the *apFAB* terminator family including [SEQ ID No.: 98]. The selected promoter and terminator may be operably linked to the respective gene, either to provide individual gene regulation or for regulation of an operon.

### XVII A method for enhancing nitrogenase assembly and nitrogen fixation catalyzed by the NIF pathway and detecting nitrogenase activity in a genetically modified bacterium according to the invention

The assembly of the nitrogenase complex and nitrogen fixation in genetically modified prokaryotic cells of the invention (e.g. genetically modified *E. coli* cells) can be detected by introducing the cells into a suitable culture medium; and finally recovering the synthesized products, as illustrated in the Example 10. A suitable culture medium includes a carbon source selected from among glucose, maltose, galactose, fructose, sucrose, arabinose, xylose, raffinose, mannose, and lactose.

A method for the catalytic activity of the nitrogenase complex in a genetically modified prokaryotic cell of the invention is described in example 10.

The present invention provides a genetically modified prokaryotic cell capable of producing enhanced levels of the nitrogenase complex due to increased NifB mediated assembly, by measuring acetylene reduction.

### XVIII A genetically modified prokaryotic cell for production of indigo

According to a further embodiment, the present invention provides a genetically modified prokaryotic cell capable of indigo production via the indigo biosynthetic pathway (Figure 23).

The prokaryotic cell is genetically modified to express a mutant IscR, according to the invention (see section I and II), in substitution for a wild type IscR, as well as comprising transgenes or up-regulated endogenous genes (i.e an endogenous genes operably linked to a genetically modified regulatory sequence capable of enhancing expression of said endogenous genes, as described in section I and II) encoding a Rieske non-heme iron di-oxygenase complex (RDO). The RDO complex catalyzes the stereoselective insertion of two hydroxy groups into indole in one enzymatic step into cis - indole - 2,3 - dihydrodiol, which spontaneously oxidizes to indigo. In one embodiment the RDO comprises a dioxygenase (e.g. naphthalene dioxygenase (NDO) having naphthalene 1,2-dioxygenase activity (EC: 1.14.12.12), which itself is composed on 2 polypeptides (NdoB and NdoC); and a reductase having ferredoxin-NAD(P)+ reductase (naphthalene dioxygenase ferredoxin-specific) activity (EC: 1.18.1.7), also composed on 2 polypeptides (NdoA and NdoR).

A NdoB and NdoC polypeptides having naphthalene 1,2-dioxygenase activity (EC: 1.14.12.12), are characterized by an amino acid sequence having at least 70, 75, 80, 85, 90, 95, 96, 98, 100% amino acid sequence identity to: SEQ ID No.: 272 and 274 respectively (origin: *Pseudomonas pudita*)*.*

A NdoR and NdoA polypeptides having ferredoxin-NAD(P)+ reductase (naphthalene dioxygenase ferredoxin-specific) activity (EC: 1.18.1.7), are characterized by an amino acid sequence having at least 70, 75, 80, 85, 90, 95, 96, 98, 100% amino acid sequence identity to: SEQ ID No.: 276 and 278 respectively (origin: *Pseudomonas pudita*)*.*

When the genes encoding the RDO (e.g. NdoBCAR) Ndo, that mediate the indigo biosynthetic pathway are transgenes, they are each individually located in the genome of the genetically modified prokaryotic cell, either integrated into the prokaryotic cell chromosome or on a self-replicating plasmid. The transgenes RDO (e.g. NdoBCAR) may be present in the genome within one or more operon.

The promoter driving expression of the transgenes encoding RDO (e.g. NdoBCAR) is preferably a non-native promoter, which may be a heterologous constitutive-promoter or an inducible-promoter. When expression driven by the promoter is constitutive, then a suitable promoter includes apFab family [SEQ ID Nos.:97] while a suitable inducible promoter includes: pBad (arabinose-inducible) [SEQ ID No.:38] and lac promoter lac p, which is regulated by repressor lacI [SEQ ID No.:40]. Suitable terminators include members of the *apFAB* terminator family including [SEQ ID No.: 98]. The selected promoter and terminator may be operably linked to the respective gene, either to provide individual gene regulation or for regulation of an operon.

### XIX A method for enhancing indigo production by a genetically modified bacterium of the invention

The indigo can be produced using genetically modified prokaryotic cells of the invention (e.g. genetically modified *E. coli* cells) by introducing the cells into a suitable culture medium; and finally recovering the isoprenoid precursors or their derivatives, as illustrated in the Example 9. A suitable culture medium includes a carbon source selected from among glucose, maltose, galactose, fructose, sucrose, arabinose, xylose, raffinose, mannose, and lactose.

A method for quantifying IPP and DMAPP produced by a genetically modified prokaryotic cell of the invention is described in example 9.

The present invention provides a genetically modified prokaryotic cell capable of producing enhanced levels of PQQ as well as its precursors.

### XX SAM or AdoMet radical Fe-S enzyme activity in genetically modified prokaryotic cells of the invention is enhanced by increased electron transfer.

Fe_S cluster enzymes containing an oxidized [4Fe-4S]²⁺ cluster, e.g oxygen-independent coproporphyrinogen III oxidase synthase (EC: 1.3.98.3); NifH nitrogenase iron protein (EC: 1.18.6.1); IspG polypeptides having 4-hydroxy-3-methylbut-2-en-1-yl diphosphate synthase (EC: 1.17.7.3); HemN and IspH polypeptides having 4-hydroxy-3-methylbut-2-enyl diphosphate reductase activity (EC: 1.17.7.4), need electron transfer for reduction to a [4Fe-4S]⁺ cluster. Only the reduced [4Fe-4S]⁺ cluster is able generate the SAM-radical needed for catalysis. The electron transfer from the electron donor NADPH to the [4Fe-4S]²⁺ cluster can be mediated by a flavodoxin/ferredoxin reductase (Fpr) and flavodoxin (FldA) reduction system or by a Pyruvate-flavodoxin/ferredoxin oxidoreductase system.

In a further embodiment, the genetically modified prokaryotic cell according to the present invention, further comprises one or more genes selected from the group: a gene encoding a flavodoxin/ferredoxin-NADP reductase (EC: 1.18.1.2 and EC 1.19.1.1); a gene encoding a pyruvate-flavodoxin/ferredoxin oxidoreductase (EC: 1.2.7); a gene encoding a flavodoxin; a gene encoding a ferredoxin; a gene encoding a flavodoxin and a ferredoxin-NADP reductase. Promoter(s) or RBS sequences, operably-linked to each of said one or more genes are capable of enhancing expression of said one or more genes in said cell; wherein each said one or more genes may be a endogenous native gene or a transgene. Preferably, the operably-linked promoter or RBS, enhances expression of said one or more genes in said cell to a level greater than in the parent cell from which the genetically-modified bacterium of the invention was derived. Preferably, the genetically modified prokaryotic cell according to the present invention comprises a gene encoding a flavodoxin/ferredoxin-NADP reductase (EC: 1.18.1.2 and EC 1.19.1.1) and a gene encoding a flavodoxin; or a single gene comprising coding sequences for both a flavodoxin and a ferredoxin-NADP reductase. Additionally said genetically modified prokaryotic cell may further comprise a gene encoding a ferredoxin.

Overexpression of genes expressing components of the electron transfer pathway in genetically modified prokaryotic cells of the present invention, enhances the cellular activity of their SAM-radical iron-sulfur cluster enzymes (as illustrated in Example 2 for biotin-producing cells of the invention).

Preferably, when the polypeptide encoded by a native gene or transgene in the genetically modified prokaryotic cell of the invention has flavodoxin/ferredoxin reductase activity (EC: 1.18.1.2 and EC 1.19.1.1), it has an amino acid sequence having 80, 85, 90, 95 or 100% sequence identity to a sequence selected from any one of: SEQ ID No.: 43 (origin: *fpr* gene from *E. coli);* SEQ ID No.:45 (origin: *yumC* gene from *Bacillus subtilis* 168); SEQ ID No.:47 (origin: *fpr-I* gene from *Pseudomonas putida* KT2440); SEQ ID No.:48 (origin: SVEN_0113 gene from *Streptomyces venezuelae* ATCC 10712 -); SEQ ID No.:51 (origin: Cgl2384 gene from *Corynebacterium glutamicum* ATTCC 13032), and SEQ ID No.:53 (origin: SJN15614.1 gene from *Sphingobacterium* sp. JB170.

Preferably, when the polypeptide encoded by an endogenous native gene or transgene in the genetically modified prokaryotic cell of the invention has pyruvate-flavodoxin/ferredoxin oxidoreductase activity (EC: 1.2.7), it has an amino acid sequence having 80, 85, 90, 95 or 100% sequence identity to a sequence selected from any one of: SEQ ID No.: 55 (origin: *YdbK* gene from *E. coli* K12 MG1655); SEQ ID No.: 57 (origin: *por* gene from *Geobacter sulfurreducens* AM-1); SEQ ID No.: 59 (origin: Sfla_2592 gene from *Streptomyces pratensis* ATCC 33331; SEQ ID No.: 61 (origin: RM25_0186 gene from *Propionibacterium freudenreichii* DSM 20271); SEQ ID No.: 63 (origin: *nifJ* gene from *Synechocystis sp.* PCC 6803)

Preferably, when the polypeptide encoded by a endogenous native gene or transgene in the genetically modified prokaryotic cell of the invention is a flavodoxin, it has an amino acid sequence having 80, 85, 90, 95 or 100% sequence identity to a sequence selected from any one of: SEQ ID No.: 65 (origin: *fldA* gene from *Escherichia coli* K12 MG1655); SEQ ID No.: 67 (origin: *fldB* gene from *Escherichia coli* K12 MG1655); SEQ ID No.: 69 (origin: *ykuN* gene from *Bacillus subtilis* 168); SEQ ID No.: 71 (origin: *isiB* gene from *Synechocystis sp.* PCC 6803; SEQ ID No.: 73 (origin: *wrbA* gene from *Streptomyces venezuelae* ATCC 10712); SEQ ID No.: 75 (origin: PRK06242 gene from *Methanococcus aeolicus* Nankai-3).

Preferably, when the polypeptide encoded by a endogenous native gene or transgene in the genetically modified prokaryotic cell of the invention is a ferredoxin, it has an amino acid sequence having 80, 85, 90, 95 or 100% sequence identity to a sequence selected from any one of: SEQ ID No.: 77 (origin: *fdx* gene from *E. coli);* SEQ ID No.: 79 (origin: *fer* gene from *Bacillus subtilis* 168); SEQ ID No.: 81 (origin: *fdxB* gene from *Corynebacterium glutamicum* ATTCC 13032); SEQ ID No.: 83 (origin: *fdx* gene from *Synechocystis sp.* PCC 6803); SEQ ID No.: 85 (origin: SVEN_7039 gene from *Streptomyces venezuelae* ATCC 10712); SEQ ID No.: 87 (origin: *fdx* gene from *Methanococcus aeolicus* Nankai-3).

When a promoter is employed to enhance gene expression of an operably-linked endogenous native gene or to a transgene encoding a polypeptide of the electron transport pathway in said cell, it is preferably a non-native promoter. Said promoter may be a member of the constitutive apFAB309 promoter family [SEQ ID Nos.:93]. Preferably said non-native promoter, when operably-linked to said native gene or transgene enhances expression of said encoded polypeptide(s) in said genetically modified bacterium to a level greater than the parent bacterium from which it was derived. Suitable terminators that may be operably-linked to said endogenous native gene or transgene includes the apFAB terminator family [SEQ ID No.: 98].

### XXI Methods for engineering a genetically modified prokaryotic cell

Prokaryotic cells are genetically engineered by the introduction into the cells of transgenes or by the upregulation of expression of endogenous genes as illustrated in the Examples.

Genetic modification of endogenous genes in a prokaryotic cell of the invention can be performed by deletion (knockout) of the endogenous gene and insertion/substitution with a transgene encoding a mutant polypeptide as defined in section I and II, by applying standard recombineering methods to a suitable parent prokaryotic cell (Datsenko KA, et al.; 2000). Genetic modification of an endogenous gene sequence and/or regulatory sequence that are operatively linked to said endogenous gene can be performed by using a range of techniques known in the art, including recombineering (e.g. MAGE with single-strand DNA) and CRISPR-Cas gene editing.

The genetically modified prokaryotic cell according to the invention, may be a bacterium, a non-exhaustive list of suitable bacteria is given as follows: a species belonging to the genus selected from the group consisting of: *Escherichia, Brevibacterium, Burkholderia, Campylobacter, Corynebacterium, Pseudomonas, Serratia, Lactobacillus, Lactococcus, Acetobacter, Acinetobacter, Pseudomonas,* etc.

Preferred bacterial species of the invention are *Escherichia coli, Pseudomonas putida, Serratia marcescens and Corynebacterium glutamicum.*

In the case of nitrogen fixation, a preferred genetically modified bacterial species of the invention belongs to the genus *Rhizobium,* associated with leguminous plants (e.g., various members of the pea family); *Frankia,* associated with certain dicotyledonous species (actinorhizal plants); and *Azospirillum,* associated with cereal grasses.

### VIII SAM or AdoMet radical Fe-S enzyme activity in genetically modified prokaryotic cells of the invention is enhanced by increased electron transfer.

Fe_S cluster enzymes containing an oxidized [4Fe-4S]²⁺ cluster, e.g oxygen-independent coproporphyrinogen III oxidase synthase (EC: 1.3.98.3); NifH nitrogenase iron protein (EC: 1.18.6.1); IspG polypeptides having 4-hydroxy-3-methylbut-2-en-1-yl diphosphate synthase (EC: 1.17.7.3); HemN and IspH polypeptides having 4-hydroxy-3-methylbut-2-enyl diphosphate reductase activity (EC: 1.17.7.4), need electron transfer for reduction to a [4Fe-4S]⁺ cluster. Only the reduced [4Fe-4S]⁺ cluster is able generate the SAM-radical needed for catalysis. The electron transfer from the electron donor NADPH to the [4Fe-4S]²⁺ cluster can be mediated by a flavodoxin/ferredoxin reductase (Fpr) and flavodoxin (FldA) reduction system or by a Pyruvate-flavodoxin/ferredoxin oxidoreductase system.

In a further embodiment, the genetically modified prokaryotic cell according to the present invention, further comprises one or more genes selected from the group: a gene encoding a flavodoxin/ferredoxin-NADP reductase (EC: 1.18.1.2 and EC 1.19.1.1); a gene encoding a pyruvate-flavodoxin/ferredoxin oxidoreductase (EC: 1.2.7); a gene encoding a flavodoxin; a gene encoding a ferredoxin; a gene encoding a flavodoxin and a ferredoxin-NADP reductase. Promoter(s) or RBS sequences, operably-linked to each of said one or more genes are capable of enhancing expression of said one or more genes in said cell; wherein each said one or more genes may be a endogenous native gene or a transgene. Preferably, the operably-linked promoter or RBS, enhances expression of said one or more genes in said cell to a level greater than in the parent cell from which the genetically-modified bacterium of the invention was derived. Preferably, the genetically modified prokaryotic cell according to the present invention comprises a gene encoding a flavodoxin/ferredoxin-NADP reductase (EC: 1.18.1.2 and EC 1.19.1.1) and a gene encoding a flavodoxin; or a single gene comprising coding sequences for both a flavodoxin and a ferredoxin-NADP reductase. Additionally said genetically modified prokaryotic cell may further comprise a gene encoding a ferredoxin.

Overexpression of genes expressing components of the electron transfer pathway in genetically modified prokaryotic cells of the present invention, enhances the cellular activity of their SAM-radical iron-sulfur cluster enzymes (as illustrated in Example 2 for biotin-producing cells of the invention).

Preferably, when the polypeptide encoded by a native gene or transgene in the genetically modified prokaryotic cell of the invention has flavodoxin/ferredoxin reductase activity (EC: 1.18.1.2 and EC 1.19.1.1), it has an amino acid sequence having 80, 85, 90, 95 or 100% sequence identity to a sequence selected from any one of: SEQ ID No.: 241 (origin: *fpr* gene from *E. coli);* SEQ ID No.:243 (origin: *yumC* gene from *Bacillus subtilis* 168); SEQ ID No.:245 (origin: *fpr-I* gene from *Pseudomonas putida* KT2440); SEQ ID No.:247 (origin: SVEN_0113 gene from *Streptomyces venezuelae* ATCC 10712 -); SEQ ID No.:249 (origin: Cgl2384 gene from *Corynebacterium glutamicum* ATTCC 13032), and SEQ ID No.:251 (origin: SJN15614.1 gene from *Sphingobacterium* sp. JB170.

Preferably, when the polypeptide encoded by an endogenous native gene or transgene in the genetically modified prokaryotic cell of the invention has pyruvate-flavodoxin/ferredoxin oxidoreductase activity (EC: 1.2.7), it has an amino acid sequence having 80, 85, 90, 95 or 100% sequence identity to a sequence selected from any one of: SEQ ID No.: 253 (origin: *YdbK* gene from *E. coli* K12 MG1655); SEQ ID No.: 255 (origin: *por* gene from *Geobacter sulfurreducens* AM-1); SEQ ID No.: 257 (origin: Sfla_2592 gene from *Streptomyces pratensis* ATCC 33331; SEQ ID No.: 259 (origin: RM25_0186 gene from *Propionibacterium freudenreichii* DSM 20271); SEQ ID No.: 261 (origin: *nifJ* gene from *Synechocystis sp.* PCC 6803)

Preferably, when the polypeptide encoded by a endogenous native gene or transgene in the genetically modified prokaryotic cell of the invention is a flavodoxin, it has an amino acid sequence having 80, 85, 90, 95 or 100% sequence identity to a sequence selected from any one of: SEQ ID No.: 263 (origin: *fldA* gene from *Escherichia coli* K12 MG1655); SEQ ID No.: 265 (origin: *fldB* gene from *Escherichia coli* K12 MG1655); SEQ ID No.: 267 (origin: *ykuN* gene from *Bacillus subtilis* 168); SEQ ID No.: 269 (origin: *isiB* gene from *Synechocystis sp.* PCC 6803; SEQ ID No.: 271 (origin: *wrbA* gene from *Streptomyces venezuelae* ATCC 10712); SEQ ID No.: 273 (origin: PRK06242 gene from *Methanococcus aeolicus* Nankai-3).

Preferably, when the polypeptide encoded by a endogenous native gene or transgene in the genetically modified prokaryotic cell of the invention is a ferredoxin, it has an amino acid sequence having 80, 85, 90, 95 or 100% sequence identity to a sequence selected from any one of: SEQ ID No.: 275 (origin: *fdx* gene from *E. coli);* SEQ ID No.: 277 (origin: *fer* gene from *Bacillus subtilis* 168); SEQ ID No.: 279 (origin: *fdxB* gene from *Corynebacterium glutamicum* ATTCC 13032); SEQ ID No.: 281 (origin: *fdx* gene from *Synechocystis sp.* PCC 6803); SEQ ID No.: 283 (origin: SVEN_7039 gene from *Streptomyces venezuelae* ATCC 10712); SEQ ID No.: 285 (origin: *fdx* gene from *Methanococcus aeolicus* Nankai-3).

When a promoter is employed to enhance gene expression of an operably-linked endogenous native gene or to a transgene encoding a polypeptide of the electron transport pathway in said cell, it is preferably a non-native promoter. Said promoter may be a member of the constitutive apFAB309 promoter family [SEQ ID Nos.:93]. Preferably said non-native promoter, when operably-linked to said native gene or transgene enhances expression of said encoded polypeptide(s) in said genetically modified bacterium to a level greater than the parent bacterium from which it was derived. Suitable terminators that may be operably-linked to said endogenous native gene or transgene includes the apFAB378 terminator family [SEQ ID No.: 41].

### Examples

### Example 1: Identification and characterization of genetically modified E. coli strains capable of enhanced biotin production

1.01: The following strains of *Escherichia coli* used in the examples are listed below.

**Table 1: Strains**

| **Name** | **Description** |
|---|---|
| BS1013 | *E. coli* K-12 BW25113 parent strain having genotype: rrnB3 ΔlacZ4787 hsdR514 Δ(araBAD)567 Δ(rhaBAD)568 rph-1 |
| BS1011 | *ΔbioB* ¹(JW0758-1) derived from *E. coli* K-12 BW25113 |
| BS1353 | BS1011 derivative comprising a H107Y mutation in *iscR* |
| BS1113 | BS1011 derivative comprising pBS412 plasmid giving IPTG - inducible BioB expression |
| BS1375 | BS1011 derivative comprising a C92Y mutation in *iscR* |
| BS1377 | BS1011 derivative comprising a L15F mutation in *iscR* |

| | |
|---|---|
| *¹* Nucleotide sequence of *ΔbioB* gene prior to deletion was SEQ ID No. 33 | |

1.02: The following plasmids used in the examples are listed below.

**Table 2: Plasmids**

| **Name** | **Description** |
|---|---|
| pBS412 | BioB [SEQ ID No: 34] overexpression plasmid (kanR, SC101) from a T5 lacO repressed promoter [SEQ ID No.: 37] |
| pBS430 | pBS412 with frame shift mutation early in *bioS* ¹(kanR, SC101) [SEQ ID No.: 36] from a T5 lacO repressed promoter [SEQ ID No.: 37] |
| pBS451 | Constitutively expressed GFP [SEQ ID No.: 89] (zeoR, p15A) |
| pBS281 | *E. coli isc* operon (iscSUA-hscBA-fdx) from an IPTG inducible T5 promoter cloned in a medium copy number plasmid (p15A ori) |
| pBS282 | *E. coli suf* operon (*sufABCDSE*) from an IPTG inducible T5 promoter cloned in a medium copy number plasmid (p15A ori) |
| pBS231 | A medium copy number plasmid (p15A ori) expressing a gene encoding a sfGFP protein from an IPTG inducible T5 promoter |
| pBS936 | Native biotin-operon from *E. coli* with "type 9" mutation in *bio* operator site (Ifuku et al., 1993) |

| | |
|---|---|
| : Nucleotide sequence of *bioB* frameshift gene has SEQ ID No. 35 | |

### 1.03 Media and additives:

The growth media (mMOPS) used in each example had the following composition: 1.32 mM K2HPO4; 2 g/L D-glucose; 0.0476 mg/L calcium pantothenate; 0.0138 mg/L p-aminobenzoic acid; 0.0138 mg/L p-hydroxybenzoic acid; 0.0154 mg/L 2,3-dihydroxybenzoic acid, and 1x modified MOPS buffer.

10x modified MOPS comprises 0.4 M MOPS (3-(N-morpholino) propane sulfonic acid); 0.04 M Tricine; 0.1 mM FeSO₄•7H₂O; 95 mM NH₄Cl; 2.76 mM K₂SO₄; 5 µM CaCl₂•2H₂O; 5.25 mM MgCl₂; 0.5 M NaCl; and 5000x dilution of micronutrient stock solution.

**Table 3: Micronutrient stock solution:**

| **Component** | **Formula** | **FW** | **Grams per 50 mL** |
|---|---|---|---|
| ammonium molybdate | (NH₄)₆Mo₇O₂₄•4H₂O | 1235.9 | 0.009 |
| boric acid | H₃BO₃ | 61.83 | 0.062 |
| cobalt chloride | CoCl₂ | 237.9 | 0.018 |
| cupric sulfate | CuSO₄ | 249.7 | 0.006 |
| manganese chloride | MnCl₂ | 197.9 | 0.040 |
| zinc sulfate | ZnSO₄ | 287.5 | 0.007 |

The following antibiotic stocks were employed: ampicillin (amp, 100 mg/mL), kanamycin (kan, 50 mg/mL), zeocin (zeo, 40 mg/mL); that were added to growth media as indicated to obtain a 1000x dilution.

### 1.04 Establishing of E. coli strain libraries:

*E. coli* libraries having evolved genomic diversity were derived from cells of *E*. *coli* strain BS1011 comprising plasmid pBS412 by subjecting the cells to stationary overnight culture in mMOPS medium supplemented with kan (MOPS-kan), preparing a 100x dilution of resulting culture in mMOPS-kan and repeating the consecutive steps of overnight culture and dilution 5 times. This procedure creates genetic diversity by allowing the accumulation of background mutation generated by imperfect error-correcting polymerases.

After each round of culture and dilution a sample of the cell culture was plated on mMOPS plates with IPTG (see below), to detect the evolution of cells adapted to tolerate enhanced BioB expression. Cells of each library were then transformed with the BioB over-expression plasmid, pBS412.

### 1.05 Selection of mutant strains

A selection assay was developed by plating respectively 10⁴, 10⁵, 10⁶ and 10⁷ cells, derived from an o/n culture in mMOPS-kan of BS1011 comprising pBS412, on a series of 1.5 % agar plates comprising mMOPS (Ø=9cm) comprising IPTG concentrations of either 0, 0.0001, 0.001, 0.01, 0.1 and 1 mM. The plates were then incubated at 37°C for up to 36 hours and cell growth was evaluated at intervals. Under these conditions, induction of BioB expression from pBS412 with 0.1 mM IPTG was found to prevent growth of up to 10⁵ cells, while induction with 1 mM IPTG prevented growth of at least 10⁷ cells, when plated on a single petri dish. A selection pressure comprising induction with 1mM IPTG for a cell population of 10⁵ cells was found optimal for identifying strains with higher robustness towards BioB expression; and accordingly was implemented as follows:
1) Approximately 10⁵ cells from each library, as described in section 1.4, were plated on each mMOPS-kan -1 mM IPTG agar plate and incubated at 37 °C for maximum 24 hours.
2) Single colonies were grown in mMOPS-kan liquid medium to produce pre-cultures, that were then evaluated for their biotin production by means of a biotin bioassay (as described in section 1.6 below) that was performed in mMOPS-kan supplemented with either 0.00, 0.01 or 0.1 mM IPTG. Cells of each pre-culture were preserved as glycerol stocks in 20% glycerol.
3) Colonies producing more than 1.5 mg biotin/L (detected as extracellular biotin) were re-streaked on mMOPS-kan agar plates, incubated at 37 °C for up to 24 hours, and then re-bioassayed for biotin production in biological replicates (as detailed in section 1.6 below).
4) Cells of the selected biotin over-producing strains were evaluated as follows:
   a) Whole genome sequencing to identify genetic mutations in the genome of cells of selected strains as compared to the genome of the parent strain BS1011 was performed on DNA isolated from cells of the selected strain as follows: Selected cells were grown in 5-10 mL mMOPS-kan and the cells were subsequently harvested; genomic DNA was isolated from the harvested cells using Invitrogen Purelink genomic DNA extraction kit: (https://www.thermofisher.com/order/catalog/product/K182001); the extracted DNA was subjected to whole genome sequencing.
   b) Curing cells of the selected strains of their pBS412 plasmid; then retransforming the cells of the cured strain with the pBS412 plasmid; and finally redoing bioassay of cultures of cells of the transformed strains for biotin production. The steps of curing cells of their pBS412 plasmid were performed by growing the cells in rich, Luria Broth (LB) media with 1 mM IPTG without antibiotics overnight at 37 °C; streaking out cells of the resulting culture on LB agar plates and incubating overnight at 37 °C. Single colonies from the agar plates were diluted in 50 µl LB media and 5 µl were used to spot on LB and LB-amp agar plates, which were incubated overnight at 37 °C. Those single colonies that grew on LB plates, but not on LB-amp plates, were re-streaked on LB plates to obtain single colonies, which were used as cured strain for re-transformation.
   c) Biotin production by cells of the transformed strains where measured in biological replicates (as detailed in section 1.6 below). Briefly, biotin production re-evaluated for biological replicates in mMOPS-kan supplemented with 0.00, 0.01 or 0.1 mM IPTG. In parallel, the growth rates of the cells of each transformed strain were measured in 200 µL mMOPS-kan medium in a microtiter plate sealed with transparent breathable seal at 37 °C with "fast shaking" for aerobic growth, for a period of 24 hours in a Multiskan FC. Cell growth was monitored by measuring OD₆₂₀ₙₘ every 30 minutes.

### 1.06 Bioassay for quantifying biotin production

Pre-cultures were each prepared from a selected single cell colony in 400 µL mMOPS-kan in a 96 deep-well plate, incubated at 37 °C with shake at 275 rpm for 16-18 hours. Production cultures were produced by inoculating 400 µL mMOPS-kan, supplemented with 0.1 g/L desthiobiotin (DTB), and optionally comprising IPTG at a final concentration of up to 1 mM, in a 96 deep-well plate, with 4 µL of the pre-culture enough to provide an initial OD600 of ∼0.03. Cultures were then grown at 37 °C with 275 rpm shake for 24 hours. Cells in the 96 deep-well plate were pelleted by centrifuging at 4000 G for 8 minutes after measuring OD₆₀₀ of the cultures. The supernatant from each culture supernatant was diluted to a concentration range of 0.05 nM to 0.50 nM biotin in ultrapure (Milli-Q) water. In parallel, >5 biotin standards in the concentration range of 0.1 nM (0.024 µg biotin/L) to 1 nM (0.24 µg biotin/L), were prepared in Milli-Q water. 15 µL of each diluted supernatant and each of the biotin standards was then added to a well of a microtiter plate; wherein each well comprised 135 µL of a biotin-starved overnight culture of BS1011 comprising plasmid pBS451; and where the overnight culture was diluted to an initial OD₆₂₀ of 0.01 in mMOPS supplemented with zeocin. The plate was sealed with a breathable seal and incubated at 37 °C with 275 rpm shaking for 20 hours before OD₆₂₀ₙₘ was measured. A biotin bioassay calibration curve obtained with this bioassay, using a range of biotin standards, is shown in Figure 5.

### 1.07 Identification of genomic mutations

For all Next-Generation Sequencing (NGS) data, CLC genomic workbench version 9.5.3 (supplied by Qiagen) was used to identify mutations in the genome of cells of selected strains as compared to the parent strain genome (single or a few substitutions, deletions or insertions by Variant Detection and bigger insertions/deletions by InDels and Structural Variants). A cut-off of 85% were used to define "significant mutations" meaning that a mutation should be present in more than 85% of the population of DNA molecules (genomes) isolated from cells of a given bacterial strain, in order to distinguish genome mutations from erroneous nucleotides introduced by the sequencing procedure.

The genome accession number CP009273 from NCBI was used as the reference sequence, while taking account of the Keio *ΔbioB* scar mutation whose sequence was confirmed by sequencing.

### 1.08 Characterizing proteomics landscape of iscR mutant

Protein content of BS1013 + pBS430, BS1011 + pBS412 and BS1353 + pBS412 at 0.025 mM IPTG induction levels as well as BS1353 + pBS412 at 1 mM IPTG induction were determined by a recently developed approach combining LC-MS and efficient protein extraction (Schmidt et al, 2015). 3 peptides were chosen as minimum number of identified peptides for analysis along with a peptide threshold of 2.0 % FDR. Significant changes in protein expression are reported with a 0.5 % confidence interval based on Analysis of Variance (ANOVA) with Benjamini-Hochberg correction for multiple testing using a Scaffold Viewer 4.7.5.

Strains were grown in mMOPS with IPTG induction for approximately 10 generations, until OD₆₀₀ of 0.5 were reached (exponential phase). 10⁸ cells were harvested by centrifugation at 4 °C at maximum speed; washed once in ice-cold PBS buffer; re-pelleted by centrifugation at 4 °C at maximum speed and snap-frozen in liquid nitrogen, after removing PBS buffer.

### 1.09 Overexpression of BioB is toxic

*E. coli* retaining its native biotin synthase gene (*bioB*) but expressing an IPTG-inducible frameshifted *E. coli bioB* gene (encoding non-functional biotin synthase due to a premature stop codon) from a low-copy plasmid (Sc101 origin of replication) is able to grow aerobically in mMOPS-kan medium with or without IPTG. This is illustrated in Figure 4 (left panel), showing the exponential growth curve of the *E. coli* BW25113, BioB frameshift mutant. In contrast, over-expression of a functional biotin synthase gene (*bioB*) in the *E*. *coli* knock-out strain Δ*bioB,* is toxic for growth, causing a very significant extension in the lag phase. This is illustrated in Figure 4 (right panel), that shows the growth of *E. coli* knock-out strain Δ*bioB* expressing an *E. coli bioB* gene from an IPTG-inducible T5 promoter on a low-copy plasmid (Sc101 origin of replication). As seen in Figure 4, induction of increasing *bioB* expression in response to increasing IPTG levels (darkness of grey) significantly affects the lag-phase while the growth rate is affected slightly (black boxes).

### 1.10 Isolation of iscR mutant strains having enhanced biotin production titers

*E. coli* libraries having evolved genomic diversity (see sections 1.4 and 1.5) were screened for strains with improved tolerance for *bioB* gene expression and increased biotin production. Whole genome sequencing of the selected strains led to the identification of three unique mutants each comprising an Iron-sulfur cluster regulator (*iscR*) gene encoding an iscR polypeptide having one of the amino acid substitutions: L15F, C92Y and H107Y, and where the amino acid sequence of the encoded regulators is SEQ ID No.: 16, 18 and 20 respectively. Biotin production levels were measured using a bioassay, as described in section 1.6 (and Figure 5). The biotin production titers for each of the *iscR* mutant strains as well as the *E. coli* BW25113 *ΔbioB* reference strain are shown in Figure 6 for 4 biological replicates (black dots) grown in mMOPS supplemented with 0.1 g/L DTB in the absence or presence of two different IPTG concentration levels (increasing IPTG with darker grey). Biotin production in the reference strain was inhibited at IPTG levels of above 0.01 mM, which corresponds to IPTG levels that are toxic for growth of the reference strain (see Figure 4), while the *iscR* mutant strains both grew and produced biotin at IPTG levels of 0.01 - 0.1mM IPTG. All three *iscR* mutant strains produce approximately 1.5-fold more biotin than the reference strain (stippled line) at an IPTG concentration of 0.01 mM. The *iscR* mutant strains produce up to 2-fold more (∼3.2 mg biotin/L) at an IPTG concentration of 0.1 mM, than the highest production titer from the reference strain (∼1.5 mg biotin/L).

### 1.11 Biotin production and growth in an IscR H107Y mutant strain

The growth profile and biotin production titer of the *iscR* (H107Y) mutant strain was characterized in 50 mL mMOPS supplemented with 0.1 g/L DTB in a 250 mL shake-flask experiment at two different IPTG induction levels (0.01 mM in Figure 7A) and 0.5 mM (Figure 7B). At low IPTG levels (Figure 7A), the IscR mutant strain (dark grey) and the reference strain (light grey) were similar with respect to growth and biotin production titers, with a final titer of ∼1.1 mg biotin/L. However, at high IPTG induction levels (0.5 mM in Figure 7B) growth of the reference strain (light grey) was severely inhibited, while the IscR mutant strain retained the same growth profile as at low IPTG induction levels. Furthermore, the biotin production titers of the IscR mutant strain increased around 2-fold, up to ∼2.2 mg biotin/L after 25 hours of growth.

### 1.12 Mechanism of action of IscR mutations

An enhanced biotin tolerance phenotype was clearly demonstrated for all three of the identified IscR mutant strains, as seen in Figure 6. The ability of the C92 mutation (C92Y) to enhance biotin tolerance is suggested to be due to a role for C92 in the [Fe-S] cluster binding properties of IscR. Loss of [Fe-S] cluster binding properties due to the C92Y mutation is proposed to inactivate the Isc-operon repression behavior of IscR. At the same time, it is proposed that the promoter function of IscR remains intact in the C92Y mutant IscR, such that it retains its function in activating other pathways essential for multiple cellular processes. A similar essential role in providing the [Fe-S] cluster binding properties of IscR is attributed to H107; where the H107Y mutation is similarly able to enhance biotin tolerance in *E. coli.* The L15F in IscR is also proposed to disrupt iron-sulfur cluster binding and thereby partially overcome iron-sulfur cluster depletion. Figure 9 shows the position of the L15 and H107 in IscR when bound to DNA (binding site of hya, PDB entry 4HF1), and residue L15 is positioned at the inside of each of the IscR subunits. Phenylalanine is a significantly larger amino acid than leucine, and it may interfere with the three-dimensional folding of the protein.

### 1.13 Overexpression of the isc-operon or suf operon in E. coli strains alone is not sufficient to enhance biotin production

In order to determine the direct effect of overexpressing the *isc*-operon (*iscSUA-hscBA-fdx,* corresponding to the native *E. coli* operon structure minus the *iscR* gene) or the suf-operon (sufABCDSE corresponding to the native *E*. *coli* operon structure) on biotin production in *E. coli,* each operon was cloned into a medium copy number plasmid (p15A ori) placed under the control of a strong RBS and an IPTG inducible T5 promoter. A plasmid, comprising a gene encoding a super folder Green Fluorescent Protein (sfGFP) in substitution for the isc- or suf-operon, was employed as a control. The respective plasmids were transformed into cells of an *E. coli* strain comprising an IPTG-inducible *bioB* expression plasmid. Biological triplicate colonies comprising one of: 1) IPTG-inducible *isc*-operon, 2) IPTG-inducible suf-operon or 3) IPTG-inducible GFP (control) in addition to the IPTG-inducible *bioB* expression plasmid were assayed for biotin production (as described in section 1.5) following cultivation in 400 µL mMOPS with 100 µg/mL ampicillin and 50 µg/mL spectinomycin under low (0.01 mM IPTG) and high (0.1 mM IPTG) induction.

Although biotin production was IPTG-inducible in all strains (Figure 10); the IPTG concentration needed to reach detectable biotin production levels was increased from 0.01 mM IPTG to 0.1 mM IPTG, when compared to the reference and mutant *iscR* strains shown in Figure 6. Furthermore, biotin production titers were significantly decreased by the overexpression of *isc-* or suf-operons, when compared to both the *sfGFP* strain in Figure 10. Additionally, overexpression of *isc*-operon depressed biotin production even more than overexpression of suf-operon. Taken together with the observed increase in biotin production seen in mutant strains having a single point mutation in *iscR* (Figure 6), it is unlikely that the resulting de-repression of *isc*-operon in these strains is the only/main reason for improved biotin production in those strains.

### 1.14 BioB protein contents correlates with biotin production

To investigate the molecular effects of BioB overexpression in wild type and mutant background strains, proteomics measurements were carried out for a wild type background strain: BS1013 holding pBS430; a wild type *iscR* strain with a *bioB* production plasmid: BS1011 holding pBS412; and a mutant *iscR* strain with a *bioB* production plasmid: BS1353 holding pBS412. All strains were grown in mMOPS with 0.1 g/L DTB and 0.025 mM IPTG. The latter strain was additionally grown at 1 mM IPTG induction. Cells were harvested for proteomics analysis in exponential phase, while the remaining cell culture were kept incubating for 24 hours in total, before biotin production were measured using the bioassay described elsewhere.

From the graph (Figure 11) the measured BioB protein level strongly correlates with the biotin production (R² value of 0.96). The linear correlation shows that facilitating enhanced BioB expression is a key to improve biotin production in IscR mutant cell factories. The ANOVA analysis of the proteomics data revealed a significant increase in expression (95% confidence interval, p-value of 0.00166) in additional 29 proteins. Among these are members of the *isc*-operon (IscA and IscS) and suf-operon (SufB and SufS).

### 1.15 Biotin production is not enhanced in iscR knockout mutants

A translational knockout of the *iscR* gene was introduced into a BW25113 *ΔbioB* strain by MAGE, by converting the codon encoding glutamic acid on position 22 in iscR (E, GAA) into a stopcodon (*, TGA). Successful conversion of the codon was verified by PCR amplification of the region followed by Sanger sequencing. Strains with genes encoding wild type *iscR, iscR* knockout (E22*), and mutant *iscR* (C92Y) were transformed with IPTG-inducible *bioB* plasmid pBS412, and tested for biotin production in biological replicates (n=3) grown in mMOPS supplemented with 0.1 g/L DTB and 50 µg/L kanamycin at three different IPTG induction levels (0, 0.01, and 0.1 mM) as described above.

No significant differences in biotin production were observed between the *iscR* knockout (*iscR* KO) and the wild type *iscR* (*iscR* WT) strains when inducing *bioB* expression by IPTG induction. This provides evidence that knocking out *iscR* does not improve biotin production. Significant improvement in biotin production was again observed for the mutant *iscR* encoding IscR C92Y substitution as compared to both *iscR* WT and *iscR* KO strains.

### 1.16 De-novo biotin production is enhanced in iscR mutant strains of the invention

A BW25113 *E. coli* strain from which both the *bioA* gene and the entire biotin-operon (*ΔbioB-bioD*) were deleted and comprising either *iscR* WT, *iscR* H107Y mutant or *iscR* C92Y mutant genes, were transformed with a tetracycline resistant plasmid, constitutively overexpressing the native *E. coli bioA* and biotin-operon, with a single point mutation in the *bioO* operator site (Type 9 mutation, Ifuku et al., 1993). Biotin production was evaluated for the three different strains in biological replicates (n=4) in mMOPS (2 g glucose/L) with 10 µg/ml tetracycline with and without the addition of 0.1 g/L DTB as described above (Figure 13).

A significant increase in biotin titers were observed for all three strains when the substrate, DTB, was added to the growth medium, indicating that the *bioB* enzyme reaction itself, converting DTB to biotin, is no longer a bottleneck for biotin production in these strains (Figure 13). Furthermore, a significant increase in de-novo production of biotin from glucose was observed for both *iscR* mutant strains as compared to the *iscR* WT strain. On view of these results it may deduced that the mutant *iscR* strains of the invention all support enhanced biotin production from both the direct precursor, DTB, and from glucose.

### Example 2 Overexpression of a flavodoxin/ferredoxin reductase (Fpr) and flavodoxin (FldA) reduction system to increase productivity of genetically modified E. coli strains capable of producing biotin

2.01: The following strains of *Escherichia coli* used in the examples are listed below.

**Table 4: Strains**

| **Name** | **Description** |
|---|---|
| BS1011 | ΔbioB (JW0758-1) derivative of *E. coli* K-12 BW25113 parent strain having genotype: rrnB3 ΔlacZ4787 hsdR514 Δ(araBAD)567 Δ(rhaBAD)568 rph-1 |
| BS1353 | BS1011 derivative comprising a H107Y mutation in iscR |
| BS1615 | BS1011 derivative with additional deletion of ΔbioAFCD |
| BS1937 | BS1615 derivative comprising pBS679 plasmid giving IPTG - inducible BioB expression |
| BS2185 | BS1615 derivative comprising pBS679 plasmid giving IPTG - inducible BioB expression and pBS1112 giving constitutive FldA-Fpr expression |
| BS2707 | BS1615 derivative comprising pBS679 plasmid giving IPTG - inducible BioB expression and pBS1054 giving constitutive GFP expression |

The following plasmids used in the example are listed below.

**Table 5: Plasmids**

| **Name** | **Description** |
|---|---|
| pBS679 | BioB [SEQ ID No.: 34] overexpression plasmid (ampR, pSC101) from a T5 lacO repressed promoter [SEQ ID No: 37] and |
| pBS1054 | GFP [SEQ ID No.: 89] overexpression plasmid (kanR, pBR322) from a constitutive apFAB309 promoter [SEQ ID No.: 93] with an apFAB378 terminator [SEQ ID No.: 41]. |
| pBS1112 | FldA-Fpr overexpression plasmid (kanR, pBR322) from a constitutive apFAB306 promoter (apFAB306-FldA-Fpr gene- apFAB378 terminator [SEQ ID No: 90]) |

An IPTG-inducible transgene encoding BioB was cloned on plasmid pBS679; a constitutively-regulated transgene encoding GFP was cloned on plasmid pBS1054; and a constitutively-regulated transgene comprising a synthetic operon encoding FldA-Fpr was cloned on plasmid pBS1112. pBS679 was introduced into an *E. coli* host strain (BS1615) in which the native *iscR* gene was substituted by a mutant *iscR* gene encoding an IscR protein having an H107Y substitution, as described in Example 1, and further comprising a knock-out of the *bioAFCD* genes resulting in the strain BS1937. The strain BS1937 was then further transformed with either plasmid pBS1054 or pBS1112 resulting in the strains BS2707 (control strain) and BS2185, respectively.
The strains were cultured in mMOPS medium (as described in example 1.3) with appropriate antibiotic(s), 0.1 g/L DTB as substrate for BioB-mediated catalysis, and supplemented with either 0, 0.01, 0.025, 0.05, 0.075 or 0.1 mM IPTG for inducing expression of the *BioB* gene. The cells were incubated for 24 hours at 37 °C in individual wells of a deep well culture plate. End OD₆₀₀ₙₘ values were estimated, supernatants were harvested by centrifugation and biotin quantified from the supernatants by a biotin bioassay carried out as described in example 1.6.

As shown in Figure 10 and Figure 11 for strain BS1937, when *BioB* gene expression in *E. coli* cells comprising a genetically modified endogenous *iscR* gene, are induced with increasing concentrations of IPTG, the cells show a corresponding progressive increase in biotin production. Biotin production in these genetically modified cells is further enhanced 2.12-fold by the co-expression of a transgene encoding FldA-Fpr (strain BS2185) when compared with its parent strain BS1937, and with a control strain expressing a transgene encoding GFP instead of FldA-Fpr.

### Example 3: Engineering and characterization of genetically modified E. coli strains capable of enhanced heme production

### 3.01 Heme production by an IscR H107Y mutant strain overexpressing hemN and hemB genes.

The following strains of *Escherichia coli* used in the example are listed below.

**Table 6: Strains**

| **Name** | **Description** |
|---|---|
| BS1011 | ΔbioB (JW0758-1) derived from *Escherichia coli* K-12 BW25113 having genotype : rrnB3 ΔlacZ4787 hsdR514 Δ(araBAD)567 Δ(rhaBAD)568 rph-1 |
| BS1353 | BS1011 derivative comprising an H107Y mutation in iscR |
| BS3128 | BS1011 transformed with pBS1610 |
| BS3129 | BS1353 transformed with pBS1610 |
| BS2629 | BS1011 transformed with pBS1259 |
| BS2630 | BS1353 transformed with pBS1259 |

The following plasmids used in the example are listed below.

**Table 7: Plasmids**

| **Name** | **Description** |
|---|---|
| pBS1610 | Plasmid (AmpR, p15A) comprising an hemN gene [SEQ ID No.: 95] and hemB gene [SEQ ID No.: 109] operably linked to an IPTG-inducible T5 LacO repressed promoter [SEQ ID No.: 37] and a rrnB terminator [SEQ ID No.: 94]. |
| pBS1259 | Control empty plasmid (AmpR, p15A) comprising a T5 LacO inducible promoter [SEQ ID No.: 39], a RBS, where AmpR is the sole open reading frame. |

IPTG-inducible genes encoding HemN and HemB were cloned to give plasmid pBS1610; and an empty plasmid pBS1259 having a p15A backbone was used as a control. pBS1610 or pBS1259 were introduced into the *E. coli* host strain (BS1353) in which the native iscR gene was substituted by a mutant iscR gene encoding an IscR protein having an H107Y substitution (as described in Example 1) resulting in the strains BS3129 and BS2630, respectively. Similarly, the plasmids pBS1610 and pBS1259 were introduced into *E. coli* strain BS1011 carrying a wild-type version of the iscR gene, resulting in strains BS3128 and BS2629, respectively.

Cells of each strain (Table 6) are cultivated in mMOPS medium (as described in Example 1.03) supplemented with 1 nmol/L biotin and 100 µg/mL ampicillin at 37 °C until a cell density (OD₆₀₀ₙₘ) of 0.6 is reached. IPTG is then added to the growth medium (to a final concentration of 0.1 mM) to induce HemN and HemB synthesis. Heme production by the cells is performed by additionally adding aminolaevulinic acid (ALA) to the medium at final concentration of 10 mM; in order to enhance HemB mediated-catalysis and flux through the heme pathway flux; and cultivating the cells for a further 48 h period under anaerobic conditions.
The free porphyrin and heme content of the cultured cells is determined according to Fyrestam and Oestman, 2017. Cells in each culture are centrifuged (17000 g, 5 min), the supernatant is discarded, and each cell pellet is re-suspended in Tris-EDTA pH 7.2 solution and the cells are sonicated in 5 sec bursts of 20-50 KHz. Each sonicated sample is then centrifuged (17000 g, 5 min) and the respective supernatant collected, followed by addition of 3 volumes of 100 w% acetonitrile. Each sample is vortexed for 5 min and centrifuged (2500 g, 5 min). Finally, the supernatant of each sample is collected for HPLC analysis using a C18 reverse-phase column; using a mobile phase consisting of water, acetonitrile and 0.1 M formic acid (pH 5.1-5.2). Free porphyrins and free heme titer of each collected sample is also quantified using a Quantichrom heme assay kit from BioAssay systems (coloration of porphyrins and reading at 400 nm).

The tests demonstrate that over-expression of a hemN gene encoding a hemN Fe-S polypeptide in combination with a HemB gene in an *E. coli* strain comprising a gene encoding a mutant form of the IscR protein (IscR protein having an C92Y or H107Y substitution) leads to both a more stable production and increased in the heme and prophyrin titer as compared to over-expression of the *HemN* and *HemB*-encoding gene in a parent *E. coli* strain comprising a gene encoding the native, wild-type form of the IscR protein.

### 3.02 Heme production in an IscR H107Y mutant strain overexpressing 'hemN or hemF' gene in combination with hemALBCDEGH genes

The parent *E. coli* used in the example listed below, was modified to knock-out the *yfeX* gene encoding a putative heme degradation enzyme; as well as to knock-out the *pta* and *IdhA* genes encoding phosphate acetyl transferase and lactate dehydrogenase, respectively, in order to enhance metabolic flux from glucose to L-glutamate. A derivative of the *E. coli* ΔyfeX-LdhA-Pta strain further comprised an iscR gene encoding an H107Y mutation in the iscR protein.

**Table 8A**

| Derivative of *E*. *coli* strain* characterised by: | Plasmids comprising IPTG-inducible heme pathway genes: |
|---|---|
| ΔyfeX-LdhA-Pta | pRSFhemBCD, pET-hemEFGH and pCDF-hemA(wt)L; KmR, ApR, SmR ** |
| ΔyfeX-LdhA-Pta H107Y IscR | pRSFhemBCD, pET-hemEFGH and pCDF-hemA(wt)L; KmR, ApR, SmR ** |
| ΔyfeX-LdhA-Pta | pRSFhemBCD, pET-hemENGH and pCDF-hemA(wt)L; KmR, ApR, SmR ** |
| ΔyfeX-LdhA-Pta H107Y IscR | pRSFhemBCD, pET-hemENGH and pCDF-hemA(wt)L; KmR, ApR, SmR ** |
| ΔYfeX-LdhA-Pta | pRSFhemBCD, pET-hemEFGH and pCDF-hemA(kk)L; KmR, ApR, SmR ** |
| ΔYfeX-LdhA-Pta H107Y IscR | pRSFhemBCD, pET-hemEFGH and pCDF-hemA(kk)L; KmR, ApR, SmR ** |
| ΔyfeX-LdhA-Pta | pRSFhemBCD, pET-hemENGH and pCDF-hemA(kk)L; KmR, ApR, SmR ** |
| ΔyfeX-LdhA-Pta H107Y IscR | pRSFhemBCD, pET-hemENGH and pCDF-hemA(kk)L; KmR, ApR, SmR ** |
| ΔyfeX-LdhA-Pta | pRSFhemBCD, pET-hemEFGH and pCDF-hemA(C107A)L; KmR, ApR, SmR ** |
| ΔyfeX-LdhA-Pta H107Y IscR | pRSFhemBCD, pET-hemEFGH and pCDF-hemA(C107A)L; KmR, ApR, SmR ** |
| ΔyfeX-LdhA-Pta | pRSFhemBCD, pET-hemENGH and pCDF-hemA(C107A)L; KmR, ApR, SmR** |
| ΔyfeX-LdhA-Pta H107Y IscR | pRSFhemBCD, pET-hemENGH and pCDF-hemA(C107A)L; KmR, ApR, SmR** |

| | |
|---|---|
| **E. coli* strain: B F⁻ *ompT gal dcm Ion hsdS_{B}*(*r_{B}⁻m_{B}⁻*) λ(DE3 [*lacI lacUV5-T7p07 indl sam7 nin5*]) [*malB*⁺]_{K-12}(*λ*^{S}) **Resistance genes for Kanamycin (KmR), ampicillin (ApR), streptomycin (SmR) in each respective plasmid. | |

The heme pathway genes, hemALBCDEGH and either hemN or hemF, cloned into 3 plasmid (see table 8A), were transformed into a derivative of an *E. coli* parent strain comprising either wild-type or H107Y mutant IscR protein. Additionally, strains were constructed wherein the hemA gene was mutated to express either of the heme feedback-insensitive hemA proteins : hemA(kk) comprising (L2K; L3K) substitutions or hemA comprising an (C107A) substitution.
Cells of each strain (Table 8A) are cultivated in biotin-supplemented mMOPS medium supplemented with 100 µg/mL ampicillin, 50 µg/mL spectinomycin and 50 µg/mL kanamycin at 37 °C, including IPTG-induction of the heme pathway genes, and the heme and prophyrin titer of the cultured cells is determined as described in Example 3.01. Strains carrying the hemF gene are cultured under aerobic conditions. Heme and prophyrin production by an *E. coli* strain over-expressing the genes of the heme pathway (i.e. hemALBCDEGH and hemN), and lacking yfeX-LdhA-Pta genes, is enhanced when the host strain expresses a gene encoding a mutant form of the IscR protein (IscR protein having an H107Y substitution) as compared to a strain expressing a gene encoding a native, wild-type IscR protein. This enhancement does not occur in strains expressing Hem F instead of HemN.

### 3.03 Hemeprotein production in an IscR H107Y mutant strain overexpressing 'hemN or hemF' in combination with hemALBCDEGH genes and a cytochrome P450 monooxygenase

The following strains of *Escherichia coli* used in the example are listed below.

**Table 8B plasmids**

| **Name** | **Description** |
|---|---|
| pBS_Heme1 | pRSFhemBCD |
| pBS_Heme2 | pET-hemENGH |
| pBS_Heme3 | and pCDF-hemA(C107A)L-BM3* |

The heme pathway genes, hemALBCDEGH and either hemN or hemF as well as BM3* mutant gene derived from *Bacillus megaterium,* cloned into 3 plasmids (see table 8B), were transformed into a derivative of an *E. coli* parent strain comprising either wild-type (ΔyfeX-LdhA-Pta) or H107Y mutant IscR protein(ΔyfeX-LdhA-Pta H107Y IscR), as shown in Table 8A.

Cells of each transformed strain are then cultivated in biotin-supplemented mMOPS medium supplemented with 100 µg/mL ampicillin, 50 µg/mL spectinomycin and 50 µg/mL kanamycin at 37 °C, including IPTG-induction of the heme pathway genes and BM3*. Indole and NADPH are added as a substrate and cofactor of the P450 for a biotransformation. Indigo production by an *E. coli* strain over-expressing the monooxygenase gene BM3* and the genes of the heme pathway (i.e. hemALBCDEGH and hemN), and lacking yfeX-LdhA-Pta genes, is enhanced when the host strain expresses a gene encoding a mutant form of the IscR protein (IscR protein having an H107Y substitution) as compared to a strain expressing a gene encoding a native, wild-type IscR protein.

### Example 4 Engineering and characterization of genetically modified E. coli strains capable of enhanced nicotinamide riboside production 4.01 Enhanced growth and quinolinate synthase activity in an IscR H107Y mutant strain overexpressing nadA genes.

The following strains of *Escherichia coli* used in the example are listed below.

**Table 9: Strains**

| **Name** | **Description** |
|---|---|
| BS1011 | ΔbioB (JW0758-1) derived from Escherichia coli K-12 BW25113 having genotype : rrnB3 ΔlacZ4787 hsdR514 Δ(araBAD)567 Δ(rhaBAD)568 rph-1 |
| BS1353 | BS1011 derivative comprising a H107Y mutation in iscR |
| BS2379 | BS1011 transformed with pBS1167 |
| BS2382 | BS1353 transformed with pBS1167 |
| BS2629 | BS1011 transformed with pBS1259 |
| BS2630 | BS1353 transformed with pBS1259 |
| BS_NR01 | BS1011 transformed with pBS_NR |
| BS_NR02 | BS1353 transformed with pBS_NR |
| BS_NAM01 | BS1011 transformed with pBS_NAM |
| BS_NAM02 | BS1353 transformed with pBS_NAM |
| BS_NA01 | BS1011 transformed with pBS_NA |
| BS_NA02 | BS1353 transformed with pBS_NA |

The following plasmids used in the example are listed below.

**Table 10: Plasmids**

| **Name** | **Description** |
|---|---|
| pBS1167 | Plasmid (AmpR, p15A) comprising a nadA gene [SEQ ID No.: 117] operably linked to a T5 LacO repressed promoter [SEQ ID No.: 37] and a rrnB terminator [SEQ ID No.: 94]. |
| pBS1259 | Control empty plasmid (AmpR, p15A) comprising a T5 LacO inducible promoter [SEQ ID No.: 37], an RBS, where AmpR is the sole open reading frame. |
| pBS1651 | Plasmid (AmpR, p15A) comprising nadAB genes[SEQ ID No.: 117 and 128] operably linked to a T5 LacO repressed promoter [SEQ ID No.: 37] and a rrnB terminator [SEQ ID No.: 94]. Constitutive promoter apFAB309 [SEQ ID No.: 93] and a rrnB terminator [SEQ ID No.: 94]. |
| pBS_NR | Plasmid (AmpR, p15A) comprising nadABCNadE*aphA genes [SEQ ID No.: 117, 128, 133, 129, and 130] operably linked to a T5 LacO repressed promoter [SEQ ID No.: 37] and a rrnB terminator [SEQ ID No.: 94]. Constitutive promoter [SEQ ID No.: 93] and a rrnB terminator [SEQ ID No.: 94]. |
| pBS_NAM | Plasmid (AmpR, p15A) comprising *nadA, nadB, nadC, nadE** and *chi* genes encoding [SEQ ID No.: 117, 133, 129, 131, 132] operably linked to a T5 LacO repressed promoter [SEQ ID No.: 3] and a rrnB terminator [SEQ ID No.: 4]. Constitutive promoter [SEQ ID No.: 93] and a rrnB terminator [SEQ ID No.: 94]. |
| pBS_NA | Plasmid (AmpR, p15A) comprising *nadB, nadC, nadE*, chi* and pncA genes encoding [SEQ ID No.: 133, 129, 132, 131] operably linked to a T5 LacO repressed promoter [SEQ ID No.: 37] and a rrnB terminator [SEQ ID No.: 94]. Constitutive promoter [SEQ ID No.: 93] and a rrnB terminator [SEQ ID No.: 94]. |

IPTG-inducible genes encoding NadA (quinolate synthase) was cloned to give plasmid pBS1167; and an empty plasmid pBS1259 having a p15A backbone was used as a control. pBS1167 or pBS1259 were introduced into the *E. coli* host strain (BS1353) in which the native iscR gene was substituted by a mutant iscR gene encoding an IscR protein having an H107Y substitution (as described in Example 1) resulting in the strains BS2382 and BS2630, respectively. Similarly, the plasmids pBS1167 and pBS1259 were introduced into *E. coli* strain BS1011 carrying a wild-type version of the iscR gene, resulting in strains BS2379 and BS2629, respectively.

Cells of each strain (Table 9) are cultivated 24 h at 37 °C in parallel cultures comprising mMOPS medium (as described in Example 1.03) supplemented with 1 nmol/L biotin, 100 µg/mL ampicillin and with 0 to 1mM IPTG in deep culture plates. Cell growth was monitored over 24 h by measuring cell density at OD₆₂₀nm (Figure 14). The lag-phase duration and growth rate of strains BS2629 (IscR WT) and BS2630 (IscR mutant), comprising control plasmids was similar under both non-inducing and inducing conditions. However, IPTG induction of nadA transgene expression resulted in an extended lag-phase and slower growth rate for IscR WT strain BS2379, indicating that NadA-overexpression is toxic for growth. This growth deficiency is largely overcome when the nadA gene is overexpressed in the IscR mutant strain BS2382. This demonstrates that the IscR mutant strain provides sufficient Fe-S clusters to meet the functional requirements of both over-expressed NadA and other native Fe-S proteins in the cell and to thereby support cell growth.

The catalytic activity of NadA expressed in IscR wild type (BS1011) and mutant (BS1353) *E. coli* strains transformed with pBS1651 or pBS1259 is determined by measuring quinolate, an intermediate in the NR pathway. Cells of each strain are grown in 50 ml mMOPS medium, with or without aspartic acid, supplemented with with 1 nmol/L biotin, 100 µg/mL ampicillin, at 37 °C and 250 rpm until the OD₆₀₀ₙₘ reaches 0.6. IPTG is then added to each culture to a final concentration of 0.064 mM, that are then incubated for a further 6 h. Every hour, a 1 mL aliquot, sampled from each culture, is normalized to the same cell density (OD₆₀₀nm) and then pelleted with centrifugation at 17000 g for 5 min. Pellets, after washing in ice-cold PBS (phosphate buffer saline) solution, are re-suspended, and then lysed by sonication and finally centrifuged at 17000 g for 5 min. Quinolate, present in the recovered lysed cell supernatant, is measured by LC-MS using a 1290 Infinity series UHPLC coupled to a 6470 triple quadrupole from Agilent Technologies (Santa Clara, USA) as described by Ollagnier-de Choudenset al., (2005).

### 4.02 Enhanced nicotinamide riboside (NR) production in an IscR H107Y mutant strain overexpressing nadABC, nadE* and aphA genes

The parent E. coli strain BS1011, and its mutant derivative expressing IscR H107Y, are transformed with a plasmid (pBS_NR) comprising the genes nadABCE*aphA operatively linked an IPTG inducible promoter, or with a control empty plasmid. The genes expressed in plasmid pBS_NAM include: E. coli nadA gene encodes quinolate synthase (NadA); the E. coli nadB encodes L-aspartate oxidase (NadB); nadC encodes Nicotinate-nucleotide pyrophosphorylase (NadC); aphA encodes a Class B acid phosphatase (AphA); and the Mannheimia succiniciproducens nadE gene encodes a polypeptide with nicotinic acid mononucleotide amidating activity (NadE*). Cells of each strain are grown in 50 ml mMOPS medium, supplemented with with 1 nmol/L biotin, 100 µg/mL ampicillin, at 37 °C and 250 rpm until the OD600nm reaches 0.6. IPTG is then added to each culture to a final concentration of 0.064 mM that are then incubated for a further 6 h, and subsequently lysed.

NR, present in the recovered lysed cell supernatant, is measured by LC-MS using a 1290 Infinity series UHPLC coupled to a 6470 triple quadrupole from Agilent Technologies (Santa Clara, USA) (Ollagnier-de Choudenset al., 2005). NR production by an E. coli strain expressing the genes of the NR pathway (i.e. nadABCE*aphA), is enhanced when the host strain expresses a gene encoding a mutant form of the IscR protein (IscR protein having an H107Y substitution) as compared to a strain expressing a gene encoding a native, wild-type IscR protein.

Cells of each strain are grown in 50 ml mMOPS medium, supplemented with with 1 nmol/L biotin, 100 µg/mL ampicillin, at 37 °C and 250 rpm until the OD600nm reaches 0.6. IPTG is then added to each culture to a final concentration of 0.064 mM that are then incubated for a further 6 h, and subsequently lysed.

NR, present in the recovered lysed cell supernatant, is measured by LC-MS using a 1290 Infinity series UHPLC coupled to a 6470 triple quadrupole from Agilent Technologies (Santa Clara, USA) (Ollagnier-de Choudenset al., 2005). NR production by an E. coli strain expressing the genes of the NR pathway (i.e. nadABCE*aphA), is enhanced when the host strain expresses a gene encoding a mutant form of the IscR protein (IscR protein having an H107Y substitution) as compared to a strain expressing a gene encoding a native, wild-type IscR protein.

### 4.03 Enhanced nicotinamide (NAM) production in an IscR H107Y mutant strains overexpressing nadABC, nadE* and NMN nucleosidase (chi) genes

For nicotinamide NAM production E. coli strain BS1011, and its mutant derivative expressing IscR H107Y, are transformed with a plasmid (pBS_NAM) comprising the genes nadABCE* and chi operatively linked an IPTG inducible promoter, or with a control empty plasmid. The genes expressed in plasmid pBS_NAM include: E. coli nadA gene encodes quinolate synthase (NadA); the E. coli nadB encodes L-aspartate oxidase (NadB); nadC encodes Nicotinate-nucleotide pyrophosphorylase (NadC); chi encodes NMN nucleosidase (chi) and the Mannheimia succiniciproducens nadE* gene encodes a polypeptide with nicotinic acid mononucleotide amidating activity.

### Example 5 Engineering and characterization of genetically modified E. coli strains capable of enhanced production cobalamin

### 5.01 Enhanced precorrin-3B synthase activity in an IscR H107Y mutant strain overexpressing a cobG gene.

The following strains of *Escherichia coli* used in the example are listed below.

**Table 11: Strains**

| **Name** | **Description** |
|---|---|
| BS1011 | ΔbioB (JW0758-1) derived from Escherichia coli K-12 BW25113 having genotype : rrnB3 ΔlacZ4787 hsdR514 Δ(araBAD)567 Δ(rhaBAD)568 rph-1 |
| BS1011, IdhA :: cbiNQOM | BS1011 derivative comprising an operon comprising cbiNQOM genes inserted into the genome |
| BS1353 | BS1011 derivative comprising a H107Y mutation in iscR |
| BS1353 IdhA :: cbiNQOM | BS1353 derivative comprising an operon comprising cbiNQOM genes inserted into the genome |
| BS2629 | BS1011 transformed with pBS1259 |
| BS2630 | BS1353 transformed with pBS1259 |
| BS4137 | BS1011 transformed with pBS1288 and pBS1637 |
| BS4138 | BS1353 transformed with pBS1288 and pBS1637 |
| BS4139 | BS1353 transformed with pBS1288 |
| BS4140 | BS1011 transformed with pBS1288 |
| BS4141 | BS1011, IdhA :: cbiNQOM transformed with pBS1738, pBS1749, pBS1750 |
| BS4142 | BS1353 IdhA :: cbiNQOM transformed with pBS1738, pBS1749, pBS1750 |

The following plasmids used in the example are listed below.

**Table 12: Plasmids**

| **Name** | **Description** |
|---|---|
| pBS1288 | Plasmid (AmpR, p15A) comprising CobG [SEQ ID No.: 135] operably linked to a T5 LacO repressed promoter [SEQ ID No.: 37] and a rrnB terminator [SEQ ID No.: 94]. |
| pBS1259 | Control empty plasmid (AmpR, p15A) comprising a T5 LacO repressed promoter [SEQ ID No.: 2] and a rrnB terminator [SEQ ID No.: 94] but no additional open reading frame. |
| pBS1637 | Plasmid (pBR322; KanR) comprising the operon of genes CobIMF encoding [SEQ ID No.: 146, 147, 148] operably linked to a constitutive promoter apFAB309 [SEQ ID No.:93] and a FAB terminator [SEQ ID No.: 27]; second operon of genes CobKHLJ encoding [SEQ ID No.: 149, 150, 151, 152] from a constitutive promoter apFAB309 [SEQ ID No.: 93] and a FAB terminator [SEQ ID No.: 27]. |
| pBS1748 | Plasmid (AmpR, p15A) comprising CobG [SEQ ID No.: 135] operably linked to a T5 LacO repressed promoter [SEQ ID No.: 37] and a rrnB terminator [SEQ ID No.: 94] and an operon of genes CobHIJLFK encoding [SEQ ID No.: 150, 146, 152, 151, 148, 149] from a constitutive promoter apFAB309 [SEQ ID No.: 93] and a FAB terminator [SEQ ID No.: 27]. |
| pBS1749 | Plasmid (pBR322; KanR) comprising the operon of genes CobMNST encoding [SEQ ID No.: 147, 153-155] operably linked to a constitutive promoter apFAB309 [SEQ ID No.:93] and a FAB terminator [SEQ ID No.: 27]; second operon of genes CobROQBtuR [SEQ ID No.:156, 157, 158, 159,] from a constitutive promoter apFAB309 [SEQ ID No.: 93] and a FAB terminator [SEQ ID No.: 27]. |
| pBS1750 | Plasmid (pSC101, SpecR) comprising the operon of genes CobCDTPduX [SEQ ID No.: 162, 161, 163, 166] operably linked to a constitutive promoter apFAB309 [SEQ ID No.:93] and a FAB terminator [SEQ ID No.: 27]; second operon of genes CobUSCbiB [SEQ ID No.: 160, 164, 165] from a constitutive promoter apFAB309 [SEQ ID No.: 93] and a FAB terminator [SEQ ID No.: 27]. |

IPTG-inducible gene encoding CobG (precorrin-3B synthase) was cloned into plasmid pBS1288; and an empty plasmid pBS1259 having a p15A backbone was used as a control. A first operon encoding CobIMF and a second operon encoding CobKHLJ was cloned on a further plasmid pBS1637. The plasmid pBS1288 (alone) or in combination with pBS1637; or pBS1259 alone were introduced into the *E. coli* host strain (BS1353) in which the native iscR gene was substituted by a mutant iscR gene encoding an IscR protein having an H107Y substitution (as described in Example 1) resulting in the strains BS4139, BS4138 and BS2630, respectively. Additionally, pBS1288 (alone), or in combination with pBS1637, or pBS1259 alone were introduced into *E. coli* strain BS1011 carrying a wild-type version of the iscR gene, resulting in strains BS4140, B43137 and BS2629, respectively.

Cells of each strain (Table 11) are cultivated aerobically at 37 °C in parallel cultures comprising mMOPS medium (as described in Example 1.03) supplemented with 1 nmol/L biotin, 100 µg/mL ampicillin (and additionally 50µg/ml kanamycin for strains BS4137 and BS4138 or 50 µg/mL spectomycin for BS4141 and BS4142). When each culture reaches a cell density of 0.8-1.0 OD₆ₒ₀ₙₘ, the culture medium is supplemented with 10 mM aminolaevulinic acid and 0.1 mM IPTG to induce cobG transgene expression, and incubated for at 28 °C for 24-48 h.

The catalytic activity of CobG transgenically expressed in the mutant *E. coli* strain (BS4139) as compared its expression in the IscR wild type (BS4140) is shown to be enhanced when determined by measuring hydrogenobyrinic acid (HBA) production, an intermediate in the Cob pathway, as follows. The cultured cells, harvested by centrifugation (17000 g, 5 min), are resuspended in IEX-Buffer A (20 mM Tris-HCl, pH 7.4, 100 mM NaCl); sonicated, centrifuged (17000 g 5 min), and the supernatant is then used for detection of HBA via via LC-MS using a 1290 Infinity series UHPLC coupled to a 6470 triple quadrupole from Agilent Technologies (Santa Clara, USA).

The production of the intermediate, HBA, is further enhanced in the IscR mutant strain, BS4138, where the transgene encoding CobG and the transgenes encoding CobIMF and CobKHLJ are co-expressed in the cells, as compared to the parent host *E. coli* strain expressing wild type IscR (BS4137).

### 5.02 Enhanced cobalamin production in an IscR H107Y mutant strain overexpressing the cobalamin pathway genes

Cobalamin is produced by *E. coli* cells expressing an IPTG inducible transgene encoding CobG and a constitutively expressing transgenes encoding transgenes encoding CobHIJLFK, CobMNST, CobCDTPduX, CobROQBtuR and CobUSCbiB; and where the host *E. coli* cells further comprise the transgenes cbiNQOM inserted into their genome. *E. coli* strains BS4141 and BS4142 are cultured as described in 5.01 above. Cobalamin produced by the cultures is measured as follows: 2.5 mL of NaNO₂ 8% (w/v) and 2.5 mL of glacial acetic acid are added to 25 mL samples of each culture; which are then boiled for 30 min, and the resulting mixture filtered. Then 20 µL NaCN 10% (w/v) is added to 1 mL of aqueous phase; and 20 µL of resulting upper aqueous phase is injected into an HP1100 HPLC system (Agilent). NH2 column (4.6 x 250 mm², 5 um) is employed for HPLC analysis with a flow rate of 1.7 mL/min and a wavelength of 360nm, using a mobile phase of 250 mM phosphoric acid/acetonitrile (30/70, v/v).

The production of cobalamin is enhanced when said host *E. coli* cells comprise the mutant iscR gene (BS4142) as compared to host *E. coli* cells comprising the WT iscR gene (BS4141).

### Example 6 Engineering and characterization of genetically modified E. coli strains capable of enhanced production of 3-methyl-2-oxobutanoate and 3-methyl-2-oxopentanoate and their derivatives

### 6.01 Enhanced growth and dihydroxy-acid dehydratase activity in an IscR H107Y mutant strain overexpressing ilvD gene.

The following strains of *Escherichia coli* used in the example are listed below.

**Table 13: Strains**

| **Name** | **Description** |
|---|---|
| BS1011 | ΔbioB (JW0758-1) derived from Escherichia coli K-12 BW25113 having genotype : rrnB3 ΔlacZ4787 hsdR514 Δ(araBAD)567 Δ(rhaBAD)568 rph-1 |
| BS1353 | BS1011 derivative comprising a H107Y mutation in iscR |
| BS2378 | BS1011 transformed with pBS1140 |
| BS2381 | BS1353 transformed with pBS1140 |
| BS2629 | BS1011 transformed with pBS1259 |
| BS2630 | BS1353 transformed with pBS1259 |
| BS2631 | BS1011 transformed with pBS1140 and pBS1652 |
| BS2632 | BS1353 transformed with pBS1140 and pBS1652 |
| BS3313 | BS1011 (Δ*aceF*Δ*mdh ΔpfkA*) |
| BS3314 | BS1353 (Δ*aceF*Δ*mdh ΔpfkA*) |
| BS3315 | BS3313 with pBS1768 and pBS1767 |
| BS3316 | BS3314 (Δ*aceF*Δ*mdh ΔpfkA*) with pBS1768 and pBS1767 |

The following plasmids used in the example are listed below.

**Table 14: Plasmids**

| **Name** | **Description** |
|---|---|
| pBS1140 | Plasmid (AmpR, p15A) comprising an ilvD gene [SEQ ID No.: 15] operably linked to a T5 LacO repressed promoter [SEQ ID No.: 37] and a rrnB terminator [SEQ ID No.: 94]. |
| pBS1259 | Control empty plasmid (AmpR, p15A) comprising a T5 LacO inducible promoter [SEQ ID No.: 39], a RBS [SEQ ID No.: 4], where AmpR is the sole open reading frame. |
| pBS1652 | Plasmid (KanR, pBR322) comprising IlvCBN encoding [SEQ ID No.:185, 183, 184] operably linked to the constitutive promoter [SEQ ID No.: 14] and a rrnB terminator [SEQ ID No.: 94]. |
| pBS1767 | Plasmid (p15A; AmpR) comprising an ilvD gene [SEQ ID No.: 15] operably linked to a T5 LacO repressed promoter [SEQ ID No.: 3] and a rrnBterminator [SEQ ID No.: 94]. Second operon of genes IlvBNbisCE [SEQ ID No.:183, 187, 185, 186] from a constitutive promoter apFAB309 [SEQ ID No.: 93] and a FAB terminator [SEQ ID No.: 27]. |
| pBS1768 | Plasmid (pSC101; SpecR)comprising the genes ygaZHIrp [SEQ ID No.:188, 189, 190] operably linked to a constitutive promoter apFAB309 [SEQ ID No.:93] and a FAB terminator [SEQ ID No.: 27] |

IPTG-inducible gene encoding IlvD (dihydroxy-acid dehydratase) was cloned to give plasmid pBS1140; and an empty plasmid pBS1259 having a p15A backbone was used as a control. pBS1140 or pBS1259 were introduced into the *E. coli* host strain (BS1353) in which the native iscR gene was substituted by a mutant iscR gene encoding an IscR protein having an H107Y substitution (as described in Example 1) resulting in the strains BS2381 and BS2630, respectively. Similarly, the plasmids pBS1140 or pBS1259 were introduced into *E. coli* strain BS1011 carrying a wild-type version of the iscR gene, resulting in strains BS2378 and BS2629, respectively.

Cells of each strain (Table 13) are cultivated 24 h at 37 °C in parallel cultures comprising mMOPS medium (as described in Example 1.03) supplemented with 1 nmol/L biotin, 100 µg/mL ampicillin and with 0 to 1mM IPTG in deep culture plates. Cell growth was monitored over 24 h by measuring cell density at OD₆₂₀ (Figure 17). The lag-phase duration and growth rate of strains BS2629 (IscR WT) and BS2630 (IscR mutant), comprising control plasmids was similar under both non-inducing and inducing conditions. However, IPTG induction of ilvD transgene expression resulted in an extended lag-phase and slower growth rate for IscR WT strain BS2378, indicating that IlvD-overexpression is toxic for the cells. This growth deficiency is largely overcome when the ilvD gene is overexpressed in the IscR mutant strain BS2381. This demonstrates that the IscR mutant strain provides sufficient Fe-S clusters to meet the functional requirements of both over-expressed IlvD protein and other native Fe-S proteins in the cell and to thereby support cell growth.

The catalytic activity of IlvD expressed in IscR wild type (BS1011) and mutant (BS1353) *E. coli* strains transformed with pBS1140 or pBS1259 is determined by measuring 3-methyl-2-oxobutanoate, produced from 2,3-dihydroxy-3-methylbutanoate by dihydroxy-acid dehydratase. Cells of each strain are grown as described above; the cultures are normalized for cell density, and then subsequently pelleted at 17,000 g, where the supernatant is passed through a cation separation column and the eluant is then subsequently analysed for 3-methyl-2-oxobutanoate, amino acid and pantothenic acid content by LC-MS using a 1290 Infinity series UHPLC coupled to a 6470 triple quadrupole from Agilent Technologies (Santa Clara, USA) as described by Ollagnier-de Choudenset al., (2005).

### 6.02 Enhanced valine production in an IscR H107Y mutant strain overexpressing ilvD, ilvC, ilvB and ilvN genes

The parent *E. coli* strain BS1011, and its mutant derivative expressing IscR H107Y BS1353, are transformed with a plasmid (pBS1652) comprising the genes ilvC, ilvB and ilvN operably linked a constitutive promoter, and pBS1140 comprising the ilvD gene, or only the control empty plasmid pBS1259. The *E. coli* ilvC gene in plasmid pBS1652 encodes ketol-acid reductoisomerase (NADP⁺); *E. coli* ilvB encodes acetolactate synthase isozyme 1 large subunit; and *E. coli* ilvN encodes acetolactate synthase isozyme 1 small subunit.

Cells of each strain are cultivated aerobically in 50 mLof NM1 Medium (NM1 composition: glucose, 20 g; (NH4)2SO4, 20g; KH2PO4, 2.0 g; MgSO4, 7H2O, 0.4 g; NaCl, 1.6 g; yeast extract, 2 g; trace metal solution, supplemented with 1 nmol/L biotin, 100 µg/mL ampicillin, 50µg/mL kanamycin, at 31 °C and 250 rpm until reaching a cell density of OD₆₀₀ reaches 0.4. IPTG is then added to the medium at a concentration of 0,028 mM and cells are further incubated for 24 h. 3-methyl-2-oxobutanoate produced in the culture medium by each strain, following normalization for cell density, is measured as described above.

The production of 3-methyl-2-oxobutanoate is enhanced in the IscR mutant strain, BS2632, when co-expressing the transgene encoding ilvD and the transgenes encoding ilvCBN, as compared to their co-expression in the parent host *E. coli* strain expressing wild type IscR (BS2631).

### 6.03 Enhanced L-valine production in an IscR H107Y mutant strain overexpressing ilvD, ilvE, ilvC, ilvB and ilvNbis as well as ygaZH and IrP.

The parent E. coli strain BS1011, and its mutant derivative expressing IscR H107Y BS1353, are first genomically engineered to yield the strains BS3313 and BS3314. BS3313 and BS3314 are transformed with a plasmid (pBS1767) comprising the genes ilvD operatively linked to a T5 LacO promoter, and ilvE, ilvC, ilvB and ilvNbis operatively linked a constitutive promoter, and pBS1768 comprising the ygaZH and IrP gene. The *E. coli* ilvC gene in plasmid pBS1652 encodes ketol-acid reductoisomerase (NADP⁺); *E. coli* ilvB encodes acetolactate synthase isozyme 1 large subunit; *E. coli* ilvNbis encodes a mutated acetolactate synthase isozyme 1 small subunit. *E. coli* ilvE encodes a branched-chain-amino-acid aminotransferase; *E. coli* ygaH encodes a valine transporter; *E. coli* ygaz encodes a inner membrane protein; and *E. coli* IrP encodes a leucine-responsive regulatory protein.

Cells of each strain are cultivated aerobically in 50 mL of NM1 Medium (NM1 composition: glucose, 20 g; (NH4)2SO4, 20g; KH2PO4, 2.0 g; MgSO4, 7H2O, 0.4 g; NaCl, 1.6 g; yeast extract, 2 g; trace metal, solution, supplemented with 1 nmol/L biotin, 100 µg/mL ampicillin, 50µg/ml kanamycin, at 31 °C and 250 rpm until reaching a cell density of OD600ₙₘ reaches 0.4. IPTG is then added to the medium at a concentration of 0,028 mM and cells are further incubated for 24 h. L-valine produced in the culture medium by each strain, following normalization for cell density, is measured as described in 6.01. L-valine production is enhanced in the IscR mutant strain, BS3316, when co-expressing the transgene encoding ilvD and the transgenes encoding ilvE, ilvC, ilvB and ilvNbis as well as ygaZH and IrP as compared to expression in a host *E. coli* strain expressing wild type IscR (BS3315).

### Example 7 Engineering and characterization of genetically modified E. coli strains capable of enhanced production of isoprenoids

### 7.01 Enhanced isoprenoid precursor synthesis activity in an IscR H107Y mutant strain overexpressing ispG and ispH genes.

The following strains of *Escherichia coli* used in the example are listed below.

**Table 15: Strains**

| **Name** | **Description** |
|---|---|
| BS3317 | BS1011 (ΔbioB ΔytjC; genRBS dxs, rpos, idi, dxr) |
| BS3318 | BS3317 derivative comprising a H107Y mutation in iscR |
| BS3140 | BS3317 transformed with pBS1139 |
| BS3141 | BS3318 transformed with pBS1139 |
| BS3142 | BS3317 transformed with pBS1259 |
| BS3143 | BS3318 transformed with pBS1259 |

The following plasmids used in the example are listed below.

**Table 16: Plasmids**

| **Name** | **Description** |
|---|---|
| pBS1139 | Plasmid (AmpR, p15A) comprising an ispG gene [SEQ ID No.: 191] and an ispH gene [SEQ ID No.: 202] operably linked to a T5 LacO repressed promoter [SEQ ID No.: 37] and a rrnB terminator [SEQ ID No.: 94]. |
| pBS1259 | Control empty plasmid (AmpR, p15A) comprising a T5 LacO inducible promoter [SEQ ID No.: 39], a RBS, where AmpR is the sole open reading frame. |

IPTG-inducible genes encoding IspG (4-hydroxy-3-methylbut-2-en-1-yl diphosphate synthase) and IspH (4-hydroxy-3-methylbut-2-enyl diphosphate reductase) were cloned to give plasmid pBS1139; and an empty plasmid pBS1259 having a p15A backbone was used as a control. pBS1139 or pBS1259 were introduced into the *E. coli* host strain (BS3318) in which the native iscR gene was substituted by a mutant iscR gene encoding an IscR protein having an H107Y substitution (as described in Example 1) resulting in the strains BS3141 and BS3143, respectively. Similarly, the plasmids pBS1139 or pBS1259 were introduced into *E. coli* strain BS3317 carrying a wild-type version of the iscR gene, resulting in strains BS3140 and BS3142, respectively. The *E. coli* host strains BS3317 and BS3318 (comprising the native iscR and mutant iscR genes respectively) are genetically modified to upregulate expression of the genes dxs, rpoS, idi and dxr; and to delete the gene ytjC. The native ribosomal binding site (RBS), upstream of these genes, is modification being the substituted with an optimized RBS sequence [dxsRBS, SEQ ID NO: 280]; [rpoSRBS, SEQ ID NO:282]; [idiRBS, SEQ ID NO: 284]; [dyrRBS, SEQ ID NO: 286]. Cells of each strain (Table 15) are cultivated 24 h at 30 °C in parallel cultures comprising LB medium supplemented with 100 µg/mL ampicillin at 250 rpm until reaching a cell density of OD600ₙₘ of 0.6. IPTG is then added to the medium at a concentration of 0.1 mM and cells are further incubated for 24 h. Next, a 6 mL cell suspension aliquot having an OD600ₙₘ of 1.0 is centrifuged for 5 min (17000xg), and re-suspended in 10 mL acetonitrile/methanol/water 40:40:20 plus 0.1 M formic acid intracellular metabolite extraction. The sample is incubated at -20°C for 60 min with periodic shaking. Following centrifugation as before, the supernatant is purified through a LC-NH2 resin and analyzed for the isoprenoid precursors, IPP and DMAPP, using a 1290 Infinity series UHPLC coupled to a 6470 triple quadrupole from Agilent Technologies (Santa Clara, USA), as described by Zhou K et al., 2012.

The catalytic activity of IspG and IspH expressed in IscR mutant (BS3140) *E*. *coli* strains transformed with pBS1139, as compared to Isc WT (BS3139) *E*. *coli* strain transformed with pBS1139 or pBS1259 is increased based on the increase in detected levels of the precursors IPP and DMAPP produced.

### Example 8 Engineering and characterization of genetically modified E. coli strains capable of enhanced production of L-glutamic acid

### 8.01 Enhanced L-glutamic acid synthesis activity in an IscR H107Y mutant strain overexpressing gltB and gltD genes.

The following strains of *Escherichia coli* used in the example are listed below.

**Table 17: Strains**

| **Name** | **Description** |
|---|---|
| BS1011 | ΔbioB (JW0758-1) derived from Escherichia coli K-12 BW25113 having genotype : rrnB3 ΔlacZ4787 hsdR514 Δ(araBAD)567 Δ(rhaBAD)568 rph-1 |
| BS1353 | BS1011 derivative comprising a H107Y mutation in iscR |
| BS_glt01 | BS1011 derivative comprising a pBS_gltBD giving IPTG inducible gltB and gltD expression |
| BS_glt02 | BS1353 derivative comprising a pBS_gltBD giving IPTG inducible gltB and gltD expression |
| BS_glt03 | BS1011 derivative comprising a pBS_gltB giving IPTG inducible gltB and gltD expression |
| BS_glt04 | BS1353 derivative comprising a pBS_gltB giving IPTG inducible gltB expression |
| BS_glt05 | BS1011 derivative comprising a pBS_gltD giving IPTG inducible gltD expression |
| BS_glt06 | BS1353 derivative comprising a pBS_gltD giving IPTG inducible gltD expression |
| BS2629 | BS1011 derivative comprising pBS1259 as an empty vector control |
| BS2630 | BS1353 derivative comprising pBS1259 as an empty vector control |
| BS3326 | BS1011 derivative comprising pBS1769 |
| BS3327 | BS1353 derivative comprising pBS1769 |

The following plasmids used in the example are listed below.

**Table 18: Plasmids**

| **Name** | **Description** |
|---|---|
| pBS_gltBD | Plasmid (AmpR, p15A) comprising genes GltB [SEQ ID No.: 219] and GltD [SEQ ID No.: 231] operably linked to a T5 LacO repressed promoter [SEQ ID No.: 39] and a rrnB terminator [SEQ ID No.: 94]. |
| pBS1259 | Control empty plasmid (AmpR, p15A) comprising a T5 LacO inducible promoter [SEQ ID No.: 39], a RBS [SEQ ID No.: 4], where AmpR is the sole open reading frame. |
| pBS_gltB | Plasmid (AmpR, p15A) comprising gene GltB [SEQ ID No.: 219] operably linked to a T5 LacO repressed promoter [SEQ ID No.: 37] and a rrnB terminator [SEQ ID No.: 94]. |
| pBS_gltD | Plasmid (AmpR, p15A) comprising gene GltD [SEQ ID No.: 231] operably linked to a T5 LacO repressed promoter [SEQ ID No.: 37] and a rrnB terminator [SEQ ID No.: 94]. |
| pBS1769 | Plasmid (AmpR, p15A) comprising genes GltB [SEQ ID No.: 219] and GltD [SEQ ID No.: 231] operably linked to a T5 LacO repressed promoter [SEQ ID No.: 37] and a rrnB terminator [SEQ ID No.: 94]. Second open reading frame comprises genes gltXHemAL encoding [SEQ ID No: 240, 107, 108] operably linked to a constitutive promoter apFAB309 [SEQ ID No.:93] and a FAB terminator [SEQ ID No.: 27]. |

IPTG-inducible genes encoding GltB (Glutamate synthase [NADPH] large chain) and GltD (Glutamate synthase [NADPH] small chain) were individually cloned to give plasmids pBS_gltB and pBS_gID respectively, as well as the two genes being cloned together to give plasmid pBS_gltBD (encoding both polypeptides of GOGAT); and an empty plasmid pBS1259 having a p15A backbone was used as a control. Each of the 3 plasmids (pBS_gltB; pBS_gID; pBS_gltBD and pBS1259) were individually introduced into the *E. coli* host strain (BS3149) in which the native iscR gene was substituted by a mutant iscR gene encoding an IscR protein having an H107Y substitution (as described in Example 1) resulting in the strains pBS_glt04, pBS_glt06, pBS_glt02 and BS2630, respectively. Similarly, these plasmids were introduced into *E. coli* strain BS1353 carrying a wild-type version of the iscR gene, resulting in strains BS_glt03, BS_glt05, BS_glt01, BS2629 respectively.

Overnight 500uL seed cultures of each strain (Table 17) are cultivated in parallel at 37 °C in 50mL LB medium supplemented with 100 µg/mL ampicillin and 5g/l of NH4(SO4) as nitrogen source at 200 rpm until the cell density reaches an OD600ₙₘ of 0.8 - 1.0. IPTG is then added to the medium at a concentration of 0.1 mM of each culture, together with glutamine at a range of concentrations; and the cell cultures are further incubated at 28 °C for 24-48 h. Cells from each culture are then harvested by centrifugation (17000 g, 5 min). The supernatant is collected and used for detection of extracellular glutamate by colorimetric assay (#K629-100, Biovision, Milpitas, CA, USA). Additionally, L-Glutamate is quantified via LC-MS using a 1290 Infinity series UHPLC coupled to a 6470 triple quadrupole from Agilent Technologies (Santa Clara, USA).

The catalytic activity of GOGAT, expressed in the IscR mutant *E. coli* strain transformed with pBS_gltBD (strain BS_glt02), as compared GOGAT expressed in Isc WT *E. coli* strain transformed with pBS_gltBD (BS_glt01), or either *E. coli* strain transformed with the control empty plasmid or plasmids pBS_gltB or pBS_gltD is increased, based on an increase in detected levels of L-glutamine produced.

### 8.02 Enhanced Aminolevulonic acid synthesis activity in an IscR H107Y mutant strain overexpressing gltB and gltD genes alongside gltx, hemA and hemL genes.

IPTG-inducible genes encoding GltDB (Glutamate synthase) and constitutively expressed GltX (Glutamate-tRNA ligase), HemA (Glutamyl-tRNA reductase), HemL (Glutamate-1-semialdehyde 2,1-aminomutase) were cloned to give the plasmid pBS1769. pBS1769 was introduced into the *E. coli* host strain (BS1353) in which the native iscR gene was substituted by a mutant iscR gene encoding an IscR protein having an H107Y substitution (as described in Example 1) resulting in the strain BS3327. Similarly, pBS1769 was introduced into *E. coli* strain BS1011 carrying a wild-type version of the iscR gene, resulting in strain BS3326.

Overnight 500 µL seed cultures of each strain (Table 17) are cultivated in parallel at 37 °C in 50mL LB medium supplemented with 100 µg/mL ampicillin and 5g/l of NH4(SO4) as nitrogen source at 200 rpm until the cell density reaches an OD600ₙₘ of 0.8 - 1.0. IPTG is then added to the medium at a concentration of 0.1 mM of each culture, together with glutamine at a range of concentrations; and the cell cultures are further incubated at 28 °C for 24-48 h. Cells from each culture are then harvested by centrifugation (17000 g, 5 min). The supernatant is collected and used for detection of extracellular aminolaevulinic acid (ALA) using an Ehrlich reagent. ALA is then quantified by spectrometric analysis at 550 nm.

The catalytic activity of GltDB, expressed in the IscR mutant *E. coli* strain transformed with pBS1769, is increased as compared GltDB expressed in IscR WT *E. coli* strain transformed with pBS1769.

### Example 9 Engineering and characterization of genetically modified E. coli strains capable of enhanced pyrroloquinoline quinone production

### 9.01 Enhanced PqqA peptide cyclase activity activity in an IscR H107Y mutant strain overexpressing a pqqA gene.

The following strains of *Escherichia coli* used in the example are listed below.

**Table 19: Strains**

| **Name** | **Description** |
|---|---|
| BS1011 | ΔbioB (JW0758-1) derived from Escherichia coli K-12 BW25113 having genotype : rrnB3 ΔlacZ4787 hsdR514 Δ(araBAD)567 Δ(rhaBAD)568 rph-1 |
| BS1353 | BS1011 derivative comprising a H107Y mutation in iscR |
| BS2629 | BS1011 transformed with pBS1259 |
| BS2630 | BS1353 transformed with pBS1259 |
| BS_PQQ1 | BS1011 transformed with pBS_pqq giving IPTG inducible PqqABCDEF expression |
| BS_PQQ2 | BS1353 transformed with pBS_pqq giving IPTG inducible PqqABCDEF expression |

The following plasmids used in the example are listed below.

**Table 20: Plasmids**

| **Name** | **Description** |
|---|---|
| pBS_PQQ | Plasmid (AmpR, p15A) comprising genes encoding PqqA [SEQ ID No.: 247], PqqB [SEQ ID No.: 248], PqqC [SEQ ID No.: 249], PqqD [SEQ ID No.: 250], PqqE [SEQ ID No.: 242] and PqqF [SEQ ID No.: 251] operably linked to a T5 LacO repressed promoter [SEQ ID No.: 37] and a rrnB terminator [SEQ ID No.: 94]. |
| pBS1259 | Control empty plasmid (AmpR, p15A) comprising a T5 LacO repressed promoter [SEQ ID No.: 37] and a rrnB terminator [SEQ ID No.: 94] but no additional open reading frame. |

IPTG-inducible operon comprising genes pqqA, pqqB, pqqc, pqqD, pqqE and pqqF genes derived from the pQQABCDEF operon of *Klebsiella pneumoniae* (ATCC 19606) encoding PqqABCDEF was cloned to create plasmid pBS_PQQ; and an empty plasmid pBS1259 having a p15A backbone was used as a control. The plasmid pBS_PQQ or the control pBS1259 were introduced into the *E. coli* host strain (BS1353) in which the native iscR gene was substituted by a mutant iscR gene encoding an IscR protein having an H107Y substitution (as described in Example 1) resulting in the strain BS_PQQ2 and BS2630, respectively. Additionally, the plasmids pBS_PQQ and pBS1259 were introduced into *E. coli* strain BS1011 carrying a wild-type version of the iscR gene, resulting in strains BS_PQQ1 and BS2629, respectively.

Overnight 500uL seed cultures of each strain (Table 19) are cultivated in parallel at 37 °C in 50mL LB medium supplemented with 1.0 nM biotin, 100 µg/mL ampicillin at 200 rpm until the cell density reaches an OD600nm of 0.8 - 1.0. IPTG is then added to the medium; and the cell cultures are further incubated at 28 °C for 24-48 h. Cells from each culture are then harvested by centrifugation (17000 g, 5 min). The supernatant is collected, and used for detection of extracellular PQQ is quantified via LC-MS using a 1290 Infinity series UHPLC coupled to a 6470 triple quadrupole from Agilent Technologies (Santa Clara, USA) as described by Noji, et al., 2007.

The catalytic activity of PqqA peptide cyclase activity (PqqE), expressed in the IscR mutant *E. coli* strain transformed with pBS_PQQ (strain BS_PQQ2), as compared with its expression in Isc WT *E. coli* strain transformed with pBS_PQQ (strain BS_PQQ2), or either *E. coli* strain transformed with the control empty plasmid or plasmids pBS_PQQ is increased, based on an increase in detected levels of PQQ produced.

### Example 10 Engineering and characterization of genetically modified E. coli strains capable of enhanced nitrogenase activity

### 10.01 Enhanced growth and nitrogenase activity in an IscR H107Y mutant strain overexpressing nifB gene.

The following strains of *Escherichia coli* used in the example are listed below.

**Table 21: Strains**

| **Name** | **Description** |
|---|---|
| BS1011 | ΔbioB (JW0758-1) derived from *Escherichia coli* K-12 BW25113 having genotype : rrnB3 ΔlacZ4787 hsdR514 Δ(araBAD)567 Δ(rhaBAD)568 rph-1 |
| BS1353 | BS1011 derivative comprising a H107Y mutation in iscR |
| BS2378 | BS1011 transformed with pBS1169 |
| BS2381 | BS1353 transformed with pBS1169 |
| BS2470 | BS1011 transformed with pBS1653 |
| BS2472 | BS1353 transformed with pBS1653 |
| BS2629 | BS1011 transformed with pBS1259 |
| BS2630 | BS1353 transformed with pBS1259 |
| BS2473 | BS1011 transformed with pBS1169, pBS1653 and pBS1654 |
| BS2474 | BS1353 transformed with pBS1169, pBS1653 and pBS1654 |

The following plasmids used in the example are listed below.

**Table 22: Plasmids**

| **Name** | **Description** |
|---|---|
| pBS1169 | Plasmid (AmpR, p15A) comprising genes encoding NifB [SEQ ID No.: 253] operably linked to a T5 LacO repressed promoter [SEQ ID No.: 37] and a rrnB terminator [SEQ ID No.: 94]. |
| pBS1653 | Plasmid (KanR, p15A) comprising genes encoding a NifHDKENXVHesA [SEQ ID No.: 263 - 270] operably linked to a a constitutive promoter [SEQ ID No.: 14] and a rrnB terminator [SEQ ID No.: 94]. |
| pBS1259 | Control empty plasmid (AmpR, p15A) comprising a T5 LacO inducible promoter [SEQ ID No.: 37], a RBS sequence [SEQ ID No.: 4], where AmpR is the sole open reading frame. |
| pBS1654 | Plasmid (CamR, pBR322) comprising genes encoding NifUSFIdA [SEQ ID No.: 33-35] and a rrnB terminator [SEQ ID No.: 94]. |

IPTG-inducible gene encoding NifB (Nitrogenase iron-molybdenum cofactor biosynthesis protein) was cloned to give plasmid pBS1169; and an empty plasmid pBS1259 having a p15A backbone was used as a control. pBS1169 or pBS1259 were introduced into the *E. coli* host strain (BS1353) in which the native iscR gene was substituted by a mutant iscR gene encoding an IscR protein having an H107Y substitution (as described in Example 1) resulting in the strains BS2472 and BS2630, respectively. Similarly, the plasmids pBS1169 or pBS1259 were introduced into *E. coli* strain BS1011 carrying a wild-type version of the iscR gene, resulting in strains BS2378 and BS2629, respectively.

Cells of strains in Table 21 were cultivated 24 h at 37 °C in parallel cultures comprising mMOPS medium (as described in Example 1.03) supplemented with 1 nmol/L biotin, 100 µg/mL ampicillin and with 0 or 1 mM IPTG in deep culture plates. Cell growth was monitored over 24 h by measuring cell density at OD₆₂₀nm (Figure 22). The lag-phase duration and growth rate of strains BS2580 (IscR WT) and BS2581 (IscR mutant), comprising control plasmids was similar under both non-inducing and inducing conditions (Figure 21). However, IPTG induction of NifB transgene expression resulted in an extended lag-phase and slower growth rate for IscR WT strain BS2472, indicating that nifB-overexpression is toxic for the cells. This growth deficiency is largely overcome when the nifB gene is overexpressed in the IscR mutant strain BS2472. This demonstrates that the IscR mutant strain provides sufficient Fe-S clusters to meet the functional requirements of both over-expressed NifB protein and other native Fe-S proteins in the cell and to thereby support cell growth.

### 10.02 Enhanced nitrogenase activity and nitrogen fixation in an IscR H107Y mutant strain overexpressing nifB, or co-overexpressing NifB and additional nitrogenase pathway genes

Additionally, *E. coli* strains having the wild type (BS1011) and mutant IscR gene (BS1353) were transformed with both pBS1169 (comprising the NifB gene) as well as pBS1653 plasmid comprising genes encoding NifHDKENXVHesA; and pBS1654 plasmid comprising genes encoding NifU, NifS and FIdA. The catalytic activity of NifB expressed in the respective *E. coli* strains (Table 21) is determined as follows. Cells of each of the strains are cultured in mMOPS medium (as described in Example 1.03) supplemented with 1 nmol/L biotin, 100 µg/mL ampicillin at 30 degrees Celsius for 16h, then centrifuged (4000 g for 5 min) and washed three times in 1 mL water. Cells from each culture are then re-suspended in nitrogen-deficient medium (10.4 g Na2HPO4, 3.4 g KH2PO4, 26 mg CaCl2·2H2O, 30 mg MgSO4, 0.3 mg MnSO4, 36 mg Ferric citrate, 7.6 mg Na2MoO4·2H2O, 10 µg p-aminobenzoic acid, 5 µg biotin and 4 g glucose per liter and supplemented with 2 mM glutamate as nitrogen source. An optimal IPTG concentration for nifB gene expression was added. When OD600ₙₘ 0.4 is reached, 1 ml of each culture is transferred to an oxygen isolated tube by using argon gas to fill headspace. After 8 h incubation at 30 °C, the obtained cells are assayed for nitrogenase activity by incubating the cells with acetylene (10 % tube headspace volume) for 3 additional hours. The incubated samples are then analysed for ethylene levels, resulting from acetylene reduction, by gas chromatography, therefore giving a measure of nitrogenase activity in the strains.

Nitrogenase activity is enhanced in the IscR mutant *E. coli* strain, BS2472, expressing the transgene encoding nifB alone, and is further enhanced in IscR mutant *E. coli* strain, BS2474, by co-expression of transgenes encoding NifHDKENXVHesA and NifUSFIdA, as compared to their co-expression in the parent host *E. coli* strain expressing wild type IscR (BS2473).

### Example 11 Engineering and characterization of genetically modified E. coli strains capable of enhanced indigo production

### 11.01 Enhanced Rieske di-oxygenase activity in an IscR H107Y mutant strain overexpressing a NdoBCRA genes.

The following strains of *Escherichia coli* used in the example are listed below.

**Table 23: Strains**

| **Name** | **Description** |
|---|---|
| BS1011 | ΔbioB (JW0758-1) derived from Escherichia coli K-12 BW25113 having genotype : rrnB3 ΔlacZ4787 hsdR514 Δ(araBAD)567 Δ(rhaBAD)568 rph-1 |
| BS1353 | BS1011 derivative comprising a H107Y mutation in iscR |
| BS2629 | BS1011 transformed with pBS1259 |
| BS2630 | BS1353 transformed with pBS1259 |
| BS_NdoBCRA1 | BS1011 transformed with pBS_NdoBCRA |
| BS_NdoBCRA2 | BS1353 transformed with pBS_NdoBCRA |

The following plasmids used in the example are listed below.

**Table 24: Plasmids**

| **Name** | **Description** |
|---|---|
| pBS_NdoBCRA | Plasmid (AmpR, p15A) comprising NdoB [SEQ ID No.: 3], NdoC [SEQ ID No.: 4], ], NdoA [SEQ ID No.: 4], NdoR [SEQ ID No.: 4], operably linked to a T5 LacO repressed promoter [SEQ ID No.: 37] and a rrnB terminator [SEQ ID No.: 3]. |
| pBS1259 | Control empty plasmid (AmpR, p15A) comprising a T5 LacO repressed promoter [SEQ ID No.: 37] and a rrnB terminator [SEQ ID No.: 94] but no additional open reading frame. |

IPTG-inducible operon comprising the naphthalene dioxygenase NdoBC and reductase NdoRA genes derived from the *Pseudomonas putida* were cloned to create plasmid pBS_NdoBCRA; and an empty plasmid pBS1259 having a p15A backbone was used as a control. The plasmid pBS_NdoBCRA and the control plasmid pBS1259 were introduced into the *E. coli* host strain (BS1353) in which the native iscR gene was substituted by a mutant iscR gene encoding an IscR protein having an H107Y substitution (as described in Example 1) resulting in the strain BS_NdoBCRA2 and BS2630, respectively. Additionally, the plasmids pBS_NdoBCRA and pBS1259 were introduced into *E. coli* strain BS1011 carrying a wild-type version of the iscR gene, resulting in strains BS_NdoBCRA1 and BS2629, respectively.

Overnight 500uL seed cultures of each strain (Table 19) are cultivated in parallel at 37 °C in 50mL LB medium supplemented with 1.0 nM biotin and 100 µg/mL ampicillin, at 200 rpm until the cell density reaches an OD600ₙₘ of 0.6. IPTG (0.5mM) is then added to the medium; and the cell cultures are further incubated at 27 °C for 24-48 h. The cells are then harvested, mixed with an equal volume of N,N dimethylformamide to solubilize and extract the indigo, and following centrifugation to remove biomass, the absorbance of the extract is read at OD₆₂₀. Indigo concentrations are calculated by comparison to a standard curve constructed using synthetic indigo.

The catalytic activity of the naphthalene dioxygenase NdoBC and reductase NdoRA complex expressed in the IscR mutant *E. coli* strain transformed with pBS_NdoBCRA (strain BS_NdoBCRA2), as compared with its expression in Isc WT *E. coli* strain transformed with pBS_NdoBCRA (strain BS_NdoBCRA1), or either *E. coli* strain transformed with the control empty plasmid is increased, based on an increase in detected levels of indigo pigment produced.

### References

Datsenko KA, Wanner BL. (2000) One-step inactivation of chromosomal genes in Escherichia coli K-12 using PCR products. Proc Natl Acad Sci U S A.;97(12):6640-5.
Fleischhacker, A. S. et al. (2012) Characterization of the [2Fe-2S] cluster of Escherichia coli transcription factor IscR. Biochemistry 51: 4453-4462.
Giel, J. L., Rodionov, D., Liu, M., Blattner, F. R. & Kiley, P. J. (2006) IscR-dependent gene expression links iron-sulphur cluster assembly to the control of O2-regulated genes in Escherichia coli. Mol. Microbiol. 60, 1058-1075.
Herbert AA, Guest JR. (1975) Lipoic acid content of Escherichia coli and other microorganisms. Arch Microbiol.;106(3):259-66. Epub 1975/12/31. pmid:814874
Ifuku, O. et al. Sequencing analysis of mutation points in the biotin operon of biotin-overproducing Escherichia coli mutants. Biosci Biotechnol Biochem 57, 760-765 (1993).
Ifuku, O. et al., (1995) "Molecular analysis of growth inhibition caused by overexpression of the biotin operon in Escherichia coli." Bioscience, biotechnology, and biochemistry 59(2):184-189.
Mutalik et al., (2013) Precise and reliable gene expression via standard transcription and translation initiation elements Nature methods VOL.10(4) 354.
Noji, N., Nakamura, T., Kitahata, N., Taguchi, K., Kudo, T., Yoshida, S., Asami, T. (2007). Simple and Sensitive Method for Pyrroloquinoline Quinone (PQQ) Analysis in Various Foods Using Liquid Chromatography/Electrospray-Ionization Tandem Mass Spectrometry. Journal of Agricultural and Food Chemistry, 55(18), 7258-7263. https://doi.org/10.1021/jf070483r
Ollagnier-de Choudenset al. (2005) Quinolinate synthetase, an iron-Sulphur enzyme in NAD biosynthesis. FEBS Letters 579 3737-3743 Py, B. & Barras, F. (2010) Building Fe-S proteins: bacterial strategies. Nat. Rev. Microbiol. 8, 436-446.
Schmitt, A, Kochanowski K., Vedelaar S., Ahrné E., Volkmer B., Callipo L., Knoops K., Bauer M., Aebersold R., & Heinemann M., (2015) The quantitative and condition-dependent Escherichia coli proteome. Nature Biotechnology 2015; doi:10.1038
Zhou K, Zou R, Stephanopoulos G, Too H-P (2012) Metabolite Profiling Identified Methylerythritol Cyclodiphosphate Efflux as a Limiting Step in Microbial Isoprenoid Production. PLoS ONE 7(11): e47513. doi:10.1371/journal.pone.0047513

## Claims

1. A genetically modified prokaryotic cell comprising:
a. a transgene or a genetically modified iscR gene encoding a mutant IscR polypeptide, and
b.transgenes or endogenous genes encoding at least one Fe-S cluster polypeptide,
wherein the at least one FE-S cluster polypeptide is not any one of i. biotin synthase (EC: 2.8.1.6), ii. lipoic acid synthase (EC: 2.8.1.8), iii. HMP-P synthase (EC: 4.1.99.17), and vi. tyrosine lyase (EC: 4.1.99.19); and
wherein the endogenous genes are operably linked to genetically modified regulatory sequences capable of enhancing expression of said endogenous genes, and
wherein the mutant IscR polypeptide as compared to a corresponding non-mutant IsR polypeptide has an increased apoprotein:holoprotein ratio in the cell; and wherein the production of at least one compound resulting from the catalytic activity of the at least one Fe-S cluster polypeptide is enhanced when compared to a prokaryotic cell comprising the genetically unmodified iscR gene and the transgenes or the endogenous genes encoding at least one Fe-S polypeptide.

2. The cell of claim 1, wherein the amino acid sequence of said mutant IscR polypeptide has at least 80% amino acid sequence identity to a sequence selected from the group consisting of SEQ ID No: 2, 4, 6, 8, 10, 12, 14, and 15-26, and wherein said amino acid sequence has at least one amino acid substitution selected from the group consisting of L15X, C92X, C98X, C104X, and H107X; wherein X is any amino acid other than the corresponding amino acid residue in SEQ ID No.: 2, 4, 6, 8, 10, 12, 14, and 15-26.

3. The cell of claim 1 or 2, wherein said at least one amino acid substitution in said mutant IscR polypeptide is selected from the group consisting of:
a. L15X, wherein X is any one of F, Y, M and W;
b. C92X, wherein X is any one of Y, A, M, F and W;
c. C98X, wherein X is any one of A, V, I, L, F and W;
d. C104X, wherein X is any one of A V, I, L, F and W; and
e. H107X; wherein X, is any one of A, Y, V, I, and L.

4. The cell of any one of claims 1 - 3, wherein said at least one Fe-S cluster polypeptide is selected from the group consisting of: Oxygen-independent coproporphyrinogen III oxidase (EC:1.3.98.3); Quinolate synthase (EC:2.5.1.72); Precorrin-3B synthase (EC:1.14.13.83); Dihydroxy-acid dehydratase (EC:4.2.1.9); 4-hydroxy-3-methylbut-2-en-1-yl diphosphate synthase (EC:1.17.7.3); 4-hydroxy-3-methylbut-2-enyl diphosphate reductase (EC:1.17.7.4); glutamate synthase [NADPH] large chain (EC:1.4.1.13); Glutamate synthase [NADPH] small chain (EC:1.4.1.13); PqqA peptide cyclase (EC:1.21.98.4); Nitrogenase FeMo cofactor biosynthesis protein (EC:-.-.-.-); Naphthalene 1,2-dioxygenase system, large oxygenase component(EC:1.14.12.12); Naphthalene 1,2-dioxygenase system, small oxygenase component(EC:1.14.12.12); Naphthalene 1,2-dioxygenase system, ferredoxin component(EC:1.18.1.7); Naphthalene 1,2-dioxygenase system reductase component(EC:1.18.1.7); NADH-ubiquinone oxidoreductase subunit E (EC: 1.6.5.3); NADH-ubiquinone oxidoreductase chain E (EC:1.6.5.3; 1.6.99.5); Limonene hydroxylase (EC:1.1.1.144; 1.1.1.243; 1.14.13.49); Tetrachlorobenzoquinone (EC:1.1.1.404); Formate dehydrogenase molybdopterin-binding subunit FdhA); (EC:1.1.5.6); Formate dehydrogenase major subunit(EC:1.2.1.2); Formate dehydrogenase H (EC:1.1.99.33); Quinone-reactive Ni/Fe hydrogenase small subunit (HydA)(EC:1.12.1.2); Sulfur reductase subunit (HydB)(EC:1.12.98.4); Mevastatin hydroxylase(EC:1.14.-.-); Anthranilate 1,2-dioxygenase large subunit (EC:1.14.12.1); Benzoate 1,2-dioxygenase subunit alpha (EC:1.14.12.10); Biphenyl dioxygenase subunit alpha(EC:1.14.12.18); 3-phenylpropionate/cinnamic acid dioxygenase (EC:1.14.12.19); Carbazole 1,9-dioxygenase(EC:1.14.12.22); p-cumate 2,3-dioxygenase system(EC:1.14.12.25); Benzene and toluene dioxygenase (EC:1.14.12.3; 1.14.12.11); Methylxanthine N3-demethylase (EC:1.14.13.179); Carnitine monooxygenase oxygenase subunit (EC:1.14.13.239); Methane monooxygenase component C (EC:1.14.13.25); Limonene hydroxylase (EC:1.14.13.48); Phenol hydroxylase (EC:1.14.13.7); Methyl-branched lipid omega-hydroxylase (EC:1.14.15.14); Chloroacetanilide N-alkylformylase(EC:1.14.15.23); Steroid monooxygenase (EC:1.14.15.28); Steroid monooxygenase (EC:1.14.15.29); 3-ketosteroid-9-alpha-monooxygenase(EC:1.14.15.30); Pentalenene oxygenase (EC:1.14.15.32); 6-deoxyerythronolide B hydroxylase (EC:1.14.15.35); Spheroidene monooxygenase (EC:1.14.15.9); Biflaviolin synthase CYP158A2 (EC:1.14.19.69); Mycocyclosin synthase (EC:1.14.19.70); 4-hydroxy-3-methylbut-2-en-1-yl diphosphate synthase (EC:1.17.7.1); Carbazole 1,9a-dioxygenase(EC:1.18.1.3); Cinnamate reductase (EC:1.3.1.-); 8-methylmenaquinol:fumarate reductase iron-sulfur subunit (EC:1.3.5.-); Probable iron-sulfur-binding oxidoreductase FadF (EC:1.3.8.7); CRISPR-associated exonuclease Cas4/endonuclease Cas1 fusion (EC:3.1.12.1); 4-hydroxyphenylacetate decarboxylase small subunit (EC:4.1.1.83); Cyclic pyranopterin monophosphate synthase (EC:4.1.99.18); 5-hydroxybenzimidazole synthase (EC:4.1.99.23); 2-methylcitrate dehydratase (EC:4.2.1.117); Fumarate hydratase class I, anaerobic(EC:4.2.1.2; 4.2.1.81); Fumarate hydratase class I, aerobic (EC:4.2.1.2; 5.3.2.2); Aconitate hydratase A (EC:4.2.1.3;); L(+)-tartrate dehydratase subunit alpha (EC:4.2.1.32); 3-isopropylmalate dehydratase large subunit (EC:4.2.1.33); Isopropylmalate/citramalate isomerase large subunit (EC:4.2.1.35; 4.2.1.31); L-serine dehydratase(EC:4.3.1.17); L-cysteine desulfidase(EC:4.4.1.28); Neomycin C epimerase (EC:5.1.3.-); L-lysine 2,3-aminomutase (EC:5.4.3.-); 3-methylornithine synthase (Pyrrolysine)(EC:5.4.99.58); Xanthine dehydrogenase (EC:1.2.99.7; 1.3.7.9); Zeaxanthin epoxidase(EC:1.14.15.21); Xanthine dehydrogenase/oxidase (EC:1.17.1.4; 1.17.3.2); Vitamin D3 hydroxylase(EC:1.14.15.16); Vitamin D3 hydroxylase (EC:1.14.15.18); Vitamin D3 dihydroxylase (EC:1.14.15.22); Vanillate O-demethylase oxygenase (EC:1.14.13.82); Terephthalate 1,2-dioxygenase(EC:1.14.12.15); Salicylate 5-hydroxylas(EC:1.14.13.172); Cytochrome b-c1 complex subunit Rieske-5(EC:1.10.2.2); Cytochrome P450 monooxygenase PikC (EC:1.14.15.33); Phthalate 4,5-dioxygenase oxygenase subunit (EC:1.14.12.7); Phenoxybenzoate dioxygenase (EC:1.14.12.-); Nicotinate dehydrogenase small FeS subunit (EC:1.17.1.5); Methanesulfonate monooxygenase hydroxylase(EC:1.14.13.111); 6-hydroxynicotinate reductase (EC:1.3.7.1); Dimethyl sulfoxide reductase (EC:1.8.5.3); Vitamin D3 25-hydroxylase(EC:1.14.15.15); 2-halobenzoate 1,2-dioxygenase large subunit (EC:1.14.12.13); Camphor 5-monooxygenase (EC:1.14.15.1); Putidaredoxin reductase CamA (Pdr) (EC:1.18.1.5); Methylxanthine N1-demethylase NdmA (EC:1.14.13.178); Butirosin biosynthesis protein N(EC:1.1.99.38); Beta-carotene hydroxylase (EC:1.14.15.24); 2-amino-4-deoxychorismate dehydrogenase(EC:1.3.99.24); Aminodeoxyfutalosine synthase(EC:2.5.1.120); hopanoid C-3 methylase (EC:2.1.1.-); aminofutalosine synthase (cofactor biosynthesis - menaquinone via futalosine)(EC:2.5.1.120); FO synthase, CofH subunit (cofactor biosynthesis - F420)(EC:2.5.1.147); GTP 3',8-cyclase (molybdenum cofactor)(EC:4.1.99.22); FO synthase, CofG subunit (cofactor biosynthesis - F420)(EC:4.3.1.32); and 7-carboxy-7-deazaguanine (CDG) synthase(EC:4.3.99.3).

5. The cell of any one of claims 1 - 4, wherein the at least one Fe-S cluster polypeptide has oxygen-independent coproporphyrinogen III oxidase synthase (EC: 1.3.98.3) activity, and wherein said cell comprises additional transgenes or additional endogenous genes operably linked to genetically modified regulatory sequences capable of enhancing expression of said endogenous genes,
wherein said additional transgenes or endogenous genes encode one or more polypeptides selected from the group consisting of:
a. a HemA polypeptide having glutamyl-tRNA reductase activity (EC: 1.2.1.70) activity;
b.a HemL polypeptide having glutamate-1-semialdehyde 2,1-aminomutase activity (EC: 5.4.3.8) activity;
c. a HemB polypeptide having delta-aminolevulinic acid dehydratase activity (EC: 4.2.1.24) activity;
d. a HemC polypeptide having porphobilinogen deaminase activity (EC: 2.5.1.61) activity;
e. a HemD polypeptide having Uroporphyrinogen III methyltransferase activity (EC: 4.2.1.75);
f. a HemE polypeptide having uroporphyrinogen decarboxylase activity (EC: 4.1.1.37);
g. a HemZ polypetide having an Oxygen-independent coproporphyrinogen-III oxidase activity (EC: 1.3.98.3);
h. a HemG polypeptide having protoporphyrinogen IX dehydrogenase (menaquinone) activity (EC: 1.3.5.3); and
i. a HemH polypeptide having protoporphyrin ferrochelatase activity (EC: 4.99.1.1).

6. The cell of claim 5, wherein said cell comprises additional transgenes or additional endogenous genes operably linked to genetically modified regulatory sequences capable of enhancing expression of said endogenous genes,
wherein said additional transgenes or endogenous genes encode one or more polypeptides selected from the group consisting of: myoglobin, hemoglobin, neuroglobin, cytoglobin and leghemoglobin, protoheme IX, siroheme, chlorophylls, cofactor F430 and B12), cytochrome P450 monooxygenase (EC: 1.14.-.-), peroxidase (EC: 1.11.1.-), perooxygenase (EC: 1.11.2.-), catechol oxidase (EC: 1.10.3.-), hydroperoxide dehydratase (EC: 4.2.1.-), tryptophan 2,3-dioxygenase (EC: 1.13.11.-), and cytochrome c oxidase (EC: 1.9.3.-).

7. The cell of any one of claims 1 - 6, wherein the at least one Fe-S cluster polypeptide has NadA quinolate synthase activity (EC: 2.5.1.72), and wherein said cell comprises additional transgenes or additional endogenous genes operably linked to genetically modified regulatory sequences capable of enhancing expression of said endogenous genes,
wherein said additional transgenes or endogenous genes encode one or more polypeptides selected from the group consisting of:
a. a NadB polypeptide having aspartate oxidase activity (synthesizes iminoaspartate from L-aspartate (EC: 1.4.3.16);
b.a NadC polypeptide having Nicotinate-nucleotide pyrophosphorylase activity (EC: 2.4.2.19);
c. a NadD polypeptide having Nicotinate-nucleotide adenylyltransferase activity (EC: 2.7.7.18);
d.a NadE polypeptide having NH(3)-dependent NAD(+) synthetase activity (EC: 6.3.1.5);
e. a NudC polypeptide having NADH pyrophospahatase activity (EC: 3.6.1.22);
f. an AphA polypetide having phosphatase activity (EC: 3.1.3.2);
g.a NadE* polypeptide having nicotinic acid mononucleotide amidating activity;
h.a chi polypeptide having NMN nucleosidase activity (EC: 3.2.2.14); and
i. a pncA polypeptide having nicotinamide deamidase activity (EC: 3.5.1.19).

8. The cell of any one of claims 1 - 7, wherein the at least one Fe-S cluster polypeptide is a CobG polypeptide having precorrin-3B synthase (EC: 1.3.98.3), and wherein said cell comprises additional transgenes or additional endogenous genes operably linked to genetically modified regulatory sequences capable of enhancing expression of said endogenous genes,
wherein said additional transgenes or endogenous genes encode one or more polypeptides selected from the group consisting of:
a. a CobA polypeptide having uroporphyrinogen-III C-methyltransferase activity (EC: 2.1.1.107);
b. a CobI polypeptide having precorrin-2 C20-methyltransferase activity (EC: 2.1.1.130);
c. a CobM polypeptide having precorrin-3 methylase activity (EC: 2.1.1.133);
d.a CobF polypeptide having cobalt-precorrin-6A synthase activity (EC: 2.1.1.195);
e.a CobK polypeptide having precorrin-6A reductase activity (EC: 1.3.1.54);
f. a CobH polypeptide having precorrin isomerase activity (EC: 5.4.99.61);
g.a CobL polypeptide having precorrin-6Y C(5,15)-methyltransferase activity (EC: 2.1.1.132);
h.a CobJ polypeptide having precorrin-3B C(17)-methyltransferase (EC: 2.1.1.131);
i. a CobB polypeptide having Hydrogenobyrinate a,c-diamide synthase activity (EC: 6.3.5.9);
j. a CobNST polypeptide having Cobaltochelatase activity (EC: 6.6.1.2);
k. a CobO polypeptide having Corrinoid adenosyltransferase activity (EC: 2.5.1.17);
l. a CobQ polypeptide having Cobyrinate a,c-diamide synthase activity (EC: 6.3.5.11);
m. a CbiB polypeptide A CobU polypeptide having Adenosylcobinamide kinase activity (EC: 2.7.1.156) and adenosylcobinamide-phosphate guanylyltransferase activity (EC: 2.7.7.62);
n.a CobC polypeptide having Adenosylcobalamin phosphatase activity (EC: 3.1.3.73);
o.a PduX polypeptide having L-threonine kinase activity (EC: 2.7.1.177);
p. a CobD polypeptide having Threonine-phosphate decarboxylase activity (EC: 4.1.1.81);
q.a CobT polypeptide having dimethylbenzimidazole phosphoribosyltransferase activity (EC: 2.4.2.21);
r. a BtuR polypetide having corrinoid adenosyltransferase activity (EC: 2.5.1.17);
s. a CbiO polypeptide having cobalt import ATP-binding protein activity (EC: 3.6.3..-);
t. a CbiN polypeptide having the function of a cobalt transport protein;
u. a CbiQ polypeptide having the function of a cobalt transport protein; and
v. a CbiM polypeptide having the function of a cobalt transport protein.

9. The cell of any one of claims 1 - 8, wherein the at least one Fe-S cluster polypeptide is an IlvD polypeptide having dihydroxy-acid dehydratase activity (EC: 4.2.1.9), and wherein said cell comprises additional transgenes or additional endogenous genes operably linked to genetically modified regulatory sequences capable of enhancing expression of said endogenous genes,
wherein said additional transgenes or endogenous genes encode one one or more polypeptides selected from the group consisting of:
a. an IlvB large subunit polypeptide and an IlvN small subunit having acetolactate synthase isozyme 1 activity (EC: 2.2.1.6);
b.an IlvC polypeptide having ketol-acid reductoisomerase (NADP+) activity (EC: 1.1.1.86); and
c. an IlvE branched-chain-amino-acid aminotransferase activity (EC: 2.6.1.42).

10. The cell any one of claims 1 - 8, wherein the at least one Fe-S cluster polypeptide is an IspG polypeptide having 4-hydroxy-3-methylbut-2-en-1-yl diphosphate synthase (EC: 1.17.7.3) and/or an IspH polypeptide having 4-hydroxy-3-methylbut-2-enyl diphosphate reductase activity (EC: 1.17.7.4), wherein said cell comprises additional transgenes or additional endogenous genes operably linked to genetically modified regulatory sequences capable of enhancing expression of said endogenous genes,
wherein said additional transgenes or endogenous genes encode:
a. a DXS polypeptide having 1-deoxy-D-xylulose-5-phosphate synthase activity (EC: 2.2.1.7);
b. an IspC polypeptide having 1-deoxy-D-xylulose 5-phosphate reductoisomerase activity (EC: 1.1.1.267);
c. an IspE polypetide having 4-diphosphocytidyl-2-C-methyl-D-erythritol kinase activity (EC: 2.7.1.148);
d.an IspD polypetide having 2-C-methyl-D-erythritol 4-phosphate cytidylyltransferase activity (EC: 2.7.7.60);
e.an IspF polypetide having 2-C-methyl-D-erythritol 2,4-cyclodiphosphate synthase (EC: 4.6.1.12);
f. an IpI polypetide having isopentenyl-diphosphate Delta-isomerase activity (EC: 5.3.3.2); and
g.a RpoS polypetide having the function of a RNA polymerase subunit sigma factor σ.

11. The cell any one of claims 1 - 8, wherein the at least one Fe-S cluster polypeptide is a large chain GltB polypeptide and a small chain GltD polypeptide having glutamate synthase [NADPH] actvity (EC: 1.4.1.13).

12. The cell of claim 11, wherein said cell comprises additional transgenes or additional endogenous genes operably linked to genetically modified regulatory sequences capable of enhancing expression of said endogenous genes, wherein said additional transgenes or endogenous genes encode one or more polypeptides selected from the group consisting of:
a. a GltX polypeptide having glutamyl-tRNA synthetase activity, (EC: 6.1.1.17);
b.a HemA polypeptide having glutamyl-tRNA reductase activity, (EC: 1.2.1.70); and
c. a HemL polypeptide having Glutamate-1-semialdehyde 2,1-aminomutase activity, (EC: 5.4.3.8).

13. The cell of any one of claims 1 - 8, wherein the at least one Fe-S cluster polypeptide is a PqqE polypeptide having PqqA peptide cyclase activity (EC: 1.21.98.4), wherein said cell comprises additional transgenes or additional endogenous genes operably linked to genetically modified regulatory sequences capable of enhancing expression of said endogenous gene,
wherein said additional transgenes or endogenous genes encode one one or more polypeptides selected from the group consisting of:
a. a PqqA polypeptide;
b.a PqqB polypeptide having a PQQ carrier function,
c. a PqqC polypeptide having Pyrroloquinoline-quinone synthase activity (EC: 1.3.3.11),
d. a PqqD polypeptide having PqqA binding activity; and
e. A PqqF polypeptide having metalloendopeptidase (EC: 3.4.24.-).

14. The cell of any one of claims 1 - 8, wherein the at least one Fe-S cluster polypeptide is a NifB polypeptide having nitrogenase FeMo cofactor biosynthesis activity, wherein said cell comprises additional transgenes or additional endogenous genes operably linked to genetically modified regulatory sequences capable of enhancing expression of said endogenous genes,
wherein said additional transgenes or endogenous genes encode one one or more polypeptides selected from the group consisting of:
a. a NifD polypeptide having nitrogenase protein alpha chain activity (EC: 1.18.6.1)
b.a NifH polypeptide having nitrogenase iron protein (EC: 1.18.6.1) activity;
c. a NifK polypeptide having nitrogenase molybdenum-iron protein beta chain (EC: 1.18.6.1) activity;
d.a NifE polypeptide having Fe-Mo co-factor biosynthesis activity;
e.a NifN polypeptide having Fe-Mo co-factor biosynthesis activity;
f. a NifX polypeptide having nitrogen fixation protein activity;
g.a HesA polypeptide; and
h.a NifV polypeptide having an isocitrate synthase activity (EC: 2.3.3.14).

15. The cell of any one of claims 1 - 14, wherein said additional transgenes or endogenous genes further encode one or more polypeptides selected from the group consisting of:
a. a gene encoding a flavodoxin/ferredoxin-NADP reductase (EC: 1.18.1.2 and EC 1.19.1.1);
b.a gene encoding a pyruvate-flavodoxin/ferredoxin oxidoreductase (EC: 1.2.7);
c. a gene encoding a flavodoxin;
d.a gene encoding a ferredoxin; and
e.a gene encoding a flavodoxin and a ferredoxin-NADP reductase; wherein said endogenous genes are operably-linked genetically modified regulatory sequences capable of enhancing expression of said endogenous genes in said cell.

16. The cell of any one of claims 1 to 15, wherein said prokaryote is a genus of bacterium selected from the group consisting of *Escherichia, Bacillus, Brevibacterium, Burkholderia, Campylobacter, Corynebacterium, Pseudomonas, Serratia, Lactobacillus, Lactococcus, Acinetobacter, Pseudomonas, Acetobacter, Rhizobium, Frankia,* and *Azospirillum.*

17. A method for producing a compound resulting from catalytic activity of the Fe-S cluster polypeptide of any one of claims 1-16, comprising the steps of:
a. introducing a genetically modified prokaryote according to any one of claims 1 - 16, into a growth medium to produce a culture;
b. cultivating the culture; and
c. recovering said compound produced by said culture, and optionally purifying the recovered compound.

18. The method of claim 17, further comprising at least one step of producing the compound which is performed *in vitro.*

19. A fermentation liquid comprising the cell culture of claim 17, and its contents of a compound resulting from catalytic activity of the Fe-S cluster protein.

20. A composition comprising the fermentation liquid of claim 19, and one or more agents, additives and/or excipients.

21. Use of a genetically modified gene encoding a mutant iscR polypeptide to increase production of at least one compound resulting from the catalytic activity of at least one Fe-S cluster polypeptide in a genetically modified prokaryotic cell as compared to a prokaryotic cell comprising the genetically unmodified iscR gene, wherein said Fe-S cluster polypeptideis not any one of biotin synthase (EC: 2.8.1.6), lipoic acid synthase (EC: 2.8.1.8), HMP-P synthase (EC: 4.1.99.17), and tyrosine lyase (EC: 4.1.99.19), wherein the mutant IscR polypeptide as compared to a non-mutant IscR polypeptide has an increased apoprotein:holoprotein ratio in the cell.

22. The use according to claim 21, wherein the amino acid sequence of said mutant IscR polypeptide has at least 80% amino acid sequence identity to a sequence selected from the group consisting of SEQ ID No: 2, 4, 6, 8, 10, 12, 14, and 15-26, and wherein said amino acid sequence has at least one amino acid substitution selected from the group consisting of L15X, C92X, C98X, C104X, and H107X; wherein X is any amino acid other than the corresponding amino acid residue in SEQ ID No.: 2, 4, 6, 8, 10, 12, 14 and 15-26.

23. The use according to claim 22, wherein said at least one amino acid substitution in said mutant IscR polypeptide is selected from the group consisting of:
a. L15X, wherein X is any one of F, Y, M and W;
b. C92X, wherein X is any one of Y, A, M, F and W;
c. C98X, wherein X is any one of A, V, I, L, F and W;
d. C104X, wherein X is any one of A V, I, L, F and W; and
e. H107X; wherein X, is any one of A, Y, V, I, and L.

24. Use of the composition of claim 23, as any one of a dietary supplement, pharmaceutical, chemical building block, pharmaceutical or fertilizer, wherein the compound is selected from the group consisting of: Rieske iron di-oxygenase, cytochrome P450 monooxygenase, vitamin B3, niacin, nicotinamide, nicotinamide riboside, nicotinamide mononucleotide, nicotinamide adenine dinucleotide, pantothenate, cobalamin, methoxatinpyrroloquinoline quinone, quinoprotein, isobutanol, isopropanol, L-valine, L-leucine, L-isoleucine, L-glutamate, δ-aminolevulinic acid, isoprenoid, hydrogen, ammonia and indigo.
